# EUROPEAN PATENT SPECIFICATION

(11) **EP 3 830 276 B1**
(45) Date of publication and mention of the grant of the patent: **24.07.2024**
(21) Application number: 19759679.4
(22) Date of filing: 01.08.2019
(51) Int. Cl.: C12N 15/82, A24B 15/10

(54) **METHOD FOR REDUCING THE CONTENT OF NITROSAMINE IN TOBACCO USING A CONSTITUTIVELY HIGH AFFINITY CATION EFFLUX TRANSPORTER**
VERFAHREN ZUR REDUZIERUNG DES NITROSAMINGEHALTS IN TABAK UNTER VERWENDUNG EINES KONSTITUTIV HOCHAFFINEN KATIONEN-EFFLUX-TRANSPORTERS
PROCÉDÉ DE RÉDUCTION DE LA TENEUR EN NITROSAMINE DU TABAC A L'AIDE DE UN TRANSPORTEUR EFFLUX DE CATIONS D'AFFINTE ELEVEE ACTIVE PAR CONSTITUTION

(30) Priority: 02.08.2018 GB 201812603
(43) Date of publication of application: 09.06.2021
(73) Proprietor: British American Tobacco (Investments) Limited, London WC2R 3LA (GB)
(72) Inventor: BEN KHALED, Sara, London WC2R 3LA (GB); ANASTACIO DE ABREU E LIMA, Francisco, London WC2R 3LA (GB)
(74) Representative: Dehns
(86) International application number: PCT/GB2019/052165
(87) International publication number: WO 2020/025963

(56) References cited:
- WO-A1-2018/067985
- DATABASE UniProtKB [online] 12 April 2017 (2017-04-12), "SubName: Full=metal tolerance protein 9-like {ECO:0000313|RefSeq:XP_016510274.1};", XP002795241, retrieved from EBI accession no. UNIPROT:Unreviewed Database accession no. A0A1S4DAN3
- DATABASE UniProt [online] 14 December 2011 (2011-12-14), "SubName: Full=Manganese tolerance protein 2 {ECO:0000313|EMBL:AEP40484.1};", XP002795242, retrieved from EBI accession no. UNIPROT:G5CCK8 Database accession no. G5CCK8
- DAVID RUSSELL ET AL: "Evidence for zinc and cadmium binding in a CDF transporter lacking the cytoplasmic domain", FEBS LETTERS., vol. 586, no. 24, 14 December 2012 (2012-12-14), NL, pages 4332 - 4338, XP055635779, ISSN: 0014-5793, DOI: 10.1016/j.febslet.2012.10.043
- SIMINSZKY B ET AL: "Conversion of nicotine to nomicotine in Nicotiana tabacum is mediated by CYP82E4, a Cytochrome P450 monooxygenase", PROCEEDINGS OF THE NATIONAL ACADEMY OF SCIENCES (PNAS), US, vol. 102, no. 41, 11 October 2005 (2005-10-11), pages 14919 - 14924, XP008124585, ISSN: 0027-8424, [retrieved on 20050928], DOI: 10.1073/PNAS.0506581102
- XU DONGMEI ET AL: "Biochemical and molecular characterizations of nicotine demethylase in tobacco", PHYSIOLOGIA PLANTARUM, vol. 129, no. 2, 1 February 2007 (2007-02-01), MUNKSGAARD INTERNATIONAL PUBLISHERS, COPENHAGEN, DK, pages 307 - 319, XP002493570, ISSN: 0031-9317, DOI: 10.1111/J.1399-3054.2006.00811.X

## Description

### FIELD OF THE INVENTION

The present invention relates to reducing specific nitrosamines or their precursors in tobacco and products derived therefrom (e.g. propagation materials, harvest leaf, processed tobacco and tobacco industry products). In particular, the invention relates to a cation efflux protein and its use in modulating (e.g. reducing) specific nitrosamines or their precursors in tobacco.

### BACKGROUND

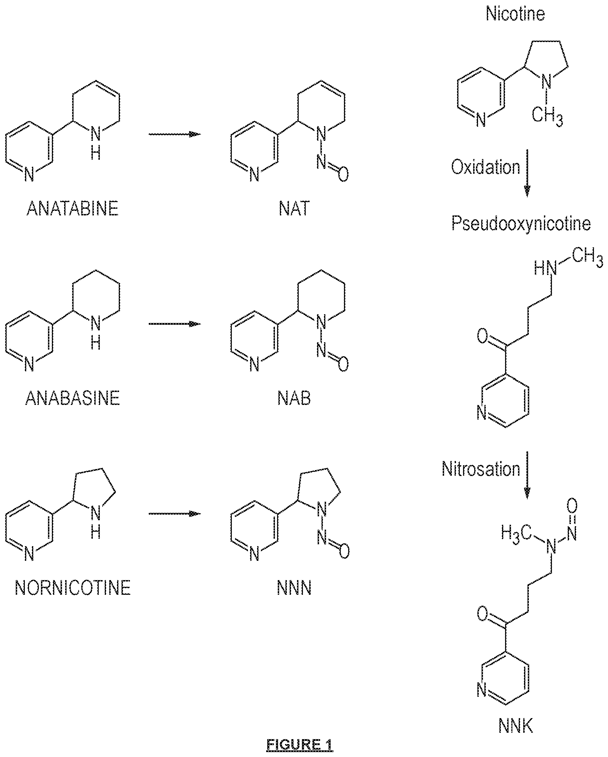

Tobacco pyridine alkaloids are precursors of tobacco-specific nitrosamines (TSNAs) that form during the post-harvest leaf curing. The four primary TSNAs found in cured tobacco leaves are N'-nitrosonornicotine (NNN), N'nitrosoanatabine (NAT), N'-nitrosoanabasine (NAB) and 4-(methyl nitrosamino)-1-(3-pyridyl)-1-butanone (NNK). TSNAs form when nitrous oxide species (e.g. NO, NO₂, N₂O₃ and N₂O₄) react with tobacco alkaloids (Figure 1). NAT and NAB are formed via the nitrosation of the secondary alkaloids anatabine and anabasine, respectively. Although early studies claimed that NNN originates from both nicotine and nornicotine, more recent reports have demonstrated that the occurrence of NNN in cured tobacco leaves is correlated with nornicotine content, not nicotine (Bush et al., Rec. Adv. Tob. Sci. 27; 23-46 (2001); Lewis et al., Plant Biotech J. 6: 346-354 (2008)). Nornicotine is the demethylated derivative of nicotine, the major alkaloid in tobacco accounting for 90% of the total alkaloid content (Saitoh et al., 1985 Phytochemistry, 24 pp. 477-480). The precursor/product relationship of NNK formation is less clear. Some studies state that NNK is a nitrosation product of nicotine, but due to the slow reaction rate of nicotine nitrosation, it is likely that an oxidized derivative(s) of nicotine, rather than nicotine itself serves as the direct precursor of NNK (Caldwell et al Ann. N.Y. Acad. Sci. 686, 213-228 (1993)). Identifying the genes responsible of the production and regulation of the TSNA precursors is of high importance.

Although nornicotine typically accounts for only 2-4% of the total pyridine alkaloid content in tobacco plants, the genetic instability that leads to the spontaneous appearance of high nornicotine-containing converter plants is a chronic problem in tobacco production. Maintaining low nornicotine levels may prevent the objectionable flavour and aroma associated with this alkaloid, as well as reducing the formation of N-nitrosonornicotine (NNN) in tobacco industry products, of which nornicotine is the direct precursor.

The gene responsible for the majority of the nicotine to nornicotine conversion is a nicotine demethylase gene CYP82E4, encoding a cytochrome P450 monooxygenase (Siminszky et al., Proc. Natl. Acad. Sci. USA, 102 (2005), pp. 14919-14924; Xu et al., Physiol. Plantarum, 129 (2007), pp. 307-319). The nicotine demethylase gene family in tobacco is extensively characterised, but little is known about other cell processes that can influence nornicotine levels.

There still exists a great need to devise methodologies that can further reduce the levels of TSNAs in tobacco plants and products produced from tobacco plants.

Cation efflux proteins are pumps that remove metal ions such as cadmium (Cd), zinc (Zn), and cobalt (Co) from the cell. These metals are not only essential micronutrients for the plant metabolism, but they also act as secondary messengers influencing several enzymatic reactions and signal transduction cascades. Co, Cd, and Zn increase the activity of N-demethylase, the content of cytochrome P-450 and microsomal heme in mice (Kadiiska et al. E. Arch Toxicol (1985) 56: 167.). It was proposed that these cations exert an enzyme-inducing effect on the hepatic monooxygenases.

Cation efflux proteins typically include an N-terminal cytoplasmic domain often containing metal binding sites, 4-6 transmembrane domains followed by a C-terminal cation efflux domain. It was proposed that the N-terminal cytoplasmic domain exhibits a negative regulation of the protein by interacting with key metal-binding residues in a central cytoplasmic domain, preventing metal transport. When metal concentration in the cell increases to a certain threshold, metal binding to the N-terminal domain induces a conformational change that releases the inhibition on the cytoplasmic binding sites. The cation efflux changes to a high affinity state and metal transport is enabled (Futai et al., Handbook of ATPases: Biochemistry, Cell Biology, Pathophysiology 2004).

### SUMMARY OF THE INVENTION

According to one aspect, the present invention provides a method of reducing the content of at least one tobacco specific nitrosamine (TSNA) or a precursor of a TSNA in tobacco comprising expressing a deregulated cation efflux protein in a tobacco plant or plant part thereof or plant cell.

The deregulated cation efflux protein may be a constitutively high affinity cation efflux transporter. The deregulated cation efflux protein may exhibit increased metal ion transport compared to a wild type cation efflux transporter. The deregulated cation efflux protein may lack a functional regulatory domain, preferably the deregulated cation efflux protein lacks at least part of a regulatory domain. The deregulated cation efflux protein may lack a functional cytoplasmic domain, preferably the deregulated cation efflux protein lacks at least part of the cytoplasmic domain. The deregulated cation efflux transporter may be deregulated when compared to a wild-type cation efflux protein comprising the amino acid sequence shown as SEQ ID No. 3, 6, 9, 12, 15, 18, 21, 24, 27 or 30 or a sequence which has at least 70% identity thereto. The deregulated cation efflux transport protein may comprises one or more mutations compared to the amino acid sequence shown as SEQ ID No. 3, 6, 9, 12, 15, 18, 21, 24, 27 or 30 or a sequence which has at least 70% identity thereto. The method may comprise introducing into the genome of said plant or plant cell a mutation within a polynucleotide encoding a cation efflux protein such that the polynucleotide encodes a deregulated cation efflux protein. The mutation which is introduced to the plant genome may produce a deletion, a splice mutant or codon encoding a non-tolerated amino acid substitution in the polynucleotide encoding said protein.

The deregulated cation efflux protein may comprise an amino acid sequence which lacks at least part of the N terminus when compared with an amino acid sequence shown as SEQ ID No. 3, 6, 9, 12, 15, 18, 21, 24, 27 or 30 or a sequence which has at least 70% identity thereto. The method may comprise introducing into the genome of said plant or plant cell an exogenous polynucleotide sequence which encodes a deregulated cation efflux protein.

The deregulated cation efflux protein may be:
a) a truncated cation efflux protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated cation efflux protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30. The TSNA is N'-nitrosonornicotine (NNN) and/or the precursor may be nornicotine.

Provided herein, but not forming part of the claimed invention, is a method of producing tobacco having a reduced content of a tobacco specific nitrosamine (TSNA) or a precursor of a TSNA, comprising:
a. crossing a donor tobacco plant which produces a reduced content of a tobacco specific nitrosamine (TSNA) or a precursor of a TSNA and comprises a deregulated cation efflux protein with a recipient tobacco plant that does not produce reduced content of a tobacco specific nitrosamine (TSNA) or a precursor of a TSNA and possesses commercially desirable traits;
b. isolating genetic material from a progeny of said donor plant crossed with said recipient plant; and
c. performing molecular marker-assisted selection with a molecular marker comprising:
   i. identifying an introgressed region comprising a mutation in a polynucleotide sequence encoding a protein defined in a.

The deregulated cation efflux protein may comprise the amino acid sequence shown as SEQ ID No. 33, or a sequence which has at least 70%, at least 75%, at least 80%, at least 85%, at least 90%, at least 95%, at least 97%, or at least 99% sequence identity thereto. The deregulated cation efflux protein may be encoded by a polynucleotide comprising the sequence shown as SEQ ID No. 34, or a sequence which has at least 70% sequence identity thereto, preferably at least 75%, at least 80%, at least 85%, at least 90%, at least 95%, at least 97%, or at least 99% sequence identity thereto

In another aspect, there is provided an isolated polynucleotide comprising a nucleotide sequence selected from the group consisting of:
a. A nucleotide sequence comprising SEQ ID No. 34;
b. A nucleotide sequence having at least 70% sequence identity to the entirety of the sequence set forth in SEQ ID No. 34, wherein said nucleotide sequence encodes a polypeptide involved in cation efflux in a plant;
c. A nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
d. A nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or2-117, or 2-117, or 2-120, or 2-125, or 2-128, or 2-130 of SEQ ID No. 3, 6, 9, 12, 15, 18, 21, 24, 27 or 30;
e. A nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
f. A nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant;
g. A nucleotide sequence according to any of preceding items (a) to (f) which further comprises a mutation in said nucleotide sequence, e.g. where said mutation produces a deletion, a splice mutant or codon encoding a non-tolerated amino acid substitution in the polynucleotide encoding said protein; or
h. A nucleotide sequence that is complementary to the sequence according any of preceding items (a) to (g).

In a further aspect, there is provided an isolated polypeptide comprising an amino acid sequence selected from the group consisting of:
a. An amino acid sequence comprising SEQ ID No. 33;
b. An amino acid sequence that is at least 70% identical to an amino acid sequence set forth in SEQ ID No. 33;
c. An amino acid sequence comprising amino acids which correspond to amino acids 125-324 of SEQ ID No. 3, 6, 9, 12, 15, 18, 21, 24, 27 or 30 and/or comprising amino acids which correspond to amino acids 323-413 of SEQ ID No. 3, 6, 9, 12, 15, 18, 21, 24, 27 or 30 wherein said polypeptide is involved in cation efflux in a plant;
d. An amino acid sequence which is a fragment of SEQ ID No. 3, 6, 9, 12, 15, 18, 21, 24, 27 or 30 comprising at least 200, or at least 250, or at least 300, or at least 350, or at least 400 contiguous residues of SEQ ID No. 3, 6, 9, 12, 15, 18, 21, 24, 27 or 30;
e. An amino acid which is a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or2-114, or 2-117, or 2-117, or 2-120, or 2-125, or 2-128, or 2-130 of SEQ ID No. 3, 6, 9, 12, 15, 18, 21, 24, 27 or 30;
f. An amino acid which is a truncated amino acid sequence which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30; or
g. An amino acid sequence having at least 70% sequence identity to the entirety of the sequence set forth in SEQ ID No. 33.

In a further aspect, there is provided a construct or vector comprising a polynucleotide according to the present invention or a polynucleotide encoding a polypeptide according to the present invention.

In another aspect, there is provided a tobacco plant or part thereof:
a. which has been modified to achieve a reduction in a TSNA or a precursor of a TSNA compared with an unmodified plant, wherein the modified plant or part thereof comprises a deregulated cation efflux protein;
b. which has a mutation within a gene of the plant encoding a cation efflux protein which mutation deregulates said cation efflux protein, wherein the gene prior to mutation comprises the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 70% sequence identity thereto;
c. obtained or obtainable by the method according to any one of claims 1-16;
d. comprising an exogenous gene which encodes a deregulated cation efflux protein wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or2-100, or2-114, or2-117, or 2-117, or 2-120, or 2-125, or 2-128, or 2-130 of SEQ ID No. 3, 6, 9, 12, 15, 18, 21, 24, 27 or 30;
e. comprising a deregulated cation efflux protein which comprises the amino acid sequence set forth in SEQ ID No. 33 or a sequence with at least 70% identity thereto, or a polynucleotide sequence set forth in SEQ ID No. 34 or a sequence with at least 70% identity thereto; or
f. comprising a construct or vector according to the present invention.

In another aspect, there is provided a cell:
a. comprising a deregulated cation efflux protein;
b. which has a mutation within a gene encoding a cation efflux protein which mutation deregulates said cation efflux protein, wherein the gene prior to mutation comprises the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 70% sequence identity thereto;
c. obtained or obtainable by the method according to the present invention;
d. comprising an exogenous gene which encodes a deregulated cation efflux protein wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
e. comprising a deregulated cation efflux protein which comprises the amino acid sequence set forth in SEQ ID No. 33 or a sequence with at least 70% identity thereto, or a polynucleotide sequence set forth in SEQ ID No. 34 or a sequence with at least 70% identity thereto; or
f. comprising a construct or vector according to the present invention.

The cell may be a plant cell (e.g. tobacco plant cell) or a yeast cell.

In a further aspect, there is provided a cell culture comprising a cell or population of cells according to the present invention.

In another embodiment there is provided a plant propagation material (e.g. a plant seed) obtainable from a plant according to the present invention.

The plant for use in the present invention may be from the species *Nicotiana tabacum* or *Nicotiana rustica.*

In another aspect, there is provided the use of a cell according to the present invention, or of a tobacco plant or tobacco plant part thereof according to the present invention, or cell culture according to the present invention, or a deregulated cation efflux protein as defined herein, for the production of a tobacco industry product.

In yet another aspect, there is provided the use of a tobacco plant according to the present invention or a plant propagation material according to the present invention to breed a tobacco plant.

In a further aspect, there is provided the use of a tobacco plant according to the present invention or a plant propagation material according to the present invention to grow a crop. In another aspect, there is provided the use of a tobacco plant according to the present invention or a plant propagation material according to the present invention to produce a processed (preferably cured) tobacco leaf.

In another aspect, there is provided a harvested leaf of a tobacco plant according to the present invention or obtainable from a tobacco plant propagated from a propagation material according to the present invention or obtainable from a tobacco plant obtainable by a method according to the present invention or obtainable by a use according to the present invention. In one aspect, there is provided a harvested leaf of a tobacco plant according to the present invention wherein the harvested leaf is a cut harvested leaf.

In a further aspect, there is provided a processed tobacco leaf (preferably a non-viable processed tobacco leaf):
a. comprising a plant cell according to the present invention;
b. obtainable from processing a tobacco plant according to the present invention;
c. obtainable from a tobacco plant propagated from a plant propagation material according to the present invention; or
d. obtainable by processing a harvested leaf according to the present invention;

The processed tobacco leaf may be processed by curing, fermentation, pasteurising or combinations thereof. The processed tobacco leaf may be a cut processed tobacco leaf.

In yet another aspect, the present invention provides cured tobacco material made from a plant or a part thereof according to the present invention or is obtained (or obtainable) from the plant propagation material according the present invention or obtained (or obtainable) by the method according to the present invention.

In one aspect there is provided a tobacco blend comprising cured tobacco material according to the present invention.

In another aspect there is provided a tobacco industry product:
a. prepared from a tobacco plant according to the present invention or a part thereof;
b. prepared from a tobacco plant or a part thereof (preferably the leaves harvested from the plant) obtained or obtainable by the method according to the present invention;
c. prepared from the plant (preferably the leaves) propagated from a plant propagation material according to the present invention;
d. prepared from a harvested leaf according to the present invention;
e. prepared from a processed leaf according to the present invention;
f. prepared from a cell according to the present invention;
g. or prepared from a cell culture according to the present invention;
h. prepared from a cured tobacco material according to the present invention; or
i. prepared from a tobacco blend according to the present invention.

The tobacco industry product may be a combustible smoking article or a smokeless tobacco industry product or a non-combustible aerosol provision system, such as a tobacco heating device (e.g. an aerosol-generating device).

In another aspect, there is provided a combustible smoking article, non-combustible aerosol provisioning system, smokeless tobacco industry product or tobacco heating device comprising a plant or a portion thereof from the species *Nicotiana tabacum* or *Nicotiana rustica* according to the present invention, or obtainable (e.g. obtained) from a plant propagation product according to the present invention, or obtainable (e.g. obtained) from a method according to the present invention.

In another aspect there is provided the use of a nucleotide sequence encoding a deregulated cation efflux protein comprising the amino acid sequence shown as SEQ ID No. 33, or sequence which has at least 70% identity thereto, or a nucleotide sequence which encodes a deregulated cation efflux protein wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30 to select a plant having modified content of a TSNA or a precursor of a TSNA.

In yet another aspect there is provided a mutant of a plant carrying a heritable mutation in a nucleotide sequence of at least one gene encoding a deregulated cation efflux protein comprising the amino acid sequence shown as SEQ ID No. 33, or sequence which has at least 70% identity thereto, or a nucleotide sequence which encodes a deregulated cation efflux protein wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30; wherein said heritable mutation decreases the content of at least one TSNA or a precursor of a TSNA relative to a comparable plant which does not carry said heritable mutation.

In a further aspect, there is provided progeny or seed of a mutant plant which carries the heritable mutation according to the present invention.

In another aspect, there is provided a harvested leaf, a processed leaf or cured tobacco material produced from a plant comprising a mutation in a nucleotide sequence encoding a deregulated cation efflux protein comprising the amino acid sequence shown as SEQ ID No. 33, or sequence which has at least 70% identity thereto, or a nucleotide sequence which encodes a deregulated cation efflux protein wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30; wherein said harvested leaf, processed leaf or cured tobacco has decreased content of at least one TSNA or a precursor of a TSNA relative to a comparable plant which does not carry said modification.

In another aspect, there is provided a method, a tobacco leaf, a tobacco plant, a tobacco plant propagation material, a harvested leaf, a processed tobacco, a tobacco industry product, a cell, a cell culture, a use or a combination thereof substantially as described herein with reference to the description and drawings.

### BRIEF DESCRIPTION OF THE DRAWINGS

Embodiments of the invention will now be described, by way of example only, with reference to accompanying drawings, in which:
**Figure 1** shows the formation of tobacco-specific nitrosamines (TSNA) from precursors such as nicotine and nornicotine via a nitrosation reaction in tobacco smoke.
**Figure 2** shows the nornicotine content of 5 week old tobacco leaves which have been modified by virus-induced gene silencing of the cation efflux protein.
**Figure 3** shows the nornicotine content of 5 week old tobacco leaves which have been modified to express a luciferase control (SEQ ID No. 44), the wild-type sequence (SEQ ID No. 2), an anti-sense (SEQ ID No. 39), cation efflux_A117C variant (SEQ ID No. 32) or cation efflux_Δ117N variant (SEQ ID No. 34).
**Figure 4a** shows a schematic of a proposed mechanism of low affinity and high affinity confirmations of a cation efflux transporter.
**Figure 4b** shows a schematic of a proposed mechanism for cation efflux_Δ117C and cation efflux_Δ117N expression.

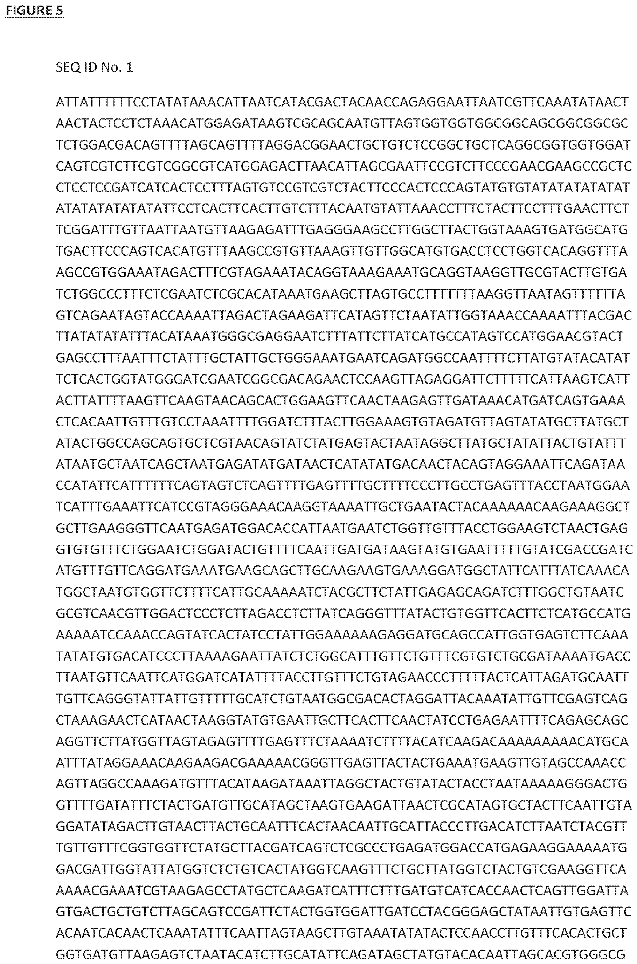

**Figure 5** shows the genomic sequence - SEQ ID No. 1 - encoding a protein according to the present invention.

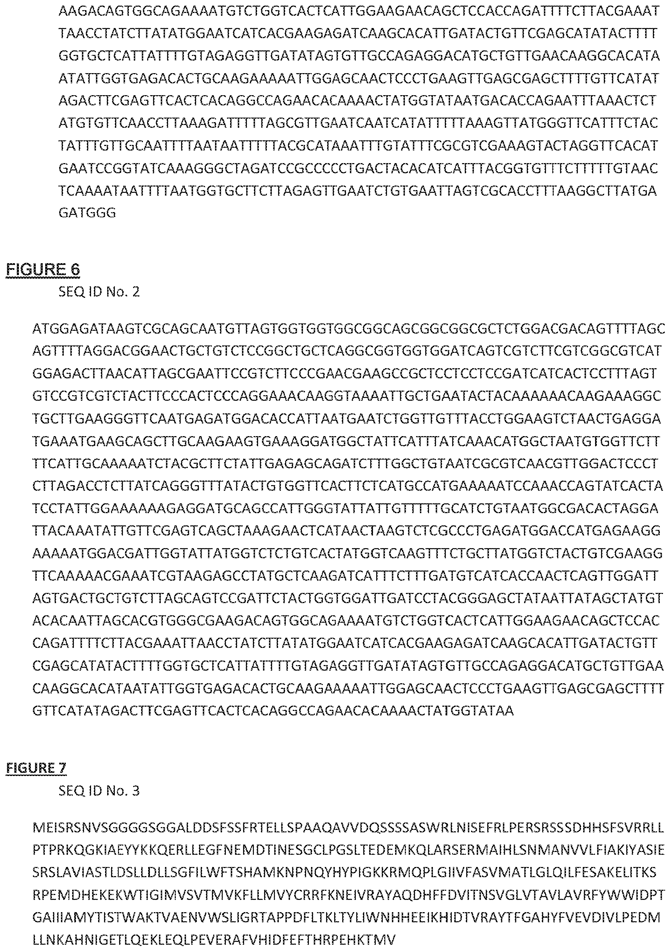

**Figure 6** shows the cDNA sequence - SEQ ID No. 2 - encoding a protein according to the present invention.
**Figure 7** shows the polypeptide sequence SEQ ID No. 3 of a protein in according to the present invention - the polypeptide sequence comprises cation efflux transmembrane domain (amino acids 125-324) and a cation efflux cytoplasmic domain (amino acids 323-413).

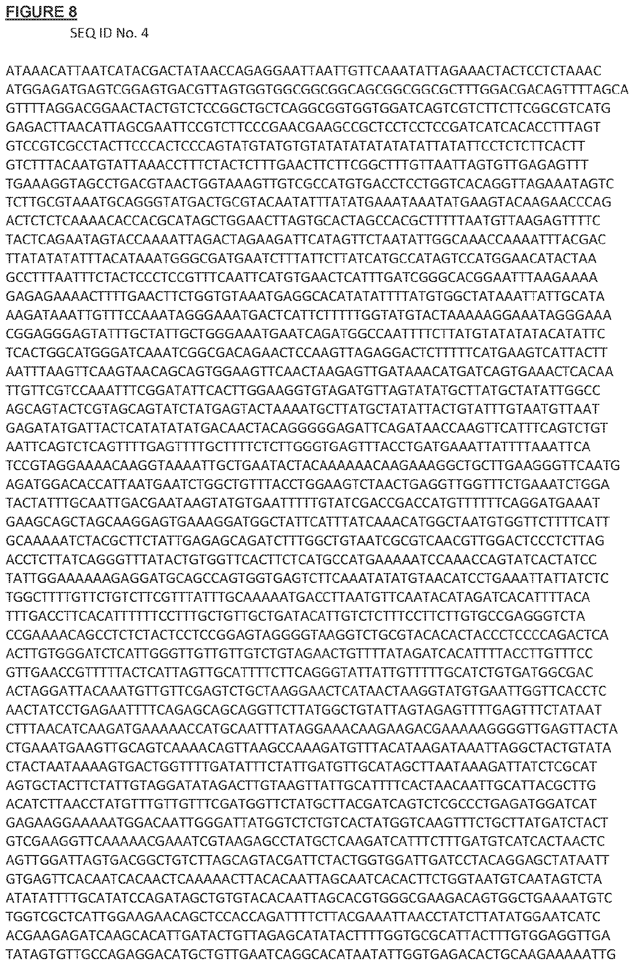

**Figure 8** shows the genomic sequence - SEQ ID No. 4 - encoding a homologous protein from *Nicotiana tabacum.*

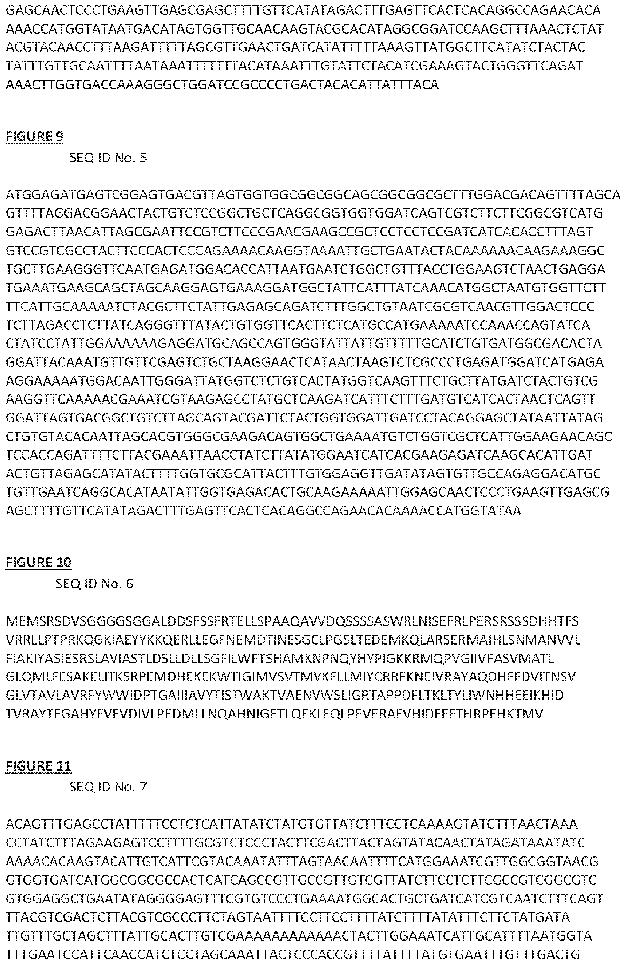

**Figure 9** shows the cDNA sequence - SEQ ID No. 5 - encoding a homologous protein from *Nicotiana tabacum.*
**Figure 10** shows the polypeptide sequence - SEQ ID No. 6 - encoding a homologous protein from *Nicotiana tabacum.*

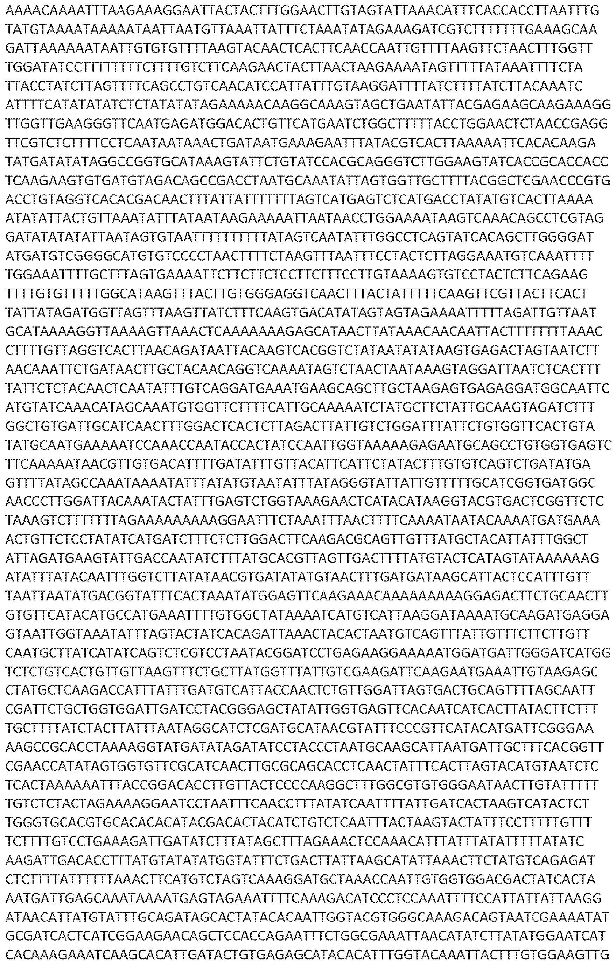

**Figure 11** shows the genomic sequence - SEQ ID No. 7 - encoding a homologous protein from *Nicotiana tabacum.*

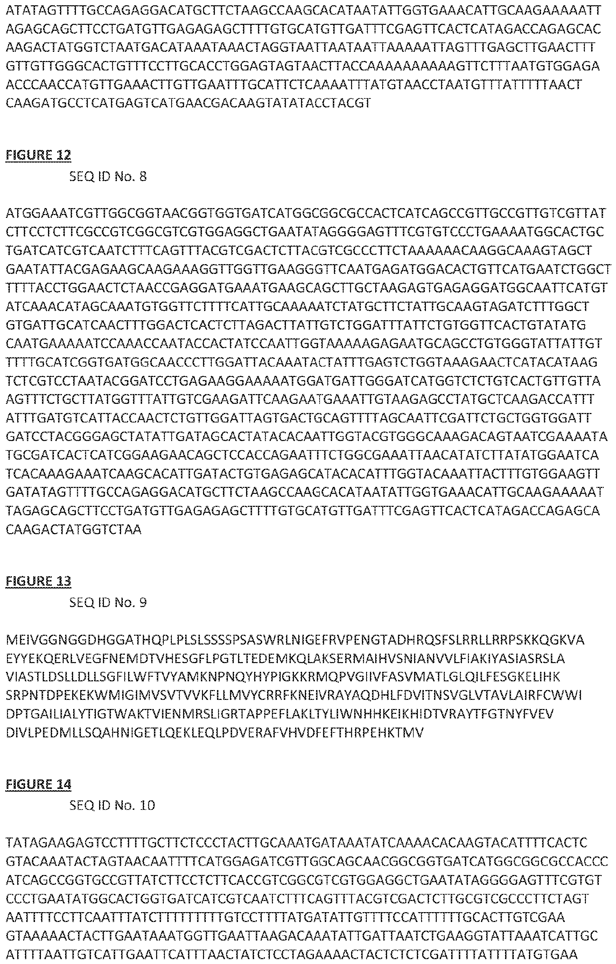

**Figure 12** shows the cDNA sequence - SEQ ID No. 8 - encoding a homologous protein from *Nicotiana tabacum.*
**Figure 13** shows the polypeptide sequence - SEQ ID No. 9 - encoding a homologous protein from *Nicotiana tabacum.*

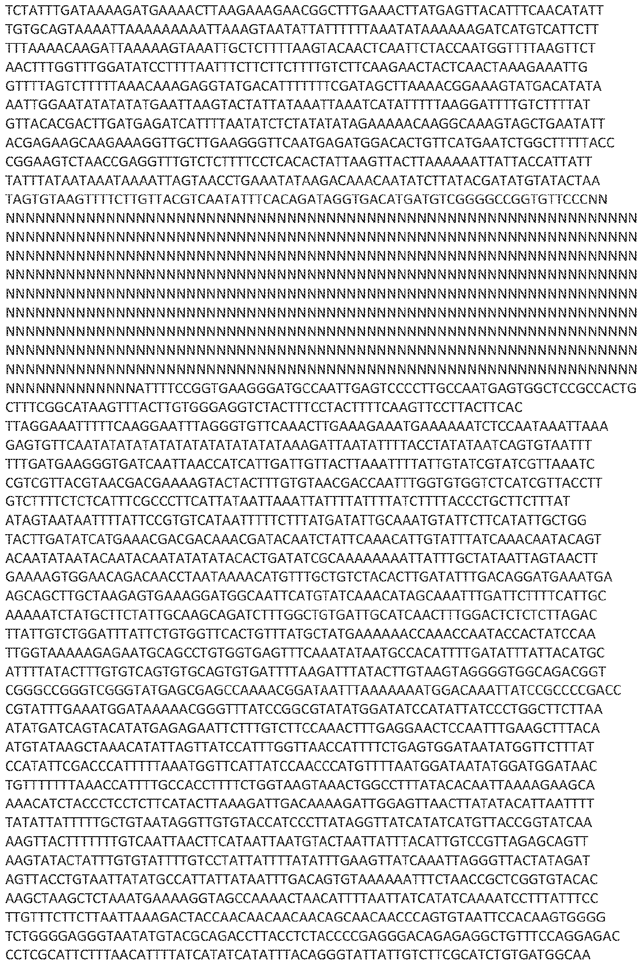

**Figure 14** shows the genomic sequence - SEQ ID No. 10 - encoding a homologous protein from *Nicotiana tabacum.*

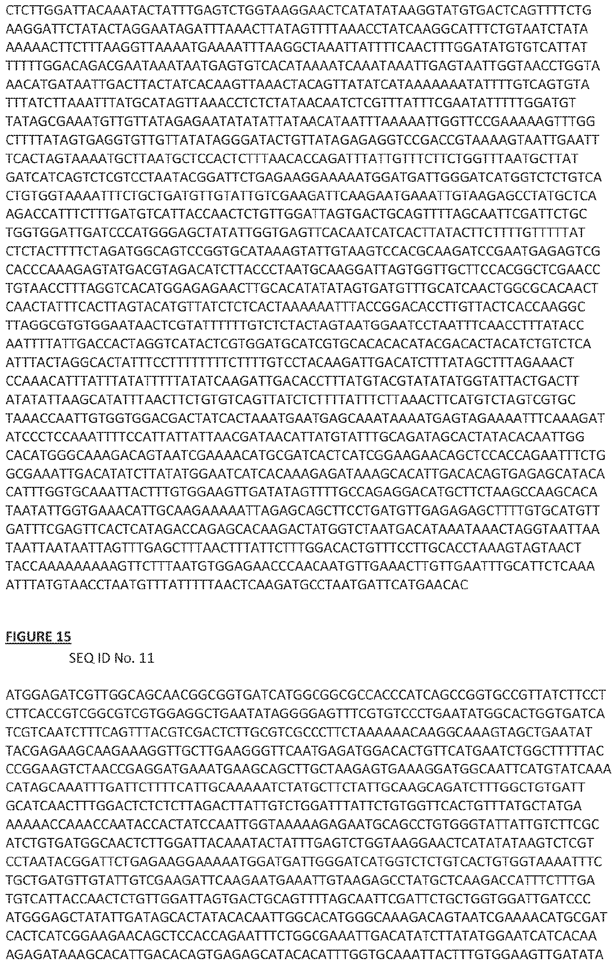

**Figure 15** shows the cDNA sequence - SEQ ID No. 11 - encoding a homologous protein from *Nicotiana tabacum.*

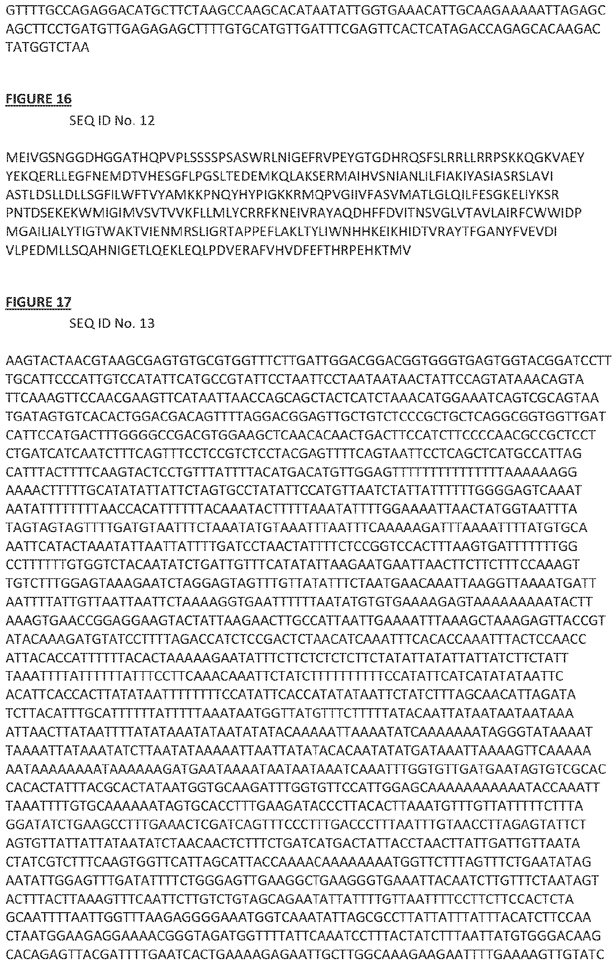

**Figure 16** shows the polypeptide sequence - SEQ ID No. 12 - encoding a homologous protein from *Nicotiana tabacum.*

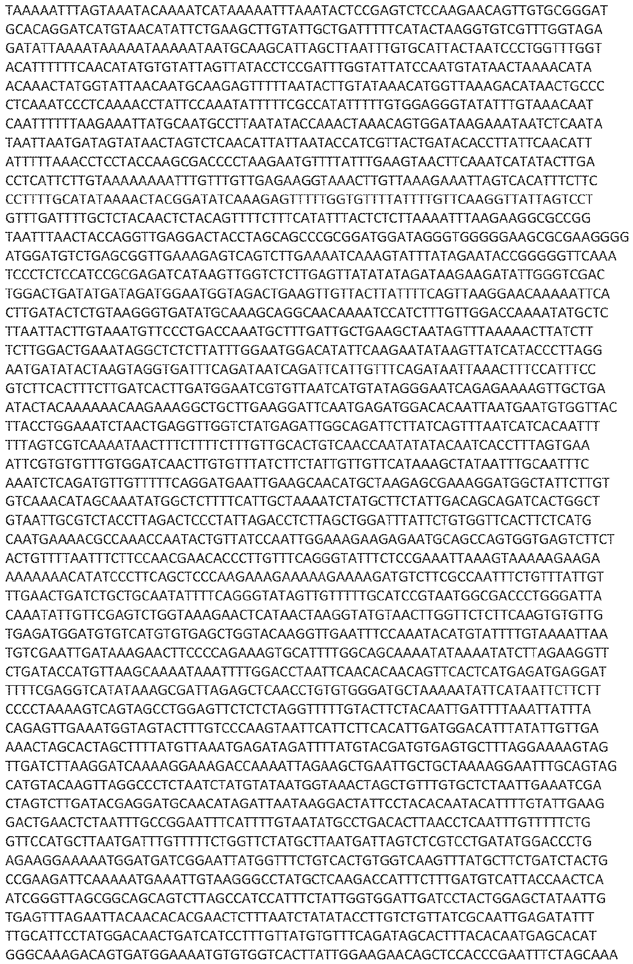

**Figure 17** shows the genomic sequence - SEQ ID No. 13 - encoding a homologous protein from *Nicotiana tabacum.*

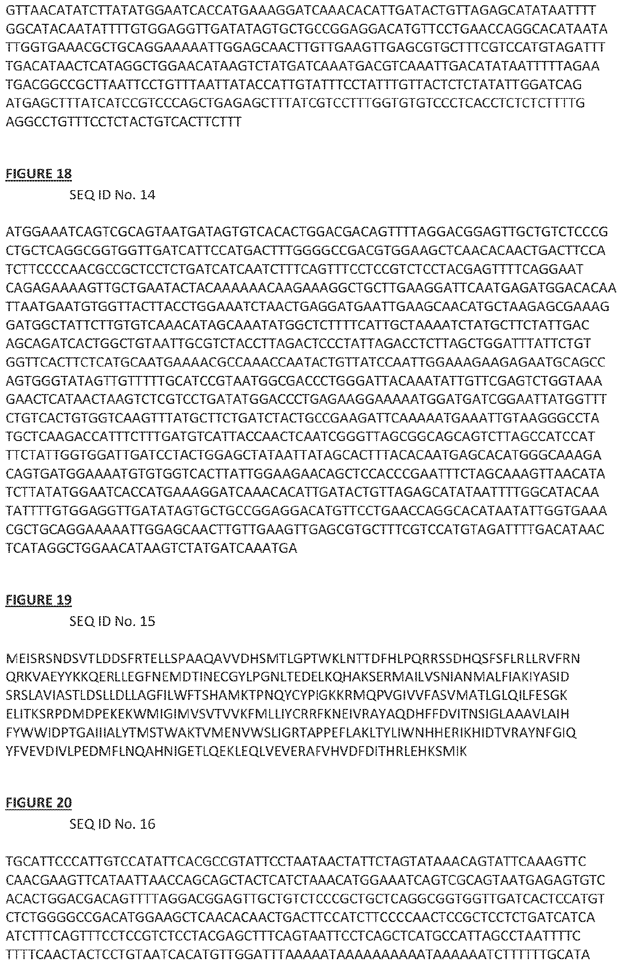

**Figure 18** shows the cDNA sequence - SEQ ID No. 14 - encoding a homologous protein from *Nicotiana tabacum.*
**Figure 19** shows the polypeptide sequence - SEQ ID No. 15 - encoding a homologous protein from *Nicotiana tabacum.*

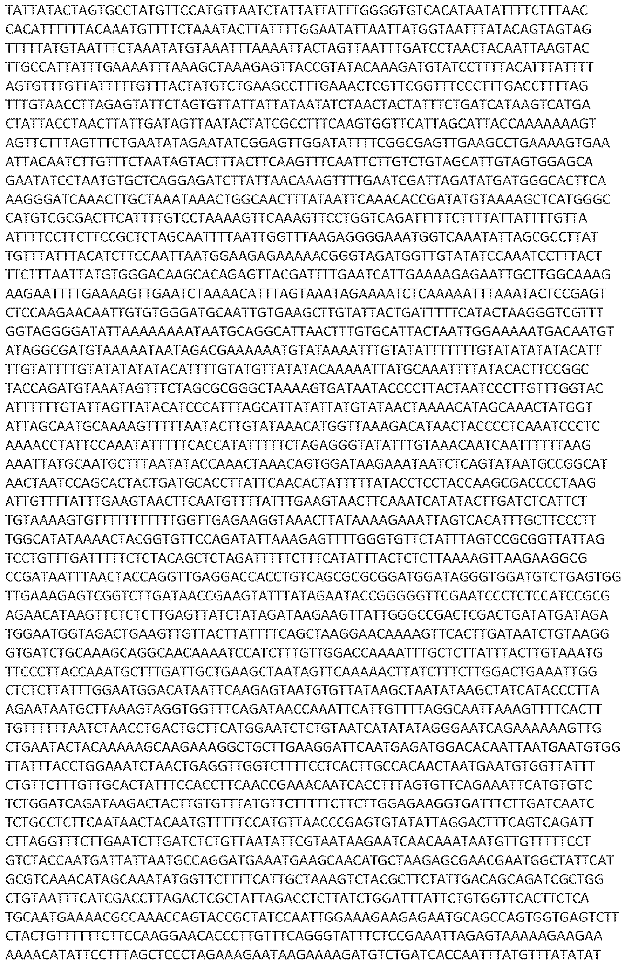

**Figure 20** shows the genomic sequence - SEQ ID No. 16 - encoding a homologous protein from *Nicotiana tabacum.*

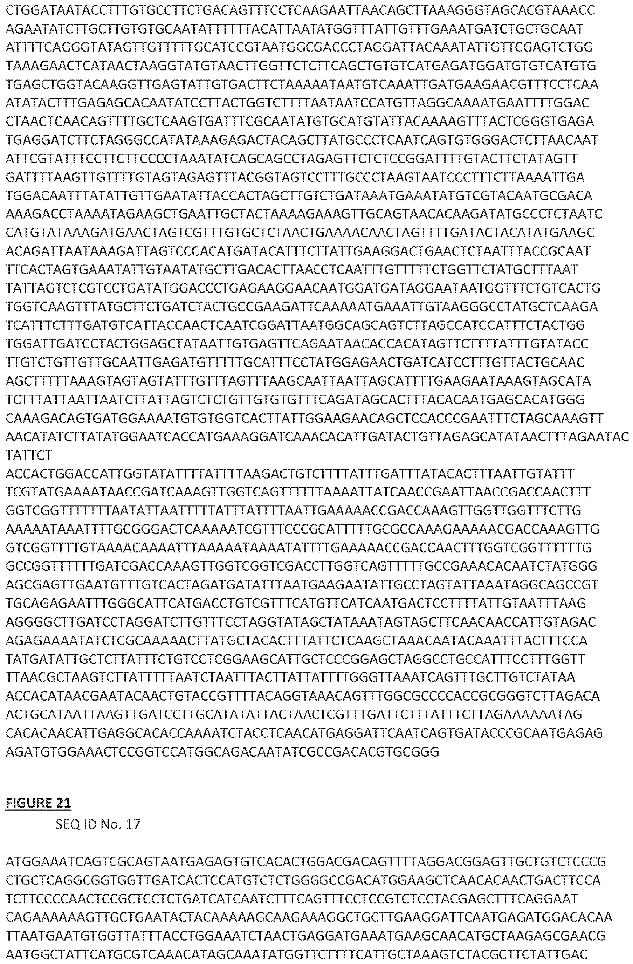

**Figure 21** shows the cDNA sequence - SEQ ID No. 17 - encoding a homologous protein from *Nicotiana tabacum.*

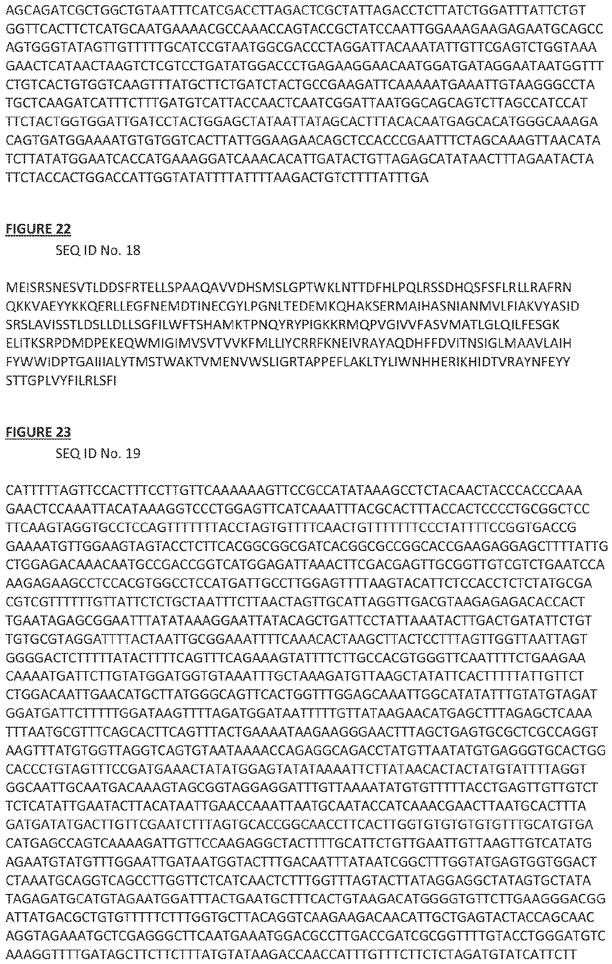

**Figure 22** shows the polypeptide sequence - SEQ ID No. 18 - encoding a homologous protein from *Nicotiana tabacum.*

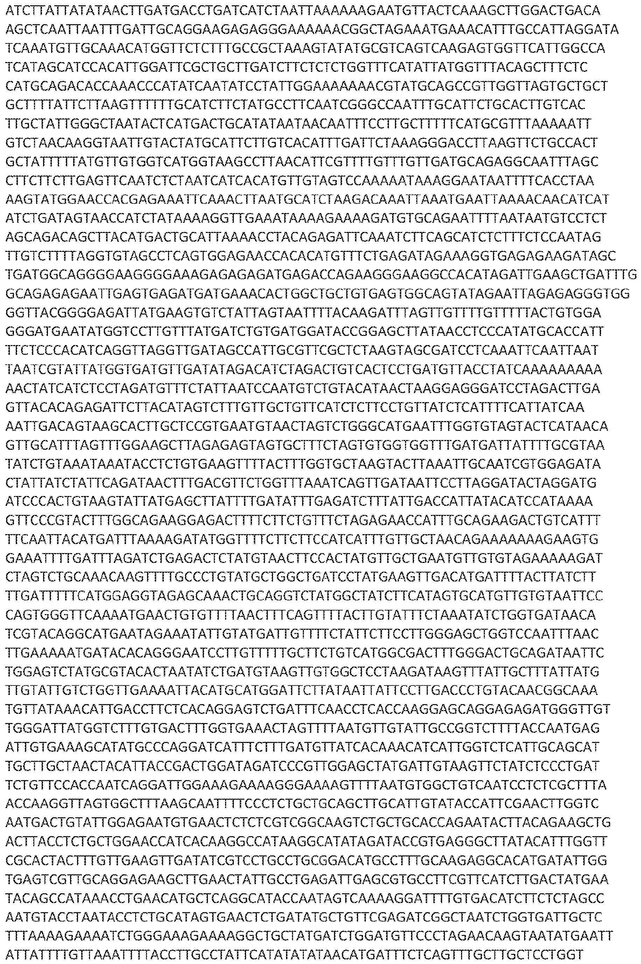

**Figure 23** shows the genomic sequence - SEQ ID No. 19 - encoding a homologous protein from *Nicotiana tabacum.*

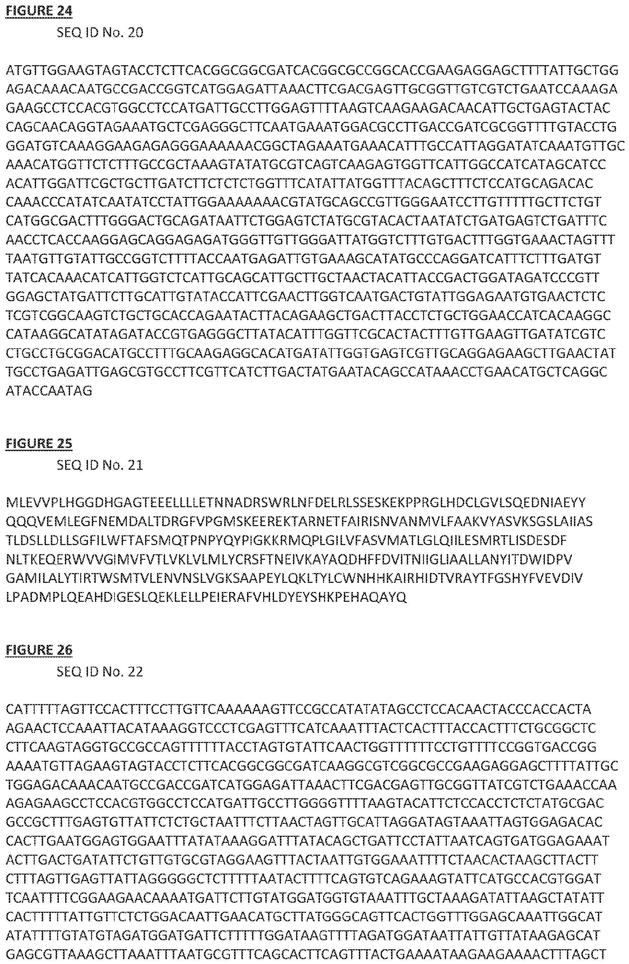

**Figure 24** shows the cDNA sequence - SEQ ID No. 20 - encoding a homologous protein from *Nicotiana tabacum.*
**Figure 25** shows the polypeptide sequence - SEQ ID No. 21 - encoding a homologous protein from *Nicotiana tabacum.*

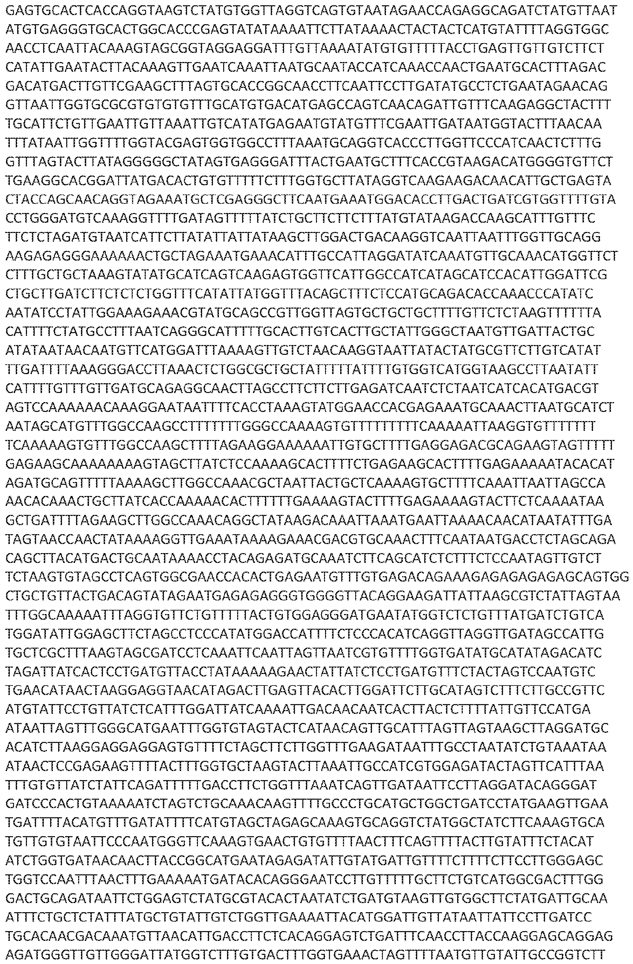

**Figure 26** shows the genomic sequence - SEQ ID No. 22 - encoding a homologous protein from *Nicotiana tabacum.*

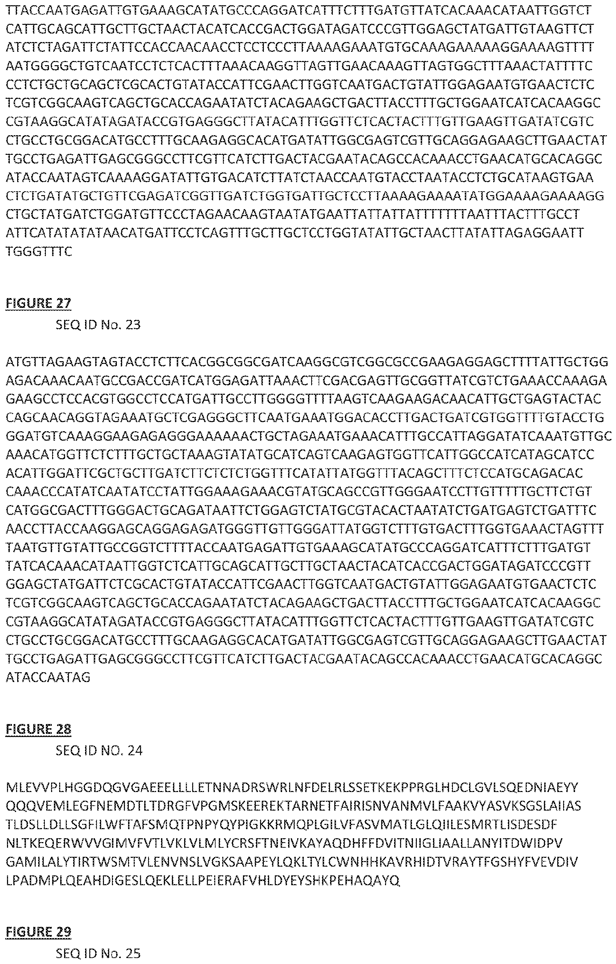

**Figure 27** shows the cDNA sequence - SEQ ID No. 23 - encoding a homologous protein from *Nicotiana tabacum.*
**Figure 28** shows the polypeptide sequence - SEQ ID No. 24 - encoding a homologous protein from *Nicotiana tabacum.*

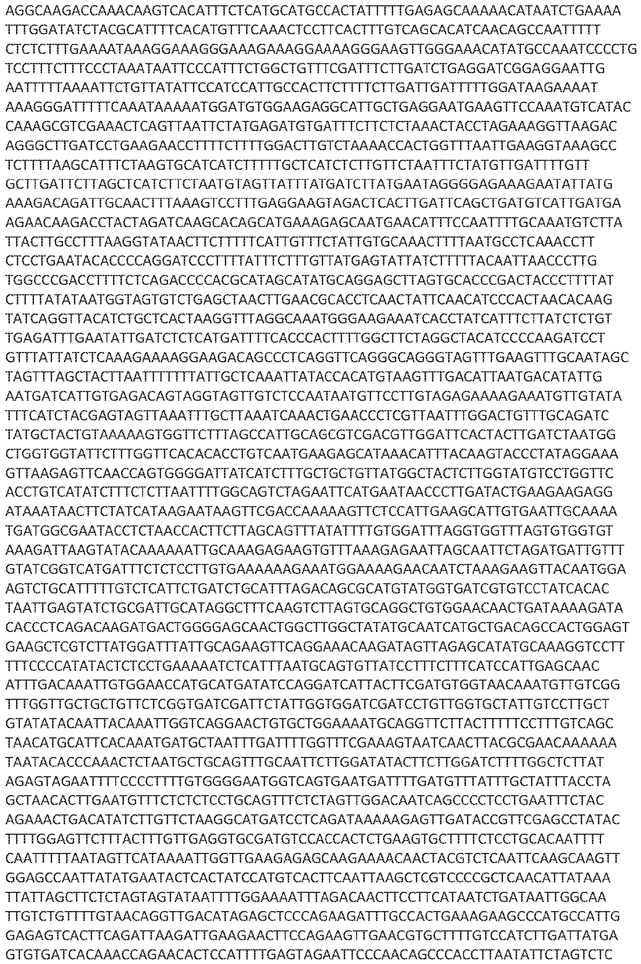

**Figure 29** shows the genomic sequence - SEQ ID No. 25 - encoding a homologous protein from *Nicotiana tabacum.*

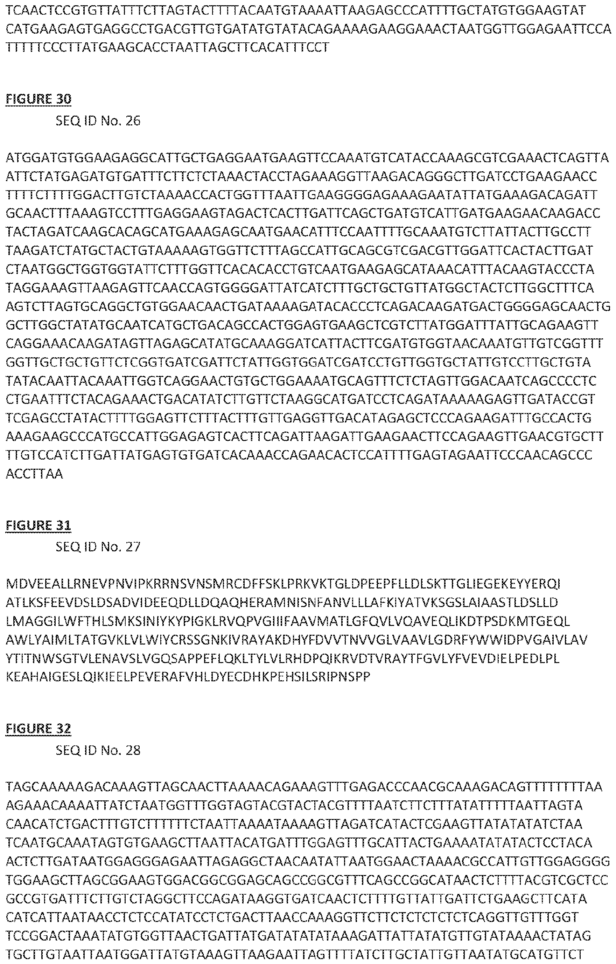

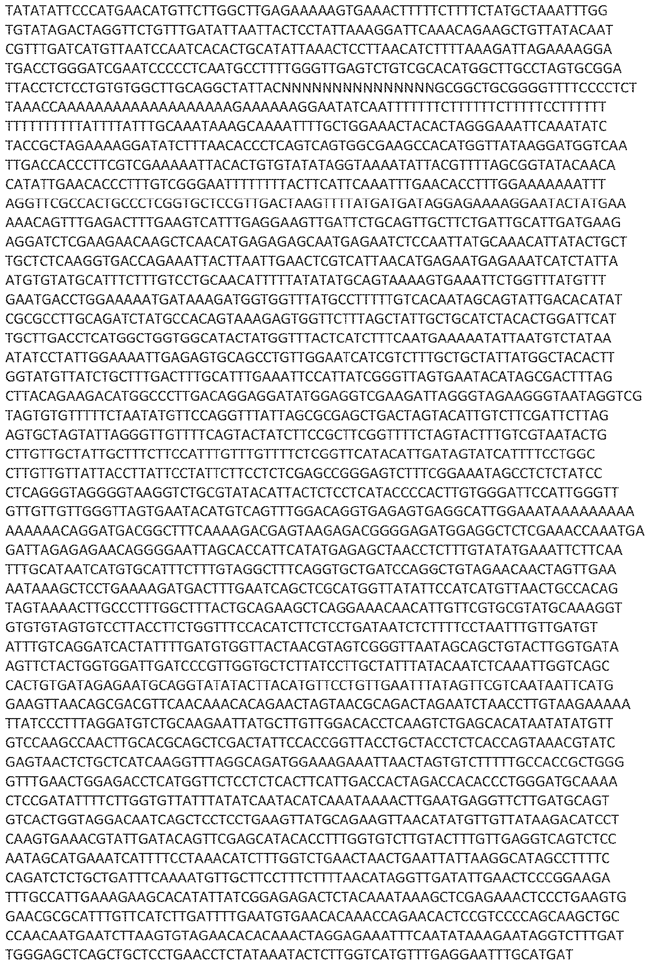

**Figure 30** shows the cDNA sequence - SEQ ID No. 26 - encoding a homologous protein from *Nicotiana tabacum.*
**Figure 31** shows the polypeptide sequence - SEQ ID No. 27 - encoding a homologous protein from *Nicotiana tabacum.*

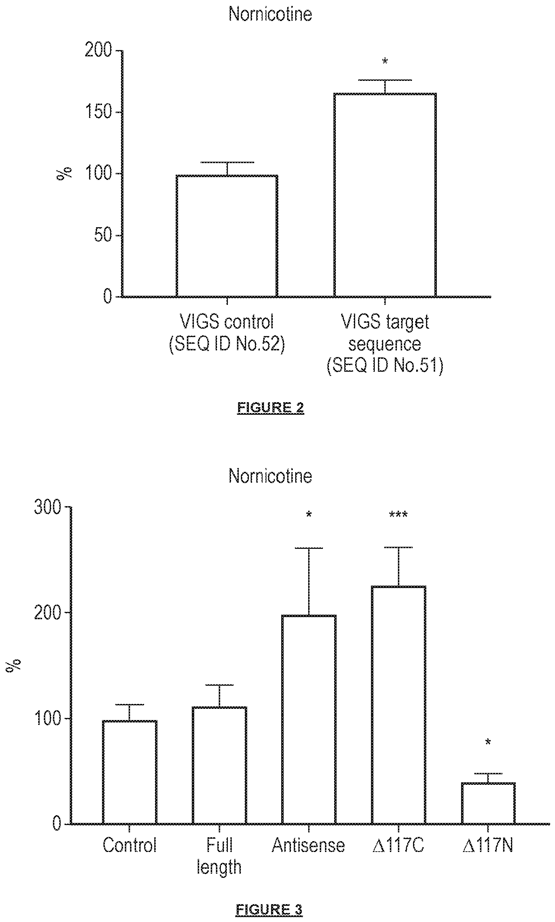

**Figure** 3**2** shows the genomic sequence - SEQ ID No. 28 - encoding a homologous protein from *Nicotiana tabacum.*

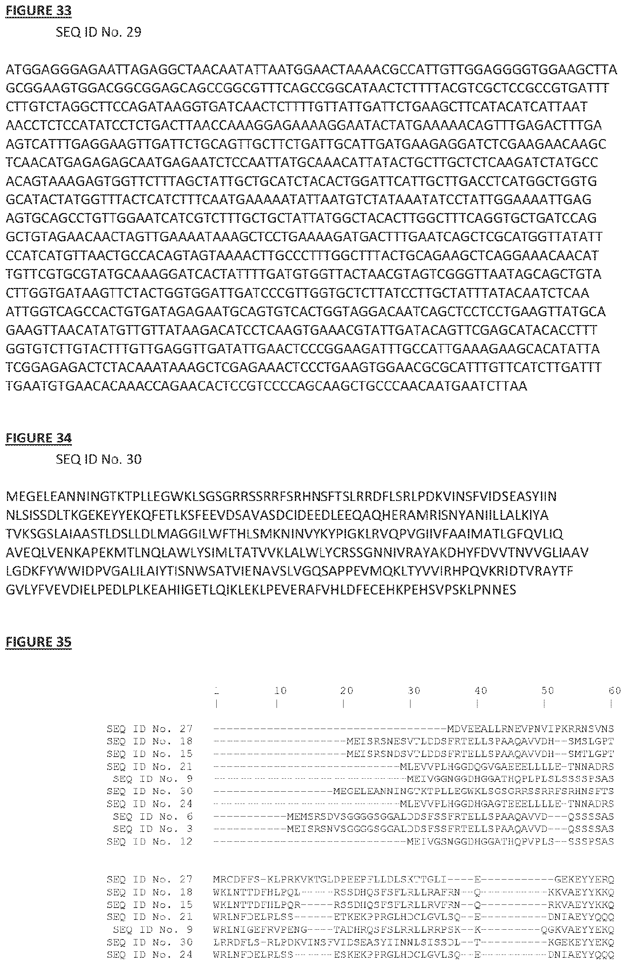

**Figure 33** shows the cDNA sequence - SEQ ID No. 29 - encoding a homologous protein from *Nicotiana tabacum.*
**Figure 34** shows the polypeptide sequence - SEQ ID No. 30 - encoding a homologous protein from *Nicotiana tabacum.*

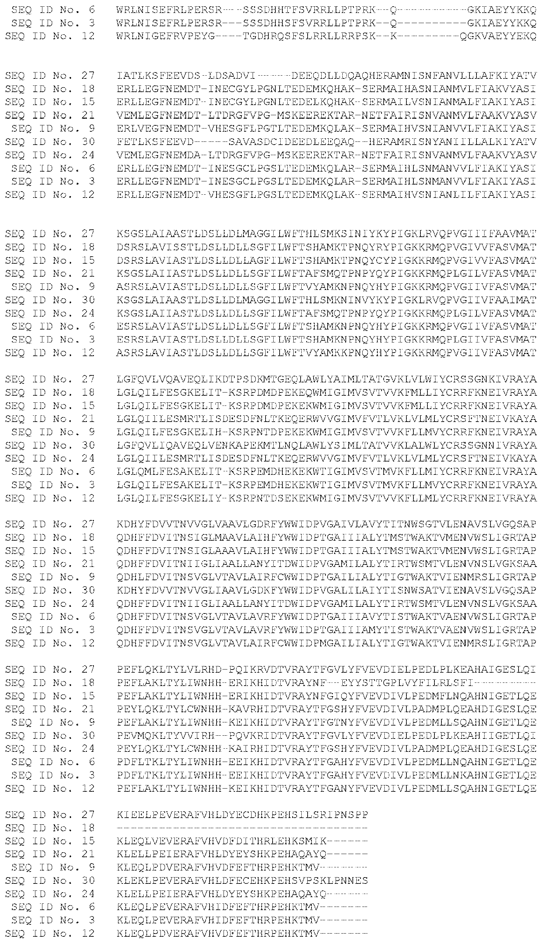

**Figure 35** shows an alignment SEQ ID No. 3 and homologous protein sequences from *Nicotiana tabacum.*

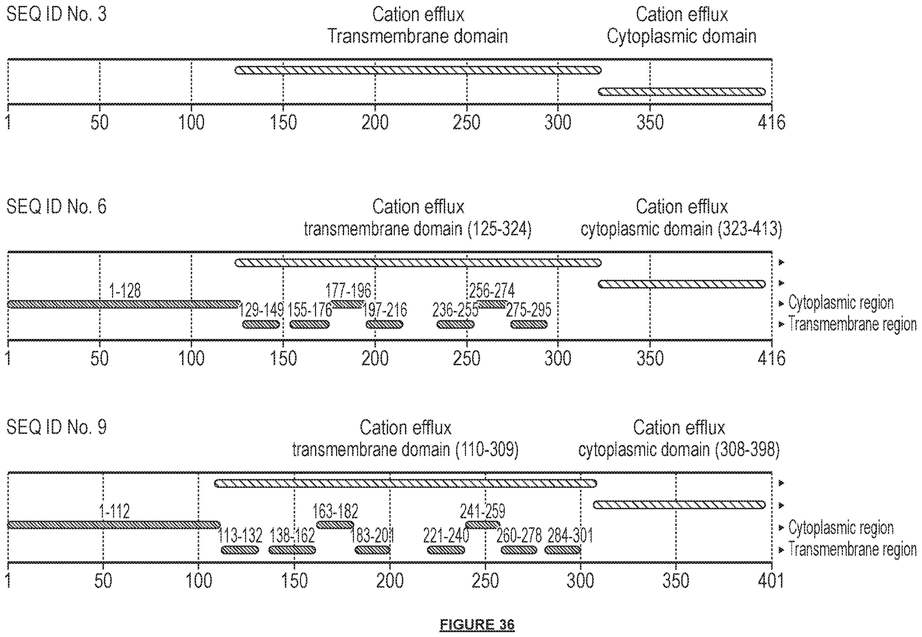

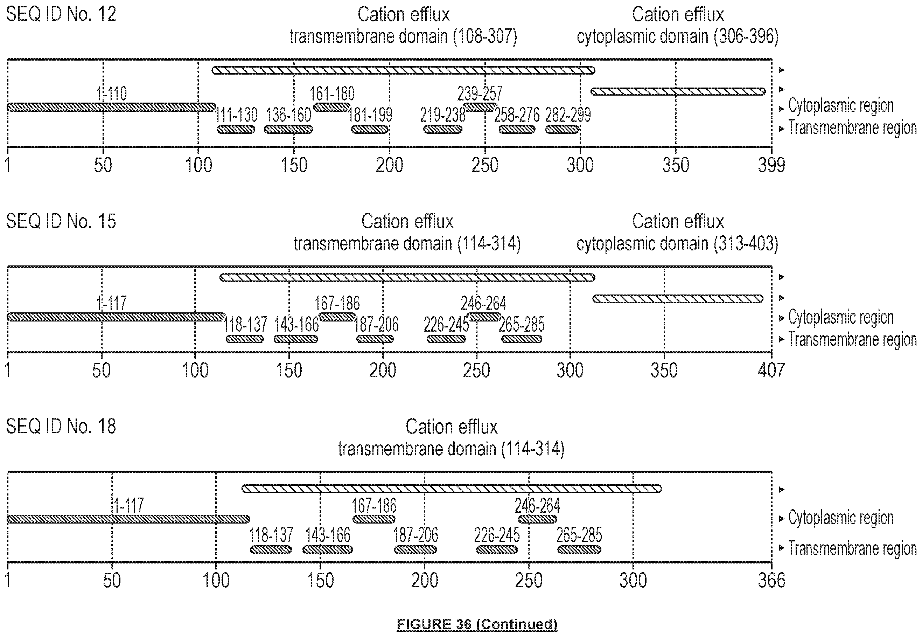

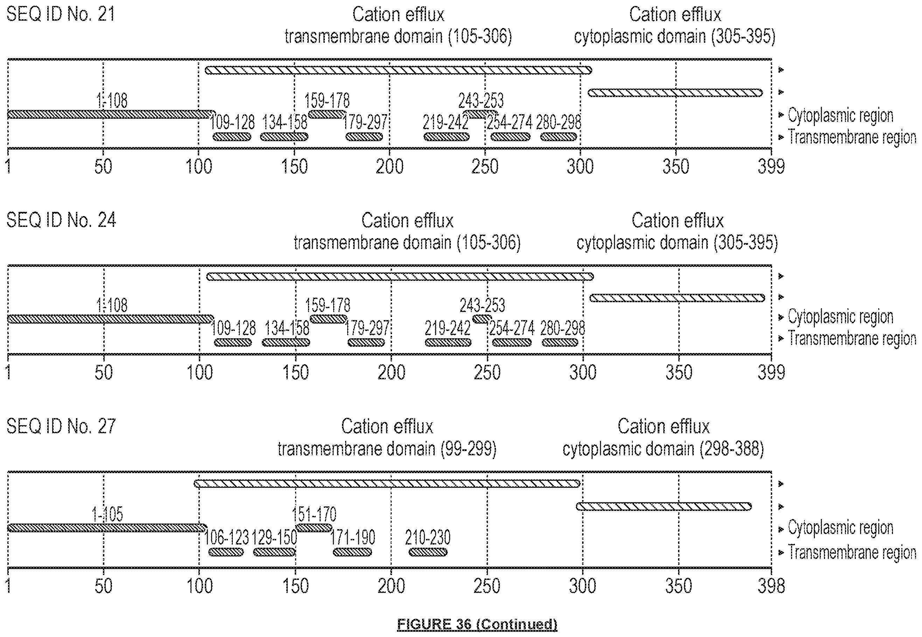

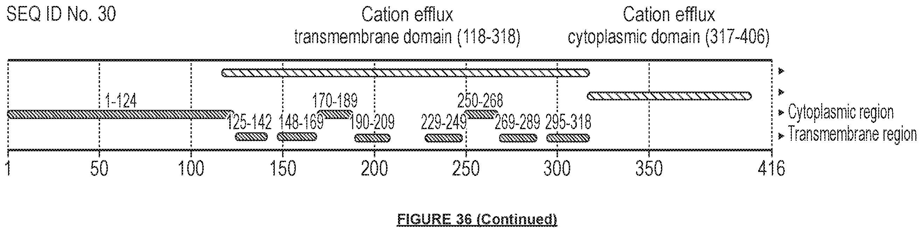

**Figure 36** shows the transmembrane domain and the cytoplasmic domain of SEQ ID No. 3 and of homologous protein sequences from *Nicotiana tabacum.* The protein sequence of SEQ ID No. 3 indicates the presence of a cation efflux transmembrane domain (amino acid positions 125-324) and a cation efflux cytoplasmic domain (amino acid positions 323-413).

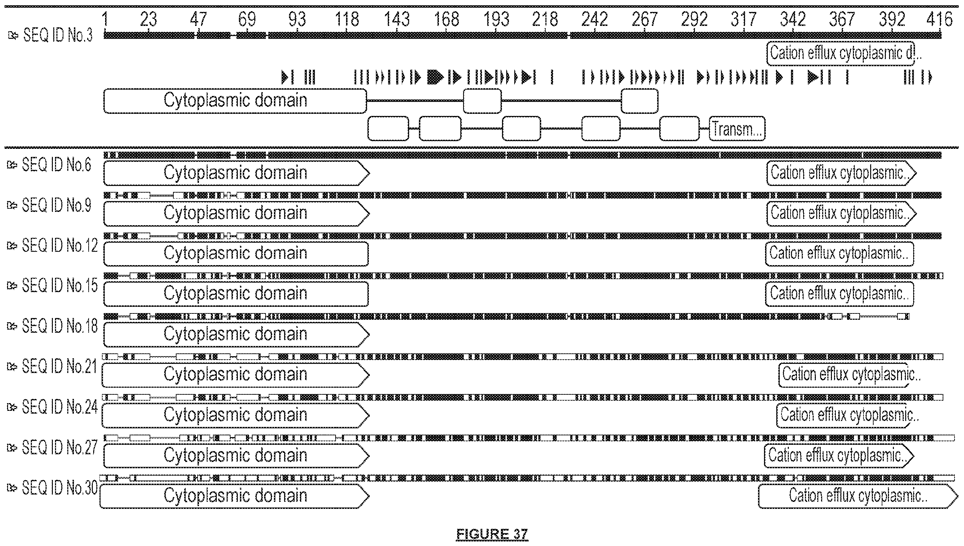

**Figure 37** shows an alignment of SEQ ID No. 3 and homologous protein sequences from *Nicotiana tabacum.*

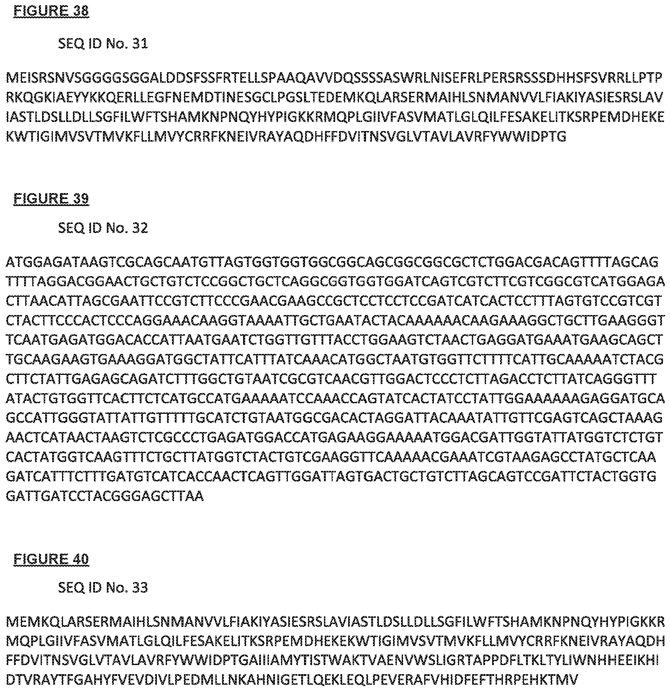

**Figure 38** shows a polypeptide sequence of a mutated cation efflux protein based on SEQ ID No. 3, wherein the last 117 amino acids of the C-terminal domain have been deleted (SEQ ID No. 31).
**Figure 39** shows the cDNA sequence of a mutated cation efflux protein based on SEQ ID No. 3, wherein the last 117 amino acids of the C-terminal domain have been deleted (SEQ ID No. 32).
**Figure 40** shows a polypeptide sequence of a mutated cation efflux protein based on SEQ ID No. 3, wherein the first 117 amino acids (i.e. from the N-terminal domain) have been deleted (SEQ ID No. 33).

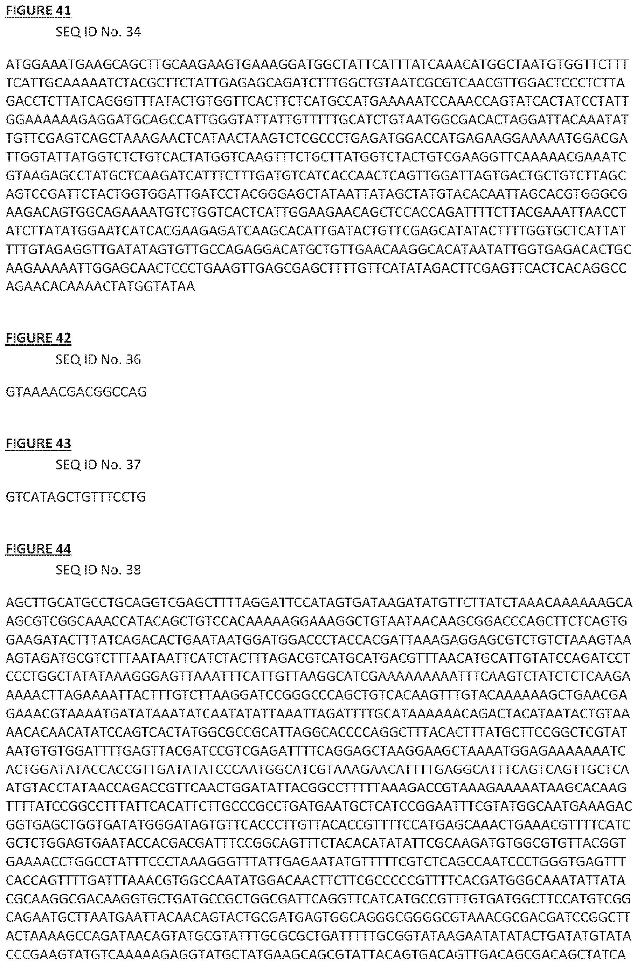

**Figure 41** shows the cDNA sequence of a mutated cation efflux protein based on SEQ ID No. 3, wherein the first 117 amino acids (i.e. from the N-terminal domain) have been deleted (SEQ ID No. 34).
**Figure 42** shows the sequence of primer M13.fw (SEQ ID No. 36).
**Figure 43** shows the sequence of primer M13.rv (SEQ ID No. 37).

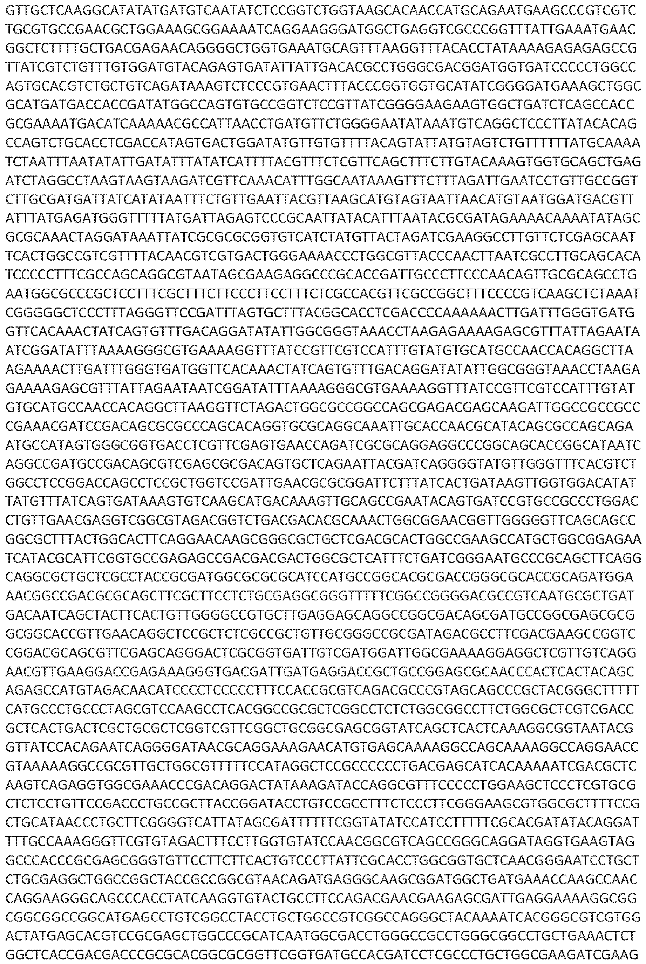

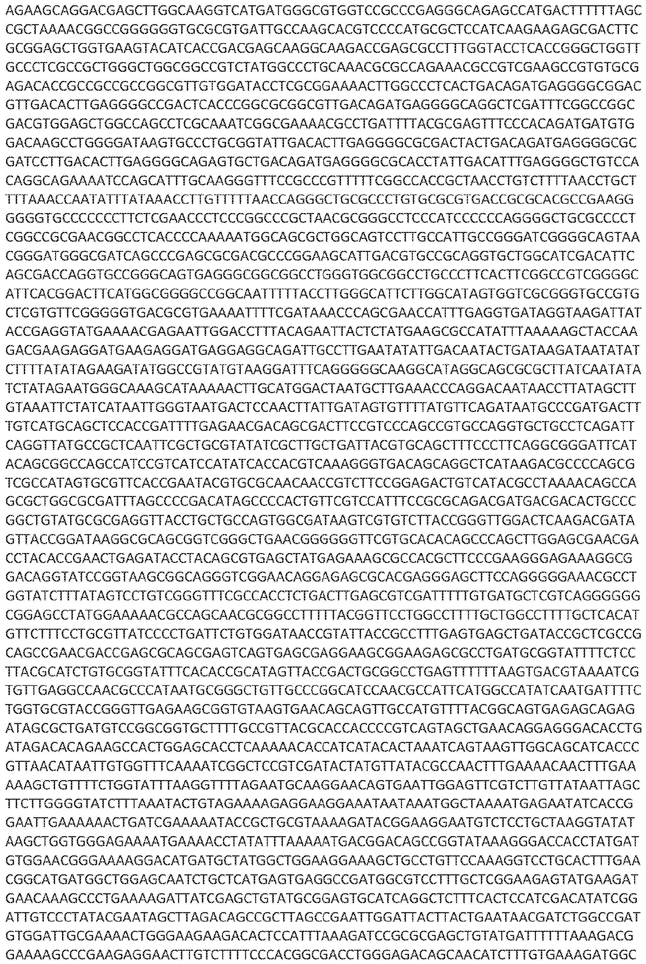

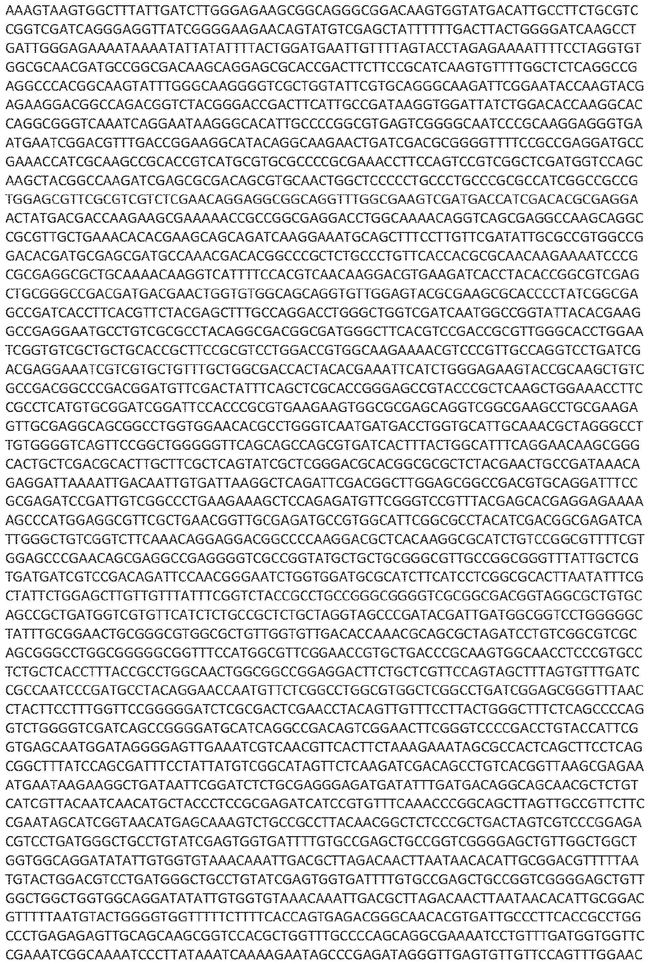

**Figure 44** shows the sequence of the Gateway Destination Binary (GDB) expression vector (SEQ ID No. 38).

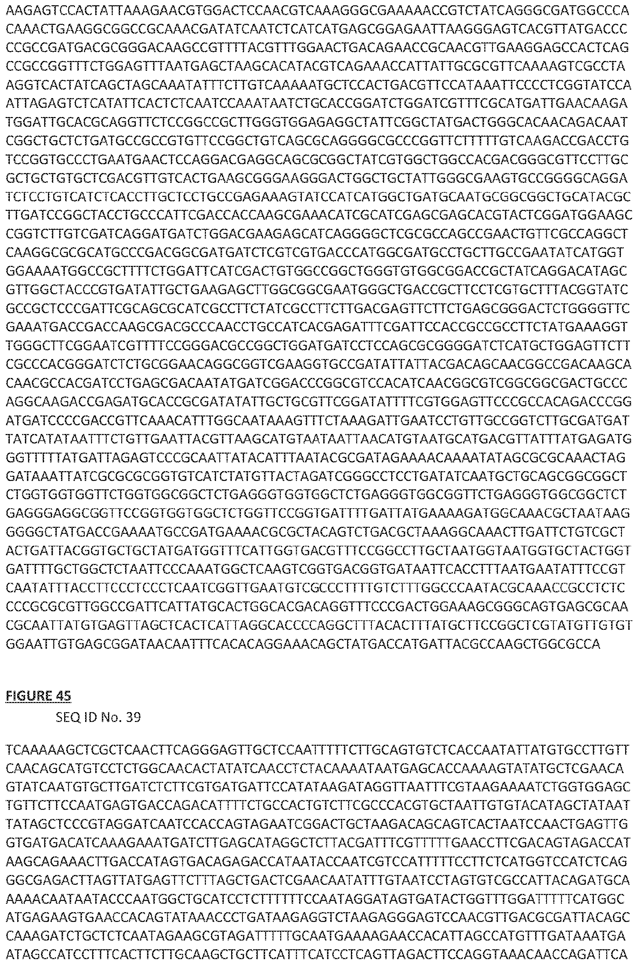

**Figure 45** shows the sequence of cation efflux antisense (AS) (SEQ ID No. 39).

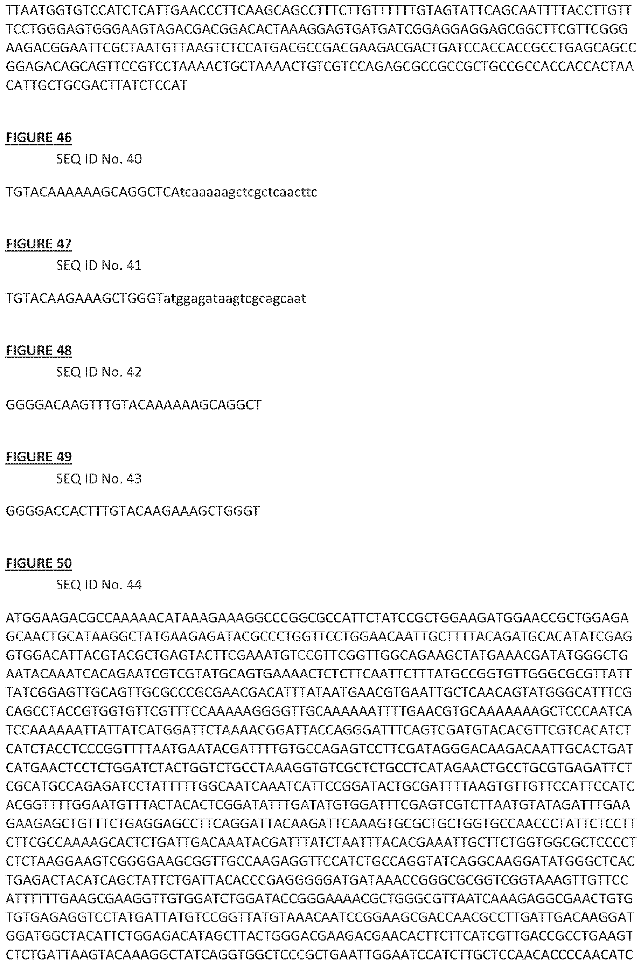

**Figure 46** shows the sequence of primer AS.fw (SEQ ID No. 40).
**Figure 47** shows the sequence of primer AS.rv (SEQ ID No. 41)
**Figure 48** shows the sequence of primer attB1 (SEQ ID No. 42).
**Figure 49** shows the sequence of primer attB2 (SEQ ID No. 43).
**Figure 50** shows the sequence of the luciferase control sequence (SEQ ID No. 44).

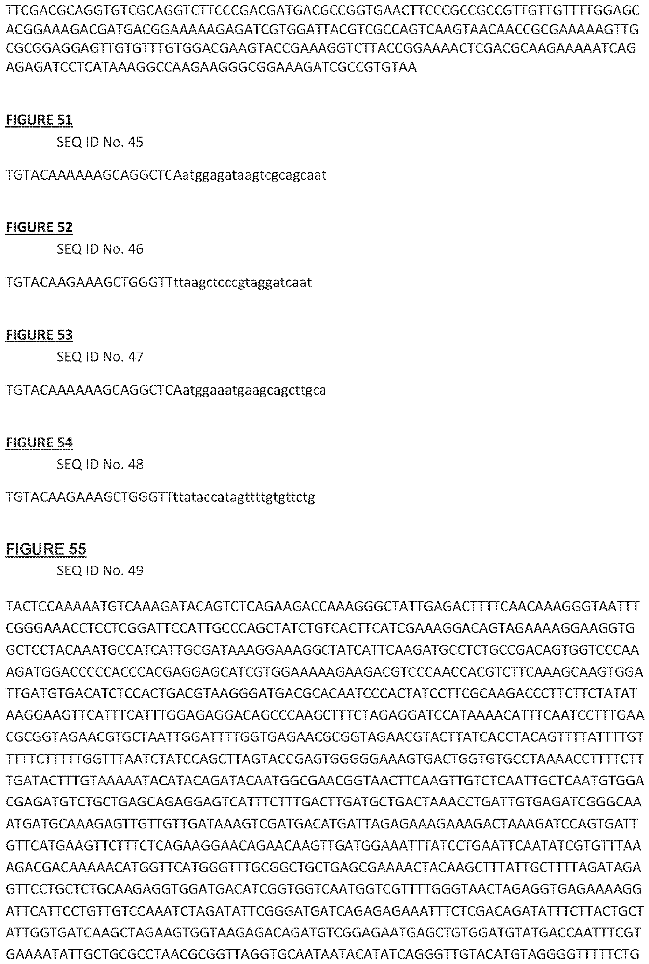

**Figure 51** shows the sequence of primer delta 117C fw (SEQ ID No. 45).
**Figure 52** shows the sequence of primer delta 117C rv (SEQ ID No. 46).
**Figure 53** shows the sequence of primer delta 117N fw (SEQ ID No. 47).
**Figure 54** shows the sequence of primer delta 117N rv (SEQ ID No. 48).

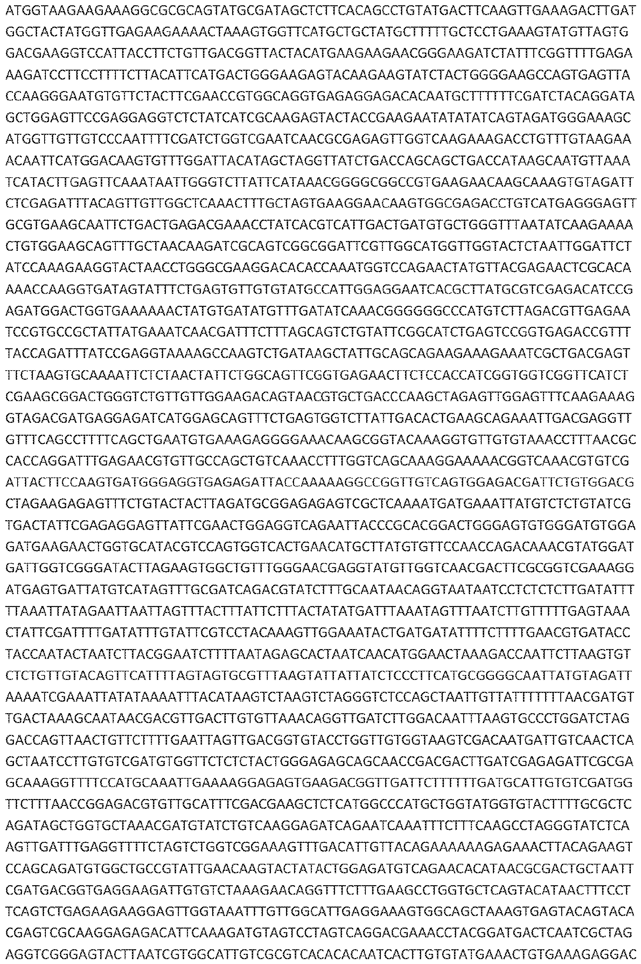

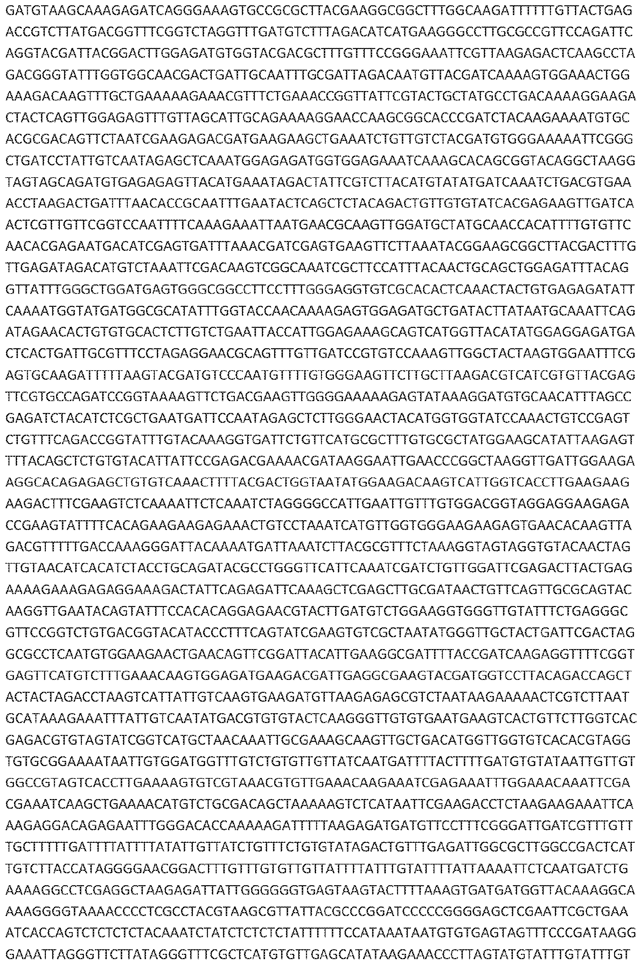

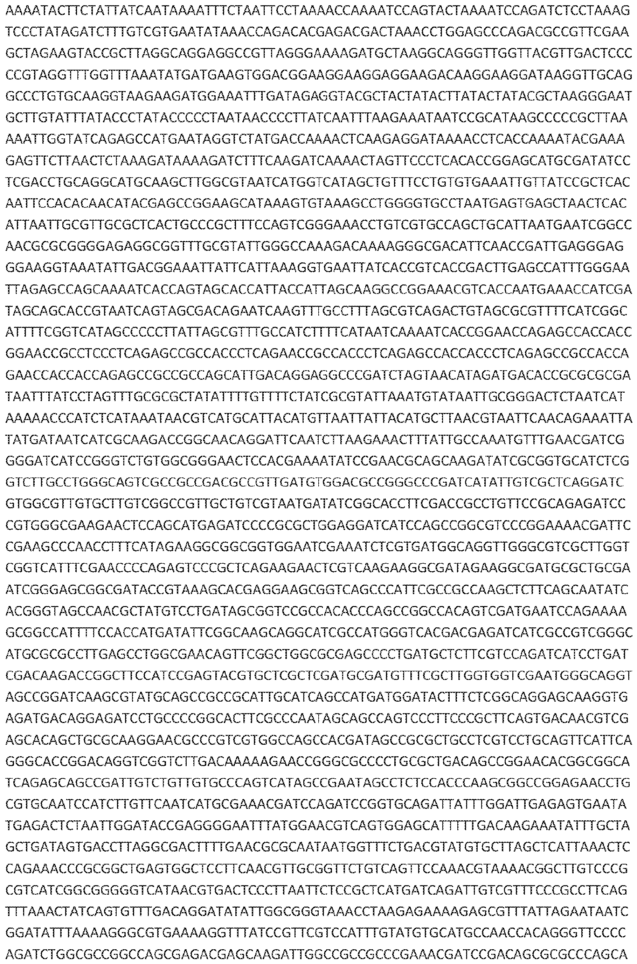

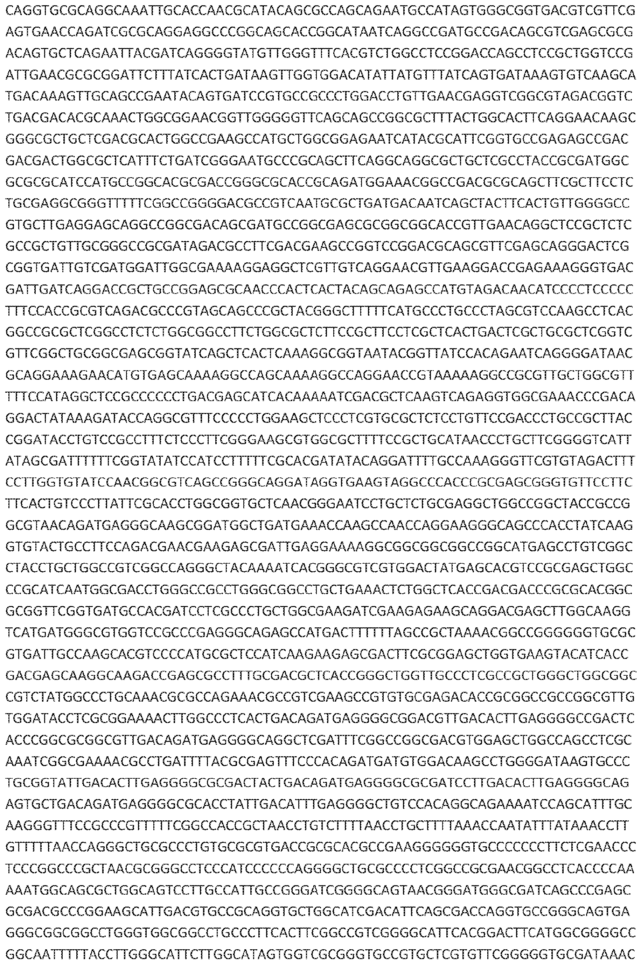

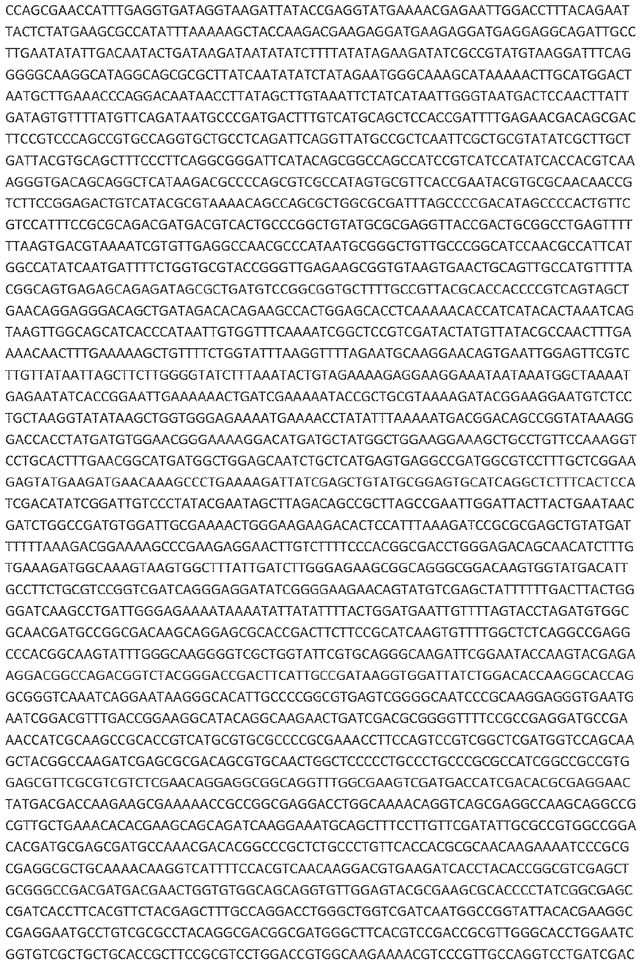

**Figure 55** shows the sequence of tobacco rattle virus (TRV) RNA1 (SEQ ID No. 49).

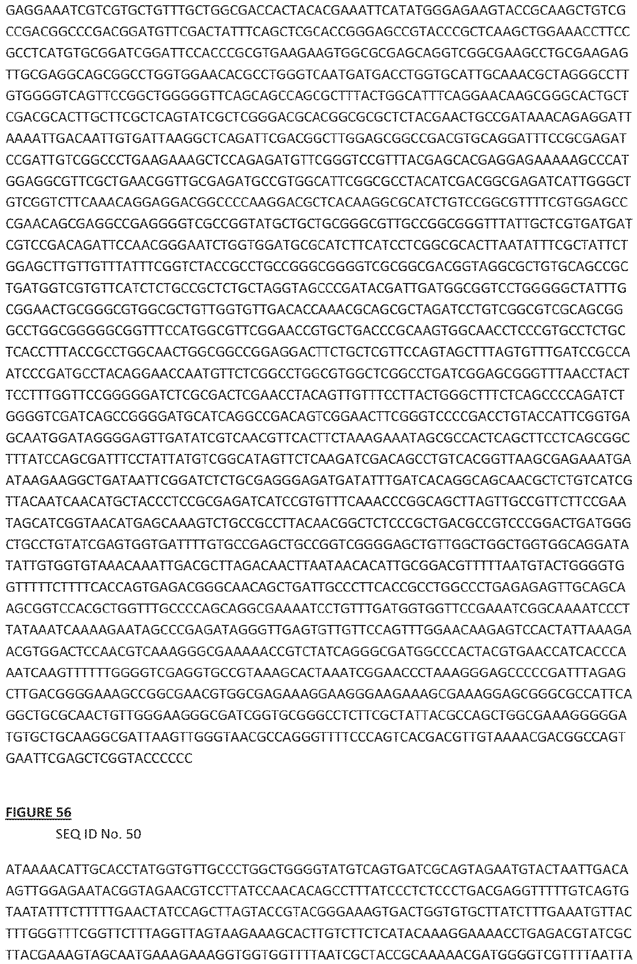

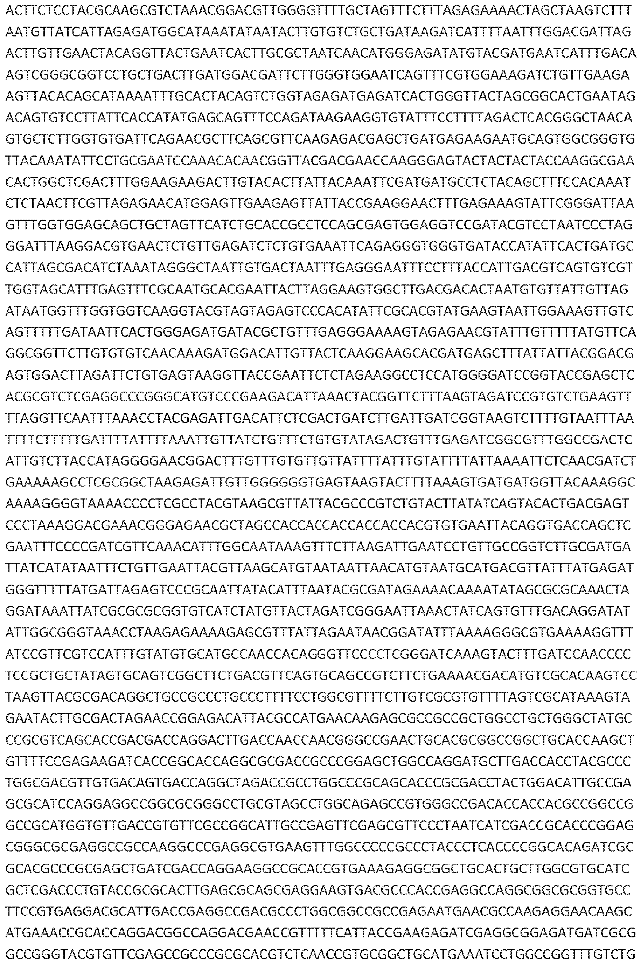

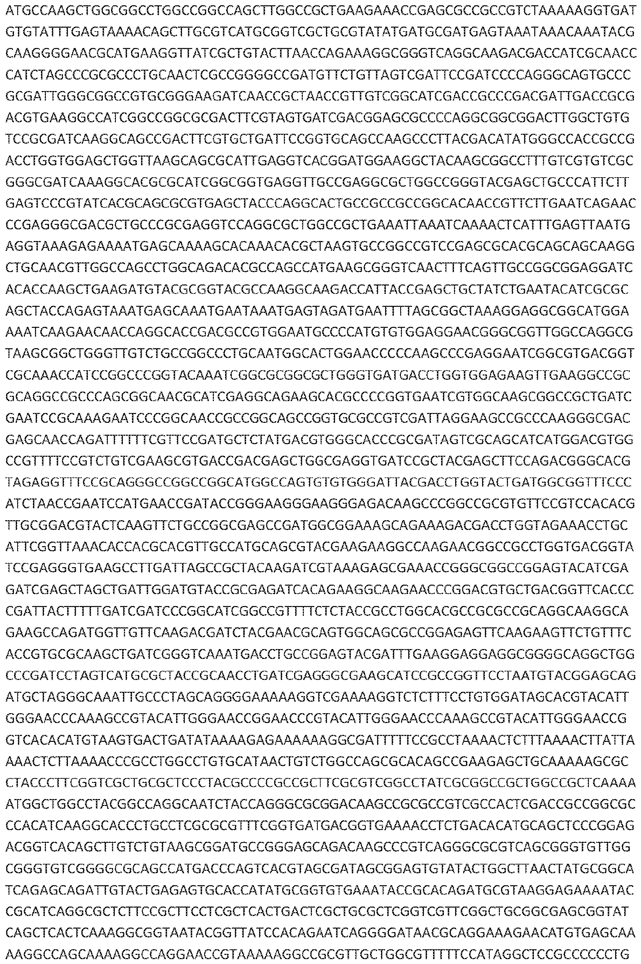

**Figure 56** shows the sequence of TRV RNA2 (SEQ ID No. 50).

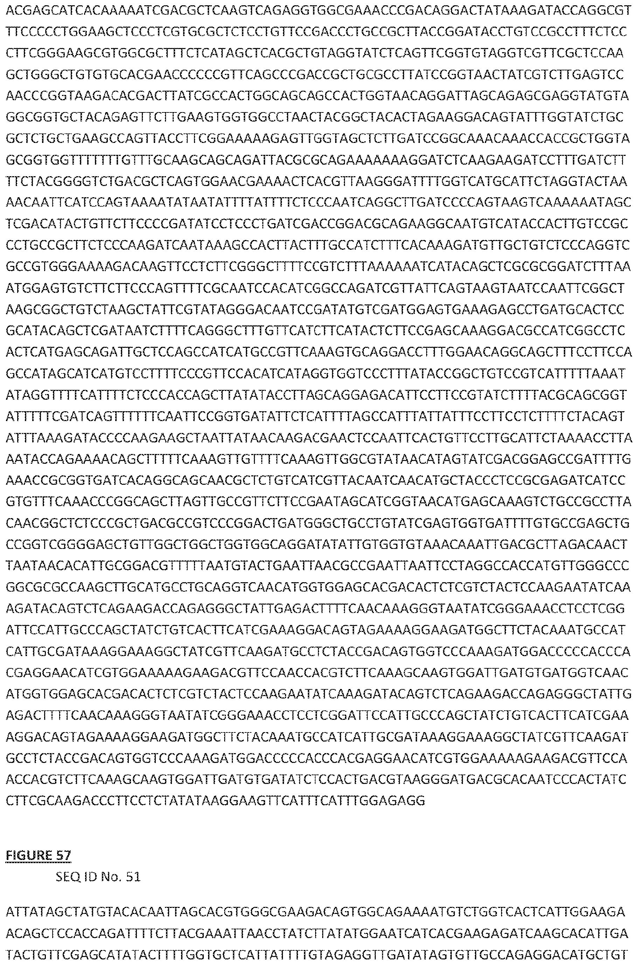

**Figure 57** shows the sequence of the virus-induced gene silencing (VIGS) target sequence (SEQ ID No. 51).

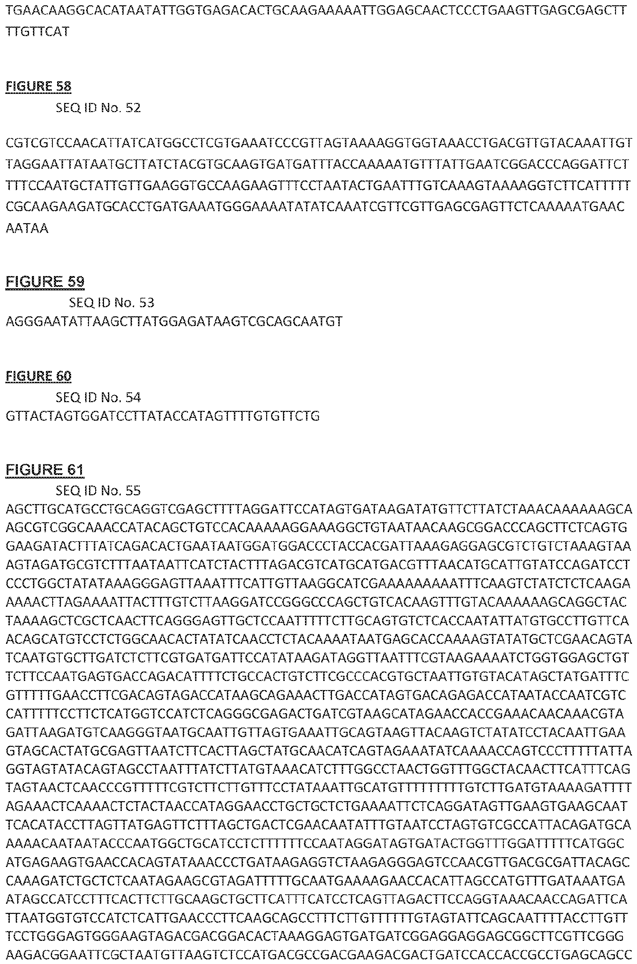

**Figure 58** shows the sequence of the VIGS control sequence (SEQ ID No. 52).
**Figure 59** shows the sequence of yeast Fw primer (SEQ ID No. 53) used in Example 5.
**Figure 60** shows the sequence of yeast Rv primer (SEQ ID No. 54) used in Example 5.

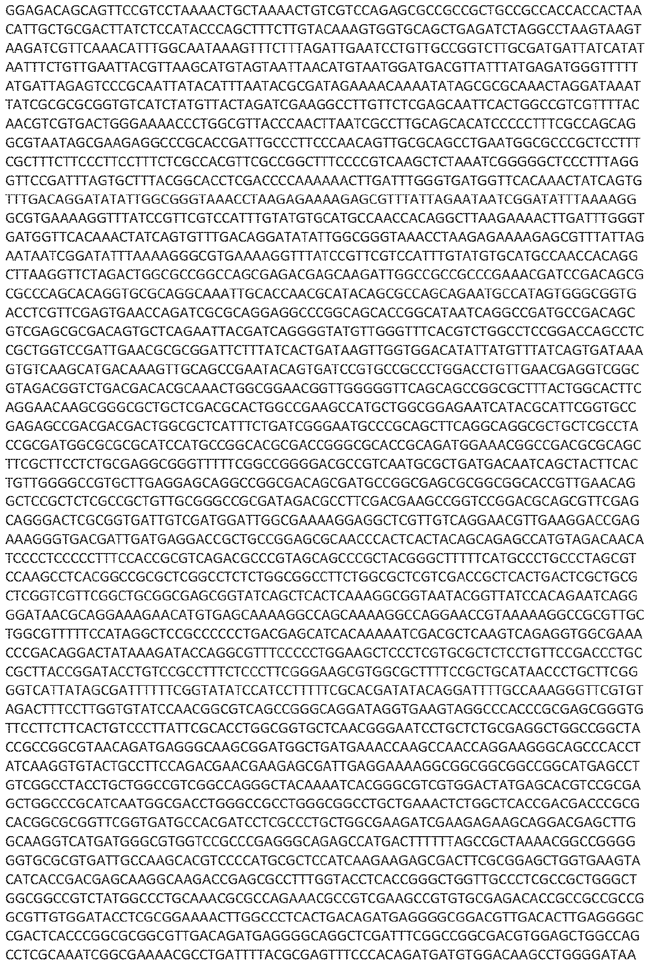

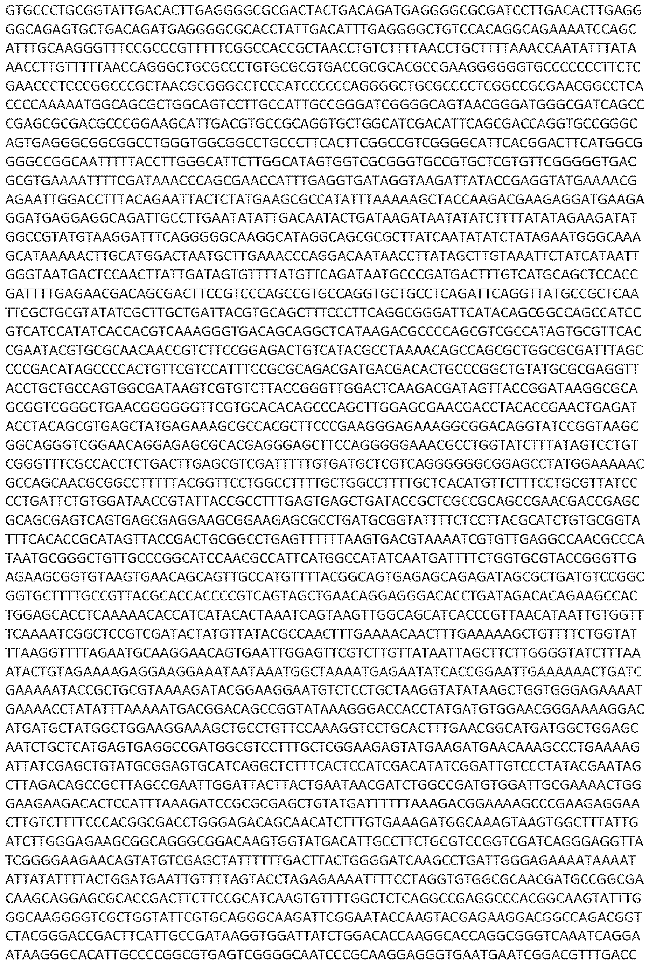

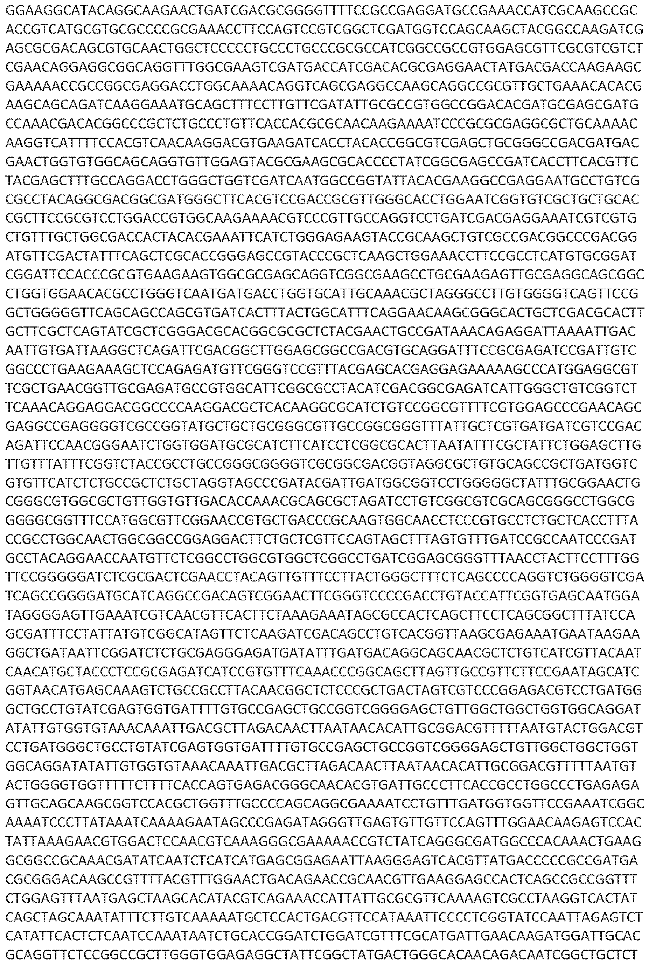

**Figure 61** shows the sequence of the modified pTES3 vector pYES3/UT.1 (SEQ ID No. 55) used in Example 5.

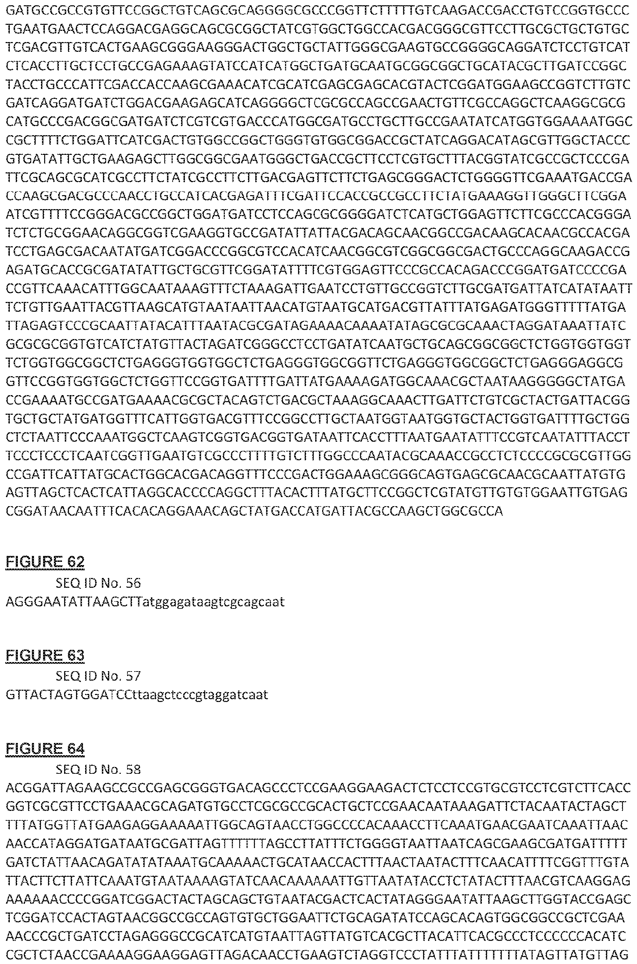

**Figure 62** shows the sequence of the Δ117C Fw primer (SEQ ID No. 56) used in Example 5.
**Figure 63** shows the sequence of the Δ117C Rv primer (SEQ ID No. 57) used in Example 5.

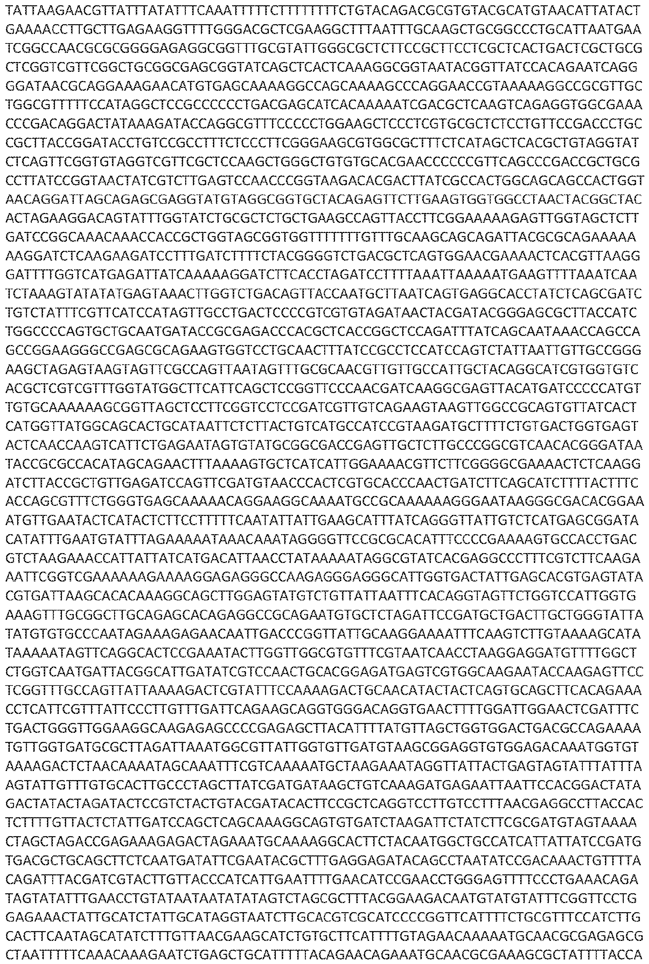

**Figure 64** shows the sequence of the pTES3 vector pYES3/UT.2 (SEQ ID No. 58) used in Example 5.

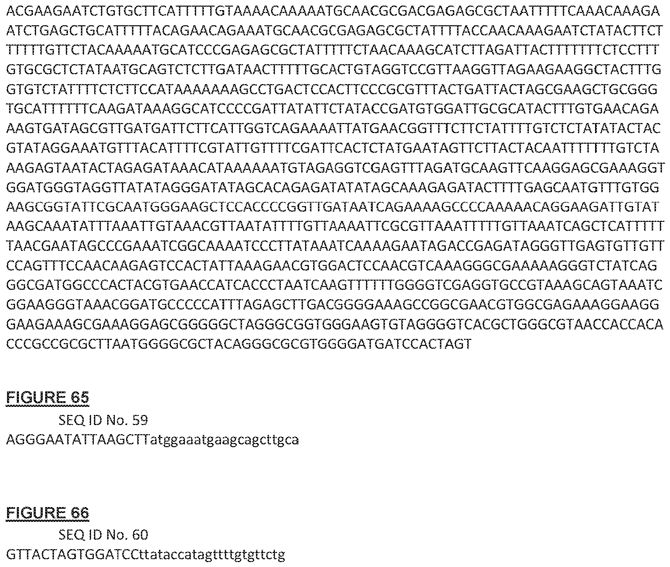

**Figure 65** shows the sequence of the Δ117N Fw primer (SEQ ID No. 59) used in Example 5.
**Figure 66** shows the sequence of the Δ117N Rv primer (SEQ ID No. 60) used in Example 5.

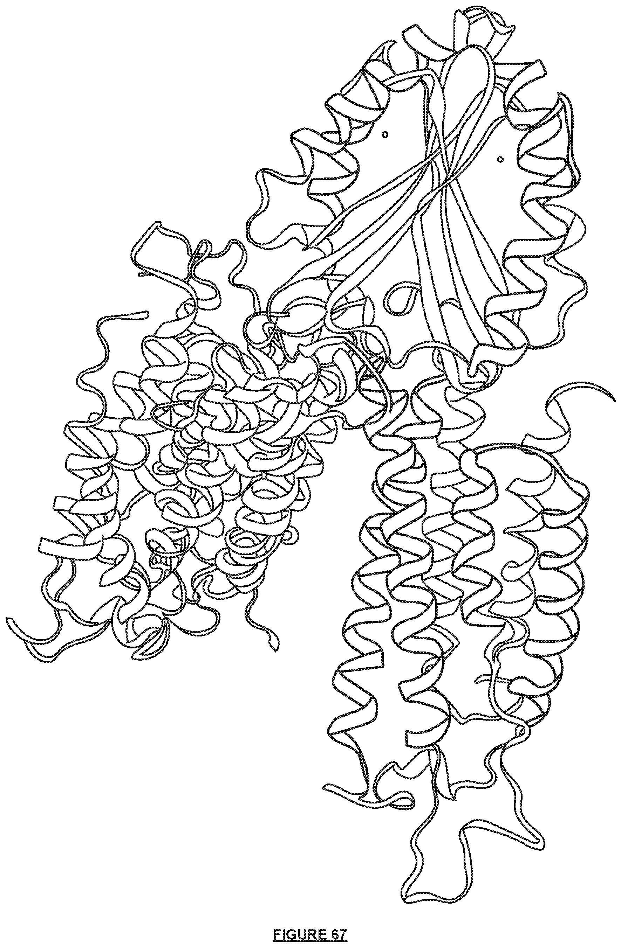

**Figure 67** shows the predicted 3D structure of SEQ ID No.3. The homodimer is predicted to contain two zinc docking sites.

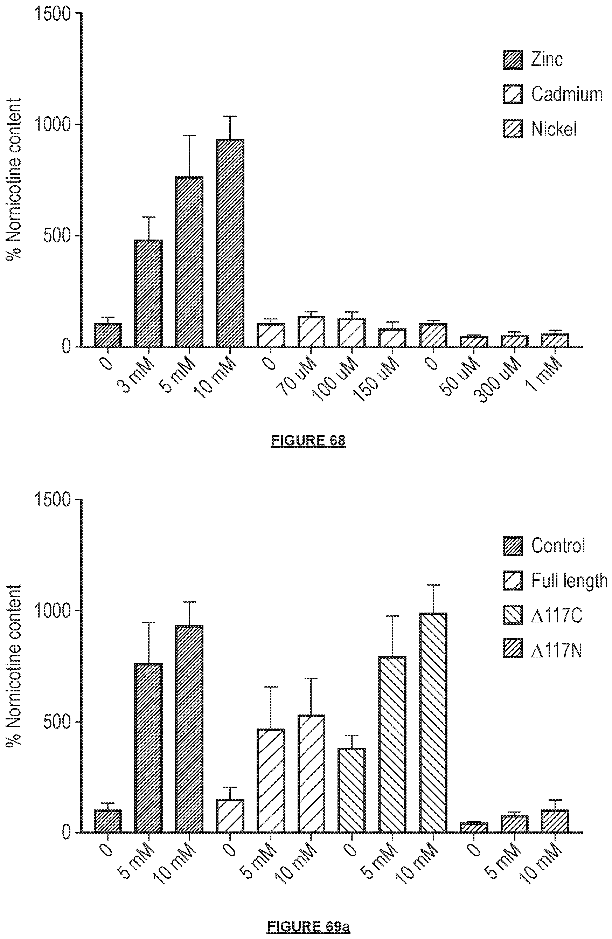

**Figure 68** shows the nornicotine content of tobacco plants treated with the indicated concentrations of zinc, cadmium, or nickel.
**Figure 69a** shows the nornicotine content of tobacco plants expressing the full-length zinc transporter, the Δ117C and Δ117N variants upon treatment with the indicated concentrations of zinc.

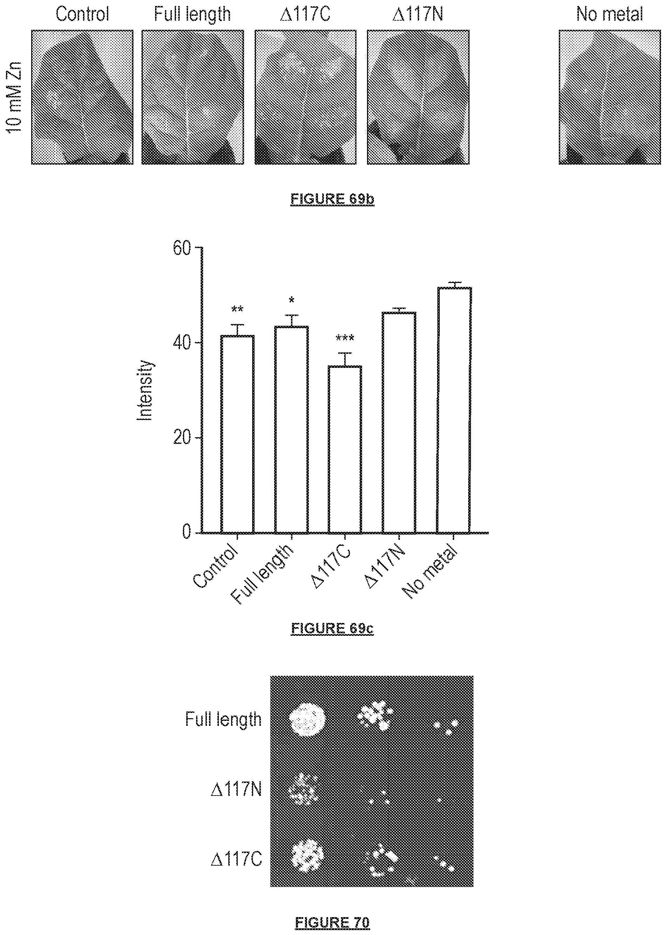

**Figure 69b** shows the phenotypes of tobacco leaves expressing the indicated constructs upon treatment with 10 mM zinc.
**Figure 69c** shows the chlorophyll intensity of treated leaf areas in Figure 69b. Values are shown as means ± SEM. Asterisks indicate statistical significance of P value ≤ 0.001 upon comparison to the no metal samples and analysis with one-way ANOVA and Tukey's multiple-comparison post-test.
**Figure 70** shows the expression of Δ117N metal efflux variant induces growth retardation in yeast.

### DETAILED DESCRIPTION

A seminal finding of the present invention is that deregulated cation efflux proteins can be used to modulate at least one tobacco-specific nitrosamine (TSNA) or a precursor thereto in tobacco.

Based on this finding a deregulated (e.g. a constitutively high affinity cation efflux transporter) can be used to reduce the content of a TSNA or a precursor or a TSNA in tobacco. In one embodiment the TSNA is N'nitrosonornicotine (NNN) and/or the precursor is nornicotine. Cation efflux proteins are pumps that remove metal ions such as cadmium, zinc, and cobalt from the cell. These metals are not only essential micronutrients for the plant metabolism, but they also act as secondary messengers initiating components of intracellular signal transduction cascades.

Based on these surprising findings, there is provided a method of reducing or decreasing the content of a tobacco specific nitrosamine (TSNA) or a precursor of a TSNA in tobacco comprising expressing a deregulated cation efflux protein in a tobacco plant or plant part thereof or plant cell. Suitably, the reduction in the TSNA content or precursor of a TSNA is compared to a tobacco plant or part thereof which does not comprise a deregulated cation efflux protein. In other words, the reduction in the TSNA content or precursor of a TSNA is compared to a tobacco plant or part thereof which has not been modified to express a deregulated cation efflux protein.

In one embodiment, the concentration and/or total content of nicotine in a tobacco plant or part thereof, such as a leaf (e.g. cured leaf), is not decreased by the method of reducing the content of a tobacco specific nitrosamine (TSNA) or a precursor of a TSNA in a tobacco plant or plant part thereof according to the present invention.

In one embodiment, the concentration and/or total content of nicotine in a tobacco plant or part thereof, such as a leaf (e.g. cured leaf), is unaltered (e.g. is not significantly altered for example is altered by less than 10%, less than 5%, less than 3%, less than 2%, less than 1%) by the method of reducing the content of a tobacco specific nitrosamine (TSNA) or a precursor of a TSNA in a tobacco plant or plant part thereof according to the present invention. In one embodiment, the concentration and/or total content of nicotine in a tobacco plant or part thereof, such as a leaf (e.g. cured leaf), may be increased by the method of reducing the content of a tobacco specific nitrosamine (TSNA) or a precursor of a TSNA in a tobacco plant or plant part thereof according to the present invention.

In a preferred embodiment the content of a TSNA or a precursor thereof in tobacco (e.g. in a tobacco leaf) is reduced by expressing a deregulated cation efflux protein in a tobacco plant or plant part thereof or plant cell.

In one embodiment the method of the present invention comprises introducing into the genome of said plant or part thereof or plant cell a mutation within a polynucleotide encoding a cation efflux protein. Suitably said mutation results the mutated polynucleotide encoding a deregulated cation efflux protein.

As used herein, a "mutation" may be a modification selected from a deletion, an insertion, a substitution.

In a preferred embodiment the mutation is a deletion. In another embodiment the mutation may be a substitution with a non-natural amino acid.

In one embodiment the method of the present invention comprises introducing into the genome of said plant a mutation within a polynucleotide encoding a protein comprising the sequence shown as SEQ ID No. 3, 6, 9, 12, 15, 18, 21, 24, 27 or 30 or a sequence which has at least 70% identity thereto.

In one embodiment the method of the present invention comprises introducing into the genome of said plant a mutation within a polynucleotide encoding a protein comprising the sequence shown as SEQ ID No. 3, 6, 9, 12, 15, 18, 21, 24, 27 or 30 or a sequence which has at least 75%, at least 80%, at least 85%, at least 90%, at least 95%, at least 97%, or at least 99% sequence identity hereto.

In one embodiment the method of the present method comprises introducing into the genome of a tobacco plant a mutation within a polynucleotide comprising the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29 or a sequence which has at least 70% sequence identity thereto.

In one embodiment the method of the present method comprises introducing into the genome of a tobacco plant a mutation within a polynucleotide comprising the sequence shown as SEQ ID No. 1,SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 75%, at least 80%, at least 85%, at least 90%, at least 95%, at least 97%, or at least 99% sequence identity thereto.

In one embodiment the protein comprising the sequence shown as SEQ ID No. 3, 6, 9, 12, 15, 18, 21, 24, 27 or 30 or a sequence which has at least 70% identity thereto is encoded by a polynucleotide comprising the sequence shown as SEQ ID No. 1,SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 70% sequence identity thereto.

In one embodiment the protein comprising the sequence shown as SEQ ID No. 3, 6, 9, 12, 15, 18, 21, 24, 27 or 30 or a sequence which has at least 70% identity thereto is encoded by a polynucleotide comprising the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 75%, at least 80%, at least 85%, at least 90%, at least 95%, at least 97%, or at least 99% sequence identity thereto.

The mutation may be any mutation which results in the expression of a deregulated cation efflux protein.

In one embodiment the mutation may produce a deletion, a splice mutant or codon encoding a non-tolerated amino acid substitution in the polynucleotide encoding said protein. Suitably, the mutant may comprise a deletion, such as deletion of at least part of a domain or deletion of an entire domain. Suitably, the mutant may comprise a deletion of at least part of the cytoplasmic domain.

In one embodiment, the mutation (e.g. deletion) may be in the N-terminal cytoplasmic domain of the protein. Suitably, the mutation may comprise deletion of at least part of the N terminus when compared with an amino acid sequence shown as SEQ ID No. 3, 6, 9, 12, 15, 18, 21, 24, 27 or 30 or a sequence which has at least 70% identity thereto.

Suitably, the mutation may comprise deletion of at least part of the N terminus when compared with an amino acid sequence shown as SEQ ID No. 3, or a sequence which has at least 70% identity thereto.

Suitably, the mutation may be in amino acids corresponding to at least amino acids 2-25, or 2-50, or 2-75, or 2-100, 2-114, 2-117, 2-120, 2-125, 2-128, or 2-130 of SEQ ID No. 3. Preferably, the mutation is in amino acids corresponding to amino acids 2-117 of SEQ ID No. 3. Preferably the mutation is a deletion.

In one embodiment, the method of the present invention comprises expressing in a tobacco plant or plant part thereof or plant cell:
a) a truncated protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, at least 98% or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30.

Suitably, the method may comprise expressing in a tobacco plant or plant part thereof or plant cell a truncated protein which lacks at least amino acids corresponding to amino acids 2-117 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, at least 98% or at least 99%) sequence identity to a truncated protein which lacks at least amino acids corresponding to amino acids 2-117 of SEQ ID No. 3, 6, 9, 12, 15, 18, 21, 24, 27 or 30.

Suitably, the method may comprise expressing in a tobacco plant or plant part thereof or plant cell a truncated protein which lacks at least amino acids corresponding to amino acids 2-117 from the N-terminal side of SEQ ID No. 3, or a protein which has at least 70% (preferably at least 75%, at least 80%, at least 85%, at least 90%, at least 95%, at least 97%, at least 98% or at least 99%) sequence identity to a truncated protein which lacks at least amino acids corresponding to amino acids 2-117 of SEQ ID No. 3.

In one embodiment, a method according to the present invention comprises expressing a protein comprising (or having or consisting of) the amino acid sequence shown as SEQ ID No. 33, or a sequence which has at least 70% or at least 75%, at least 80%, at least 85%, at least 90%, at least 95%, at least 97%, or at least 98%, or at least 99% sequence identity thereto in a tobacco plant or plant part thereof or plant cell.

In another embodiment, a method according to the present invention comprises expressing a protein encoded by a polynucleotide comprising the sequence shown as SEQ ID No. 34, or a sequence which has at least 70% sequence identity, or at least 75%, at least 80%, at least 85%, at least 90%, at least 95%, at least 97%, or at least 99% sequence identity thereto in a tobacco plant or plant part thereof or plant cell.

The tobacco plant or part thereof or plant cell may express said protein using any method known in the art.

Suitably, the method according to the present invention may comprise introducing a nucleotide sequence to a plant or part thereof or plant cell which encodes said protein. This may be achieved for example by introducing a construct or vector which encodes said protein. Suitably, the method may comprise introducing a construct or vector according to the present invention to a plant or part thereof or plant cell.

Alternatively, the method may comprise gene editing the endogenous nucleotide sequence to encode the variant protein.

In one embodiment the TSNA may be one or more of group selected from: N'-nitrosonornicotine (NNN), N'nitrosoanatabine (NAT), N'-nitrosoanabasine (NAB) and 4-(methyl nitrosamino)-1-(3-pyridyl)-1-butanone (NNK).

In a preferred embodiment the TSNA is N'-nitrosonornicotine (NNN).

The TSNA may be measured in a processed tobacco, e.g. cured tobacco or reconstituted tobacco. In one embodiment the TSNA content is measured and/or modified (e.g. reduced) in a cured tobacco plant or part thereof (e.g. in cured tobacco leaf).

The term "tobacco-specific nitrosamine" or "TSNA" as used herein has its usual meaning in the art, namely a nitrosamine which is found only in tobacco products or other nicotine-containing products. Suitably the at least one tobacco-specific nitrosamine may be 4-(methylnitrosamino)-1-(3-pyridyl)-1-butanone (NNK), N'-nitrosonornicotine (NNN), N'-nitrosoanatabine (NAT) or N-nitrosoanabasine (NAB).

More suitably the at least one tobacco-specific nitrosamine may be NNK or NNN.

In one embodiment the tobacco-specific nitrosamine is NNN.

The term "precursor thereto" when used in relation to at least one tobacco-specific nitrosamine refers to one or more chemicals or compounds of a tobacco plant that give rise to the formation of a tobacco-specific nitrosamine or are involved in the nitrosation reaction leading to tobacco-specific nitrosamine production. Suitably the term "precursor thereto" may refer to nitrate, nitrite or nitric oxide.

In one embodiment the precursor of the TSNA is one or more of the group selected from nornicotine, anabasine, anatabine, and an oxidised derivative of nicotine such as pseudooxynicotine (PON).

In one embodiment, the TSNA precursor is the direct precursor to the TSNA.

In one embodiment, a direct precursor of a TSNA is one or more of the group selected from nornicotine, anabasine, anatabine, and an oxidised derivative of nicotine such as pseudooxynicotine (PON).

In a preferred embodiment the precursor of the TSNA is nornicotine.

In one embodiment, the precursor of the TSNA is PON.

In one embodiment, the TSNA precursor is not nicotine.

The precursor of the TSNA (e.g. NNN, NNK, NAB and/or NAT) may be measured in green tobacco leaf, e.g. prior to processing, e.g. prior to curing. In one embodiment the precursor of the TSNA (e.g. NNN, NNK, NAB and/or NAT) is measured and/or modified (e.g. reduced) in a green tobacco leaf, e.g. prior to processing, e.g. prior to curing.

In one embodiment carrying out a method and or use of the invention results in a reduction of at least one TSNA or a precursor thereto in the modified tobacco plant (or part thereof) when compared to a tobacco plant (or part thereof) which has not been modified in accordance with the present invention.

The terms "reducing at least one TSNA or precursor thereto" or "reduction of at least one TSNA or precursor thereto" are used herein to mean that the concentration and/or total content of the at least one TSNA or precursor thereto in the product, method or use of the invention is lower in relation to a comparable product, method or use. For example, a comparable tobacco industry product would be derived from a tobacco plant which had not been modified according to the present invention, but in which all other relevant features were the same (e.g. plant species, growing conditions, method of processing tobacco, etc).

The term "a comparable product" as defined herein may mean a tobacco plant or a part thereof, such as a tobacco leaf, a harvested leaf, a cut harvested leaf, a processed tobacco leaf or tobacco plant propagation material, or a tobacco industry product or combinations thereof obtainable or obtained from a tobacco plant which has not been altered to express a deregulated cation efflux protein.

In one embodiment, a comparable product is obtainable or obtained from a tobacco plant which does not express a truncated protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30.

In another embodiment, a comparable product is obtainable or obtained from a tobacco plant which does not express protein comprising the amino acid sequence shown as SEQ ID No. 33, or a sequence which has at least 70% or at least 75%, at least 80%, at least 85%, at least 90%, at least 95%, at least 97%, or at least 99% sequence identity thereto.

Suitably, the comparable product may be obtainable or obtained from a tobacco plant which does not express a protein encoded by a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

The term "unmodified plant" as defined herein would be a tobacco plant which had not been modified according to the present invention, to express a deregulated cation efflux protein. In another embodiment, an unmodified plant would be a tobacco plant which had not been modified according to the present invention to express a truncated protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30 and in which all other relevant features were the same (e.g. plant species, growing conditions, method of processing tobacco, etc.).

Any method known in the art for determining the concentration and/or levels of at least one TSNA or precursor thereto may be used. In particular a method may comprise the addition of deuterium labelled internal standard, an aqueous extraction and filtration, followed by analysis using reversed phase high performance liquid chromatography with tandem mass spectrometry (LC-MS/MS). Other examples for determining the concentration and/or level of a precursor to a tobacco-specific nitrosamine include a method such as the one detailed in CORESTA recommended method CRM-72: Determination of Tobacco Specific Nitrosamines in Tobacco and Tobacco Products by LC-MS/MS; CRM being developed into ISO/DIS 21766 or Wagner et al. Analytical Chemistry (2005), 77(4), 1001-1006.

Suitably the concentration and/or total content of the at least one tobacco-specific nitrosamine or precursor thereto may be reduced by carrying out a method and/or use of the present invention. Suitably the concentration and/or level of the at least one tobacco-specific nitrosamine or precursor thereto may be reduced in a tobacco plant of the invention (e.g. obtainable or obtained by a method and/or use of the invention) when compared to the concentration and/or level of the at least one tobacco-specific nitrosamine(s) or precursor thereto in a tobacco plant which has not been modified in accordance with present invention. The concentration and/or total content of the at least one tobacco-specific nitrosamine(s) or precursor thereto may be reduced in a tobacco leaf, harvested leaf, processed tobacco leaf, tobacco industry product or combinations thereof obtainable or obtained from a tobacco plant (or part of a tobacco plant) of the invention when compared with a tobacco leaf, harvested leaf, processed tobacco leaf, tobacco industry product or combinations thereof obtainable or obtained from a tobacco plant (or part of a tobacco plant) which has not been modified in accordance with the present invention.

Suitably the concentration and/or total content of the at least one tobacco-specific nitrosamine or precursor thereto may be reduced in a processed tobacco leaf.

Suitably the concentration and/or level of the at least one tobacco-specific nitrosamine or precursor thereto may be reduced in a tobacco industry product.

In one embodiment the at least one tobacco-specific nitrosamine or precursor thereto may be reduced by at least about 1%, at least about 3%, at least about 5%, at least about 10%, at least about 20%, at least about 30%, at least about 40%, at least about 50%, at least about 60%, at least about 70%, at least about 80% or at least about 90%. In some embodiments the at least one tobacco-specific nitrosamine or precursor thereto may be reduced by between about 5% and about 95%, by between about 10% and about 90%, by between 20% and about 80%, by between 30% and about 70%, or by between about 40% and 60%.

In relation to processed tobacco leaf (e.g. cured or reconstituted), the at least one tobacco-specific nitrosamine or precursor thereto may be reduced by between about 5000 ng/g and about 50 ng/g, by between about 4000 ng/g and about 100 ng/g, by between about 3000 ng/g and 500 ng/g or by between 2000 ng/g and 1000 ng/g. In some embodiments the at least one tobacco-specific nitrosamine or precursor thereto may be reduced by at least about 5000 ng/g, at least about 4000 ng/g, at least about 3000 ng/g, at least about 2000 ng/g, at least about 1000 ng/g, at least about 500 ng/g, at least about 100 ng/g or at least about 50 ng/g.

### CATION EFFLUX PROTEIN

Cation efflux proteins are pumps that remove metal ions such as cadmium, zinc, and cobalt from the cell. These metals are not only essential micronutrients for the plant metabolism, they also act as secondary messengers initiating components of intracellular signal transduction cascades.

In one embodiment a wild type cation efflux protein comprises an amino acid sequence shown as SEQ ID No. 3, 6, 9, 12, 15, 18, 21, 24, 27 or 30, or a sequence which has at least 90% identity thereto (preferably at least 75%, at least 80%, at least 85%, at least 90%, at least 95%, at least 97%, or at least 99% identity thereto).

Suitably, a wild type cation efflux protein according to the present invention may comprise an amino acid sequence shown as SEQ ID No. 3, or a sequence which has at least 90% identity thereto (preferably at least 95%, at least 97%, or at least 99% identity thereto).

Suitably, a wild type cation efflux protein may comprise an amino acid sequence shown as SEQ ID No. 6, or a sequence which has at least 90% identity thereto (preferably at least 95%, at least 97%, or at least 99% identity thereto).

Suitably, a wild type cation efflux protein may comprise an amino acid sequence shown as SEQ ID No. 9, or a sequence which has at least 90% identity thereto (preferably at least 95%, at least 97%, or at least 99% identity thereto).

Suitably, a wild type cation efflux protein may comprise an amino acid sequence shown as SEQ ID No. 12, or a sequence which has at least 90% identity thereto (preferably at least 95%, at least 97%, or at least 99% identity thereto).

Suitably, a wild type cation efflux protein may comprise an amino acid sequence shown as SEQ ID No. 15, or a sequence which has at least 90% identity thereto (preferably at least 95%, at least 97%, or at least 99% identity thereto).

Suitably, a wild type cation efflux protein may comprise an amino acid sequence shown as SEQ ID No. 18, or a sequence which has at least 90% identity thereto (preferably at least 95%, at least 97%, or at least 99% identity thereto).

Suitably, a wild type cation efflux protein may comprise an amino acid sequence shown as SEQ ID No. 21, or a sequence which has at least 90% identity thereto (preferably at least 95%, at least 97%, or at least 99% identity thereto).

Suitably, a wild type cation efflux protein according to the present invention may comprise an amino acid sequence shown as SEQ ID No. 24, or a sequence which has at least 90% identity thereto (preferably at least 95%, at least 97%, or at least 99% identity thereto). Suitably, a wild type cation efflux protein according to the present invention may comprise an amino acid sequence shown as SEQ ID No. 27, or a sequence which has at least 90% identity thereto (preferably at least 95%, at least 97%, or at least 99% identity thereto). Suitably, a wild type cation efflux protein according to the present invention may comprise an amino acid sequence shown as SEQ ID No. 30, or a sequence which has at least 90% identity thereto (preferably at least 95%, at least 97%, or at least 99% identity thereto).

In one embodiment the wild type cation efflux protein comprises an amino acid sequence selected from: SEQ ID No. 3, 6, 9, 12, 15, 18, 21, 24, 27 or 30.

Suitably, the cation efflux protein may be from *Nicotiana tabacum.*

In one embodiment the wild type cation efflux protein is encoded by a polynucleotide sequence wherein the gene (prior to mutation) comprises the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 70% sequence identity thereto.

Suitably, the wild type protein for use according to the present invention may be encoded by a polynucleotide sequence wherein the gene (prior to mutation) comprises the sequence shown as SEQ ID No. 1, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 2, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 4, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 5, or a sequence which has at least 90% sequence identity thereto. Suitably, the protein for use according to the present invention may be encoded by a polynucleotide sequence wherein the gene (prior to mutation) comprises the sequence shown as SEQ ID No. 7, or a sequence which has at least 90% sequence identity thereto.

Suitably, the wild type protein for use according to the present invention may be encoded by a polynucleotide sequence wherein the gene (prior to mutation) comprises the sequence shown as SEQ ID No. 8, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 10, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 11, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 13, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 14, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 16, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 17, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 19, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 20, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 22, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 23, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 25, or a sequence which has at least 90% sequence identity thereto.

Suitably, the wild type protein for use according to the present invention may be encoded by a polynucleotide sequence wherein the gene (prior to mutation) comprises the sequence shown as SEQ ID No. 26, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 28, or a sequence which has at least 90% sequence identity thereto. Suitably, the wild type protein for use according to the present invention may be encoded by a polynucleotide sequence wherein the gene (prior to mutation) comprises the sequence shown as SEQ ID No. 29, or a sequence which has at least 90% sequence identity thereto. In one embodiment the wild type cation efflux protein is encoded by a polynucleotide sequence wherein the gene (prior to mutation) is selected from: SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29.

Suitably, the wild type protein for use according to the present invention may be encoded by a polynucleotide sequence from *Nicotiana tabacum.*

### DEREGULATED CATION EFFLUX PROTEIN

In one embodiment the protein for use according to the present invention is a deregulated cation efflux protein.

As used herein "cation efflux protein" has its usual meaning in the art and refers to a protein which transports cations, for example transports cations out of a cell.

Cation efflux proteins are usually integral membrane proteins which increase tolerance to divalent metal ions, such as zinc, cadmium and cobalt. Cation efflux proteins may act as efflux pumps to remove these ions from cells.

The cation efflux protein may be capable of transporting zinc, cobalt, cadmium or nickel.

In one embodiment, the cation efflux protein is capable of transporting zinc. In one embodiment, the cation efflux protein is a zinc transporter.

As used herein, "deregulated cation efflux protein" means that a cation efflux protein for use in accordance with the present invention does not respond to or exhibits a reduction in response to the concentration of metal ions in a cell when compared to a wild type cation efflux protein found in a tobacco plant, i.e. a cation efflux protein that is not deregulated.

In other words such a cation efflux protein is of reduced sensitivity to or no longer responsive to changes in metal ion concentration within a cell.

For example, the deregulated cation efflux protein is not capable of undergoing a conformational change upon modulation of metal ion concentration (i.e. from low metal ion concentration to high metal ion concentration) within the cell.

A cation efflux protein which is no longer capable of changing conformation in response to the metal ion concentration within the cell may be referred to herein as a deregulated cation efflux protein.

In one embodiment, the deregulated cation efflux protein for use according to the present invention is a constitutively high affinity cation efflux transporter.

As used herein the term "high affinity cation efflux protein" or "high affinity cation efflux transporter" refers to a protein which is in an open conformation which allows metal ion transport. For example, the protein may be in a constitutively open conformation.

In one embodiment the high affinity cation efflux protein is in a conformation which allows metal binding to the cytoplasmic binding sites between the transmembrane domains. See

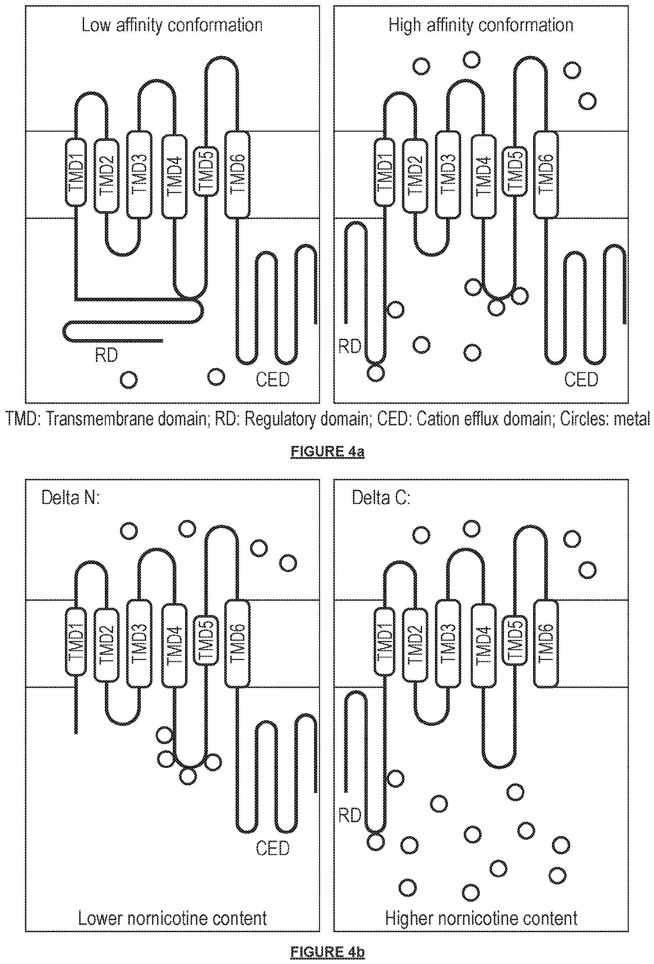

Figure 4 a. In other words, in the high affinity cation efflux protein the cytoplasmic binding sites between the transmembrane domains are not obscured by the cytoplasmic regulatory domain.

In one embodiment, a deregulated cation efflux protein is a protein which transports cations (such as zinc ions) out of a cell at a higher rate than a comparable, wild-type cation efflux protein. Suitably the wild-type cation protein may have an amino acid sequence as set forth in SEQ ID No. 3, 6, 9, 12, 15, 18, 21, 24, 27 or 30.

In one embodiment, the deregulated cation efflux protein exhibits increased metal ion transport compared to a wild type cation efflux transporter. The deregulated cation efflux protein may exhibit at least about 1%, at least about 3%, at least about 5%, at least about 10%, at least about 20%, at least about 30%, at least about 40%, at least about 50%, at least about 60%, at least about 70%, at least about 80% or at least about 90% more metal ion transport compared to a wild type cation efflux transporter. Suitably, metal ion transport may be measured under comparable conditions.

The activity of a cation efflux transporter (such as metal ion transport) may be measured using any method known in the art.

For example metal ion transport may be measured using fluorescent cation sensors, such as fluorescent Zn²⁺ sensors, fluorescence microscopy (described by Carpenter et al., Arch Biochem Biophys. 2016 Dec 1; 611: 20-29.

In one embodiment, a cell which expresses the deregulated cation efflux protein exhibits a lower concentration of intracellular metal ions (such as a lower intracellular concentration of zinc ions) compared to a cell which does not express the deregulated cation efflux protein, for example compared to a cell which only expresses the wild type cation efflux transporter.

Suitably the wild-type cation protein may have an amino acid sequence as set forth in SEQ ID No. 3, 6, 9, 12, 15, 18, 21, 24, 27 or 30.

A cell which expresses a deregulated cation efflux protein may exhibit a reduction in intracellular cation concentration (such as intracellular zinc concentration) of at least about 5%, at least about 10%, at least about 20%, at least about 30%, at least about 40%, at least about 50%, at least about 60%, at least about 70%, at least about 80% or at least about 90% more ion transport compared to a wild type cation efflux transporter.

The concentration of cations (such as zinc) in a cell may be measured by first washing a cell, removing extracellular matrix and lysing the cell then measuring the concentration of cations (such as zinc). A cell which expresses a deregulated cation efflux protein according to the invention may have a lower intracellular concentration of cations (such as zinc) compared to a cell which does not express a deregulated cation efflux protein i.e. a cell which only expresses wild-type cation efflux proteins.

In one embodiment, the deregulated cation efflux protein increases tolerance of a cell to divalent metal ions (such as zinc).

Without wishing to be bound by theory, the deregulated cation efflux protein may reduce the intracellular concentration of cations (such as zinc), thereby increasing tolerance to divalent metal ions. Suitably, the deregulated cation efflux protein may allow a cell to tolerate higher concentrations of divalent metal ions (such as zinc) than a cell which does not express a deregulated cation efflux protein.

The deregulated cation efflux protein may increase tolerance to divalent metal ions (such as zinc) at least about 5%, at least about 10%, at least about 20%, at least about 30%, at least about 40%, at least about 50%, at least about 60%, at least about 70%, at least about 80% or at least about 90% compared to a wild type cation efflux transporter.

Increased tolerance to metal ions (such as zinc) may be measured using any method in the art. For example, tolerance may be measured by determining leaf quality, such as chlorophyll intensity of leaves treated with metal ions as shown in Figure 69b. Without wishing to be bound by theory, the leaves of plants which express a deregulated cation efflux protein exhibit tolerance to treatment with metal ions because the cation efflux protein reduces the intracellular concentration of said metal ion.

In one embodiment, upon treatment with a metal ion (such as zinc) a cell expressing the deregulated cation efflux protein exhibits a similar leaf phenotype (such as chlorophyll intensity) to a leaf which has not been treated with a metal ion (such as zinc) - see for example Figure 69c. Suitably, a cell expressing the deregulated cation efflux protein may exhibit a reduction in chlorophyll intensity which is not statistically significant when compared to a leaf which has not been treated with said metal ion, for example using one-way ANOVA and Tukey's multiple-comparison post-test.

A cell expressing the deregulated cation efflux protein may exhibit a decrease in chlorophyll intensity of less than 15%, less than 10%, less than 5% when treated with metal ions compared to a leaf which has not been treated with said metal ions.

In one embodiment, the deregulated cation efflux protein may lack a functional regulatory domain; preferably the deregulated cation efflux protein lacks at least part of a regulatory domain.

The term "regulatory domain" as used herein has its usual meaning in the art namely a conserved part of a protein sequence which controls the activity of the protein. Regulatory domains include activation domains, repression domains and epigenetic effector domains.

In one aspect, the regulatory domain according to the present invention is the cytoplasmic domain.

In one aspect, the regulatory domain is a sensor of metal ion content in the cell.

When a protein lacks a functional regulatory domain according to the present invention, the activity of said protein is no longer capable of being regulated by that regulatory domain.

In one aspect, a cation efflux protein which lacks a functional regulatory domain is not capable of switching between low affinity and high affinity conformations (as shown in Figure 4a).

Suitably, the deregulated cation efflux protein may lack a functional cytoplasmic domain.

In one embodiment the cytoplasmic domain may be rendered non-functional by mutation. In another embodiment, the cytoplasmic domain may be rendered non-functional by deletion. Suitably, the deregulated cation efflux protein may lack at least part of the cytoplasmic domain when compared to a wild type protein. Suitably, the deregulated cation efflux protein may lack at least part of the N terminal when compared to a wild type protein.

The term "cytoplasmic domain" as used herein has its usual meaning in the art, and describes the topology of a protein domain as an intracellular domain which interacts with the interior of the cell.

Topological domains of proteins including cytoplasmic domains may be annotated based on predictions provided by algorithms such as the program TMHMM provided by the Technical University of Denmark (DTU) Department of Bio and Health Informatics; http://www.cbs.dtu.dk/services/TMHMM/ .

Alternatively, cytoplasmic domains may be annotated or predicted by amino acid sequence comparison with known protein structures. For example, cytoplasmic domains of cation efflux proteins may be identified by sequence alignment against SEQ ID No. 3, wherein amino acid resides 1-128 of SEQ ID No. 3 indicate a cytoplasmic domain.

In one embodiment, a cytoplasmic domain is a region of a protein which corresponds to amino acid 1 to 128 of the N terminus of SEQ ID No. 3. In one embodiment, a cytoplasmic domain is a region of a protein which corresponds to amino acid 2 to 128 of the N terminus of SEQ ID No. 3.

In one embodiment the first amino acid (amino acid number 1) is not counted as part of the N terminus. The first amino acid (amino acid number 1) is always required for translation of the protein.

In the context of the present invention, the cytoplasmic domain regulates the activity of the cation efflux protein. The cytoplasmic domain contains metal binding sites. When metal concentration in the cell increases to a threshold, metal binding to the N-terminal domain induces a conformational change in the cation efflux protein (see Figure 4).

In one embodiment, the deregulated cation efflux transporter is deregulated when compared to a wild type cation efflux protein.

In one embodiment a wild type cation efflux protein comprises an amino acid sequence shown as SEQ ID No. 3, 6, 9, 12, 15, 18, 21, 24, 27 or 30 or a sequence which has at least 70% identity thereto (preferably at least 75%, at least 80%, at least 85%, at least 90%, at least 95%, at least 97%, or at least 99% identity thereto).

In one embodiment, a wild type cation efflux protein is encoded by a polynucleotide sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a nucleic acid sequence which has at least 70% identity thereto (preferably at least 75%, at least 80%, at least 85%, at least 90%, at least 95%, at least 97%, or at least 99% identity thereto). Suitably, the method according to the present invention may comprise modifying an endogenous wild type sequence to encode a deregulated cation efflux protein. The endogenous sequence may be modified for example by gene editing.

In one embodiment, the deregulated cation efflux protein comprises one or more mutations compared to the amino acid sequence shown as SEQ ID No. 3, 6, 9, 12, 15, 18, 21, 24, 27 or 30 or a sequence which has at least 70% identity thereto.

The mutation may be a deletion, a splice mutant or codon encoding a non-tolerated amino acid substitution. The mutation may be in the cytoplasmic domain. The mutation may be in the N terminus.

In one embodiment the deregulated cation efflux protein comprises an amino acid sequence which lacks at least part of the N terminus when compared with an amino acid sequence shown as SEQ ID No. 3, 6, 9, 12, 15, 18, 21, 24, 27 or 30 or a sequence which has at least 70% identity thereto.

In another embodiment, the deregulated cation efflux protein comprises:
a) a truncated protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30; or
b) a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30. IN one embodiment the deregulated cation efflux protein comprises the amino acid sequence shown as SEQ ID No. 33, or a sequence which has at least 70% or at least 75%, at least 80%, at least 85%, at least 90%, at least 95%, at least 97%, or at least 99% sequence identity thereto.

In another embodiment the deregulated cation efflux protein is encoded by a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

In one embodiment, a deregulated cation efflux protein comprises an amino acid sequence shown as SEQ ID No. 3, 6, 9, 12, 15, 18, 21, 24, 27 or 30, or a sequence which has at least 90% identity thereto (preferably at least 75%, at least 80%, at least 85%, at least 90%, at least 95%, at least 97%, or at least 99% identity thereto) wherein upon alignment with the amino acid sequence SEQ ID No. 3, the deregulated cation efflux protein comprises:
a methionine at amino acid position number three;
an aspartic acid residue at amino acid position number seven: and/or
a threonine residue at amino acid residue number 68,
wherein the amino acid numbering is with reference to SEQ ID No. 3.

In some embodiments, the invention provides a cation efflux protein which is deregulated.

The deregulated cation efflux protein may be modified relative to a wild-type cation efflux protein to promote the formation of or stabilize dimers (such as homodimers) or oligomers. In one embodiment, a deregulated cation efflux protein according to the invention forms more stable dimers (such as homodimers) or other oligomers relative to a wild-type cation efflux protein. Suitably, mutations in the amino acid sequence may promote dimerization (such as homodimerisation) or oligomerisation of the deregulated cation efflux protein.

In some embodiments, the deregulated cation efflux protein may comprise one or more modified transmembrane domains (for example, relative to a wild-type cation efflux protein). Suitably, the deregulated cation efflux protein may comprise additional transmembrane domains or may comprise stabilized transmembrane domains.

### SEQUENCE IDENTITY

Sequence identity comparisons can be conducted by eye, or more usually, with the aid of readily available sequence comparison programs. These commercially available computer programs can calculate % identity between two or more sequences.

% identity may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % identity when a global alignment is performed. Consequently, most sequence comparison methods are designed to produce optimal alignments that take into consideration possible insertions and deletions without penalising unduly the overall identity score. This is achieved by inserting "gaps" in the sequence alignment to try to maximise local identity.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % identity therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel *et al* 1999, pages 7-58 to 7-60).

Although the final % identity can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage identities may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % sequence identity. The software typically does this as part of the sequence comparison and generates a numerical result.

Should Gap Penalties be used when determining sequence identity, the following parameters may be used for pairwise alignment:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, BLAST may be used with the gap penalty and gap extension set as defined above.

In one embodiment, CLUSTAL may be used with the gap penalty and gap extension set as defined above.

In some embodiments the gap penalties used for BLAST or CLUSTAL alignment may be different to those detailed above. The skilled person will appreciate that the standard parameters for performing BLAST and CLUSTAL alignments may change periodically and will be able to select appropriate parameters based on the standard parameters detailed for BLAST or CLUSTAL alignment algorithms at the time.

Suitably, the degree of identity with regard to a nucleotide sequence or an amino acid sequence is determined over at least 20 contiguous nucleotides/amino acids, preferably over at least 30 contiguous nucleotides/amino acids, preferably over at least 40 contiguous nucleotides/amino acids, preferably over at least 50 contiguous nucleotides/amino acids, preferably over at least 60 contiguous nucleotides/amino acids, preferably over at least 100 contiguous nucleotides/amino acids.

Suitably, the degree of identity with regard to a nucleotide sequence or the amino acid sequence may be determined over the whole sequence.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid ^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The present invention also encompasses sequences that are complementary to the nucleic acid sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also relates to nucleotide sequences that can hybridise to the nucleotide sequences of the present invention (including complementary sequences of those presented herein).

Preferably, hybridisation is determined under stringent conditions (e.g. 50°C and 0.2xSSC {1xSSC = 0.15 M NaCl, 0.015 M Na₃citrate pH 7.0}).

More preferably, hybridisation is determined under high stringent conditions (e.g. 65°C and 0.1xSSC {1xSSC = 0.15 M NaCl, 0.015 M Na₃citrate pH 7.0}).

### REDUCING OR PREVENTING EXPRESSION AND/OR FUNCTION

In one aspect the present invention provides a method of decreasing the concentration and/or total content of the at least one tobacco-specific nitrosamine or precursor thereto in tobacco comprising expressing a deregulated cation efflux protein in a tobacco plant or plant part thereof or plant cell.

In another aspect the present invention provides a method of decreasing the concentration and/or total content of the at least one tobacco-specific nitrosamine or precursor thereto in tobacco comprising expressing in a tobacco plant or plant part thereof or plant cell:
a) a truncated cation efflux protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated cation efflux protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, in a tobacco plant or plant part thereof or plant cell;
c) a protein comprising the amino acid sequence shown as SEQ ID No. 33, or a sequence which has at least 70%, at least 75%, at least 80%, at least 85%, at least 90%, at least 95%, at least 97%, or at least 99% sequence identity thereto; or
d) a protein encoded by a polynucleotide comprising the sequence shown as SEQ ID No. 34, or a sequence which has at least 70%, at least 75%, at least 80%, at least 85%, at least 90%, at least 95%, at least 97%, or at least 99% sequence identity thereto.Any method known in the art for expressing a protein may be used in the present method.

By way of example, the present method may comprise:
- providing a mutation (e.g. deletion) in a nucleic acid sequence which encodes a protein comprising the amino acid sequence shown as SEQ ID No. 3, 6, 9, 12, 15, 18, 21, 24, 27 or 30 or an amino acid sequence which has at least 70% sequence identity thereto;
- providing a mutation in a regulatory region (e.g. cytoplasmic domain) of a protein comprising the amino acid sequence shown as SEQ ID No. 3, 6, 9, 12, 15, 18, 21, 24, 27 or 30 or an amino acid sequence which has at least 70% sequence identity thereto;
- providing a mutation (e.g. deletion) in a regulatory region (e.g. a cytoplasmic domain, preferably an N-terminal cytoplasmic domain,) of a protein comprising the amino acid sequence shown as SEQ ID No. 3, 6, 9, 12, 15, 18, 21, 24, 27 or 30 or an amino acid sequence which has at least 70% sequence identity thereto;
- providing a nucleic acid sequence which encodes the amino acid sequence shown as SEQ ID No. 33, or a sequence which has at least 70% or at least 75%, at least 80%, at least 85%, at least 90%, at least 95%, at least 97%, or at least 99% sequence identity thereto;
- providing a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

Each of the above approaches result in the decrease of the content of at least one TSNA or a precursor of a TSNA in tobacco. Suitably, each of the above approaches may result in the expression of a deregulated cation efflux protein..

As used herein, the term "mutation" encompasses a natural genetic variant or an engineered variant. In particular, the term "mutation" refers to a variation in the nucleotide sequence encoding the amino acid sequence or in the amino acid sequence compared to the sequence shown as SEQ ID No. 3, 6, 9, 12, 15, 18, 21, 24, 27 or 30 or an amino acid sequence which has at least 70% sequence identity thereto. Suitably the mutation may reduce the content of at least one TSNA or a precursor of a TSNA in tobacco.

In one embodiment, a method according to the present invention may comprise providing a nucleic acid sequence according to the present invention to a plant or part thereof or plant cell. Suitably said nucleic acid sequence may be introduced to the plant or part thereof or cell. Suitably an endogenous nucleic acid sequence in the plant or part thereof or cell may be modified to encode the polypeptide according to the present invention (e.g. by gene editing). In a preferred embodiment, each copy of a nucleic acid sequence encoding a protein comprising a sequence shown as SEQ ID No. 3, 6, 9, 12, 15, 18, 21, 24, 27 or 30 or a sequence which has at least 70% sequence identity thereto which is present in the plant is modified e.g. mutated as defined herein (e.g. each genomic copy of a gene encoding said protein in a plant is mutated). For example, each copy of the gene in the allotetraploid genome of *N. tabacum* may be mutated.

In a preferred embodiment, all homologues of the cation efflux protein are modified e.g. mutated. Suitably, all of SEQ ID No. 3, 6, 9, 12, 15, 18, 21, 24, 27 or 30 or corresponding sequences which have at least 70% sequence identity thereto are modified e.g. mutated.

In a preferred embodiment the plant or plant cell according to the present invention is homozygous. Suitably, the plant or plant cell may be homozygous for the modification e.g. mutation.

In one embodiment preferably the plant or plant cell according to the present invention expresses only the modified e.g. mutated nucleic acid. In other words, in some embodiments no endogenous (or endogenous and functional protein) is present in the plant according to the present invention. In other words, if any endogenous protein is present it is preferably in an inactive form.

In one embodiment the present method may comprise providing a mutation in the sequence shown as SEQ ID No. 1, SEQ ID No. 2 or SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a nucleic acid sequence which has at least 70% identity thereto.

The mutation may alter the plant genome such that a nucleic acid sequence encoding a protein comprising the amino acid sequence shown as SEQ ID No. 3, 6, 9, 12, 15, 18, 21, 24, 27 or 30 or an amino acid sequence which has at least 70% sequence identity thereto is completely or partially deleted or otherwise made non-functional.

The nucleic acid sequence may comprise one or more nucleotide change(s) that reduce or prevent expression of the protein or affect protein trafficking. For example, expression of the protein may be reduced or prevented by introduction of one or more of a frame shift, a splice mutant or a non-tolerated amino acid substitution in the open reading frame.

A frame-shift mutation (also called a framing error or a reading frame shift) is a mutation caused by indels (insertions or deletions) of a number of nucleotides in a nucleic acid sequence that is not divisible by three. Due to the triplet nature of gene expression by codons, the insertion or deletion can change the reading frame, resulting in a completely different translation from the original. A frameshift mutation will often cause the reading of the codons after the mutation to code for different amino acids. The frameshift mutation will commonly result in the introduction of a premature stop codon.

A splice mutant inserts, deletes or changes a number of nucleotides in the specific site at which splicing takes place during the processing of precursor messenger RNA into mature messenger RNA. The deletion of the splicing site results in one or more introns remaining in mature mRNA and may lead to the production of abnormal proteins.

A non-tolerated amino acid substitution refers to a mutation which causes a non-synonymous amino acid substitution in the protein which results in reduced or ablated function of the protein.

Any method known in the art for providing a mutation in a nucleic acid sequence may be used in the present method. For example, homologous recombination may be used, in which a vector is created in which the relevant nucleic acid sequence(s) are mutated and used to transform plants or plant cells. Recombinant plants or plant cells expressing the mutated sequence may then be selected.

The nucleic acid sequence may be wholly or partially deleted. The deletion may be continuous, or may comprise a plurality of sections of sequence. The deletion preferably removes a sufficient amount of nucleotide sequence such that the nucleic acid sequence no longer encodes a functional protein.

The deletion may remove at least part of one or more domains of the cation efflux protein. The deletion may, for example, remove at least 10%, 20%, 30%, 40%, 50%, 60%, 70%, 80% or 90% of the coding portion of the nucleic acid sequence of at least one domain.

The deletion may, for example, remove at least 10%, 20%, 30%, 40%, 50%, 60%, 70%, 80% or 90% of the coding portion of the nucleic acid sequence.

The N terminal regulatory cytoplasmic domain is shown in Figure 37. The N terminal regulatory cytoplasmic domain may be identified by sequence alignment with SEQ ID NO. 3. In one embodiment, the amino acids encoding N-terminal regulatory cytoplasmic domain correspond to amino acids 1-128 of when aligned with SEQ ID No. 3.

Suitably, at least part of the N terminal regulatory cytoplasmic domain may be deleted. The deletion may, for example, remove at least 10%, 20%, 30%, 40%, 50%, 60%, 70%, 80% or 90% of the coding portion of the cytoplasmic domain. Suitably, the deletion may remove the entire N terminal regulatory cytoplasmic domain.

The cation efflux transmembrane domain and the cation efflux cytoplasmic domains are shown in Figure 36.

Suitably, at least part of the cation efflux transmembrane domain may be deleted. The deletion may, for example, remove at least 10, 20, 30, 40, 50, 60, 70, 80 or 90% of the coding portion of the cation efflux transmembrane domain.

Suitably, at least part of the cation efflux cytoplasmic domains may be deleted. The deletion may, for example, remove at least 10, 20, 30, 40, 50, 60, 70, 80 or 90% of the coding portion of the cation efflux cytoplasmic domain.

Methods for deletion of nucleic acid sequences in plants are known in the art. For example, homologous recombination may be used, in which a vector is created in which the relevant nucleic acid sequence(s) are missing and used to transform plants or plant cells. Recombinant plants or plant cells expressing the new portion of sequence may then be selected.

Plant cells transformed with a vector as described above may be grown and maintained in accordance with well-known tissue culturing methods such as by culturing the cells in a suitable culture medium supplied with the necessary growth factors such as amino acids, plant hormones, vitamins, etc.

Modification of the nucleic acid sequence may be performed using targeted mutagenesis methods (also referred to as targeted nucleotide exchange (TNE) or oligo-directed mutagenesis (ODM)). Targeted mutagenesis methods include, without limitation, those employing zinc finger nucleases, TALENs (see WO2011/072246 and WO2010/079430), Cas9-like, Cas9/crRNA/tracrRNA, Cas9/gRNA, or other CRISPR systems (see WO 2014/071006 and WO2014/093622), meganucleases (see WO2007/047859 and WO2009/059195), or targeted mutagenesis methods employing mutagenic oligonucleotides, possibly containing chemically modified nucleotides for enhancing mutagenesis with sequence complementarity to the gene, into plant protoplasts (e.g., KeyBase^{®} or TALENs). Alternatively, mutagenesis systems such as TILLING (Targeting Induced Local Lesions IN Genomics; McCallum et al., 2000, Nat Biotech 18:455, and McCallum et al. 2000, Plant Physiol. 123, 439-442) may be used to generate plant lines which comprise a gene encoding a protein having a mutation. TILLING uses traditional chemical mutagenesis (e.g. ethyl methanesulfonate (EMS) mutagenesis, which produces random mutations) followed by high-throughput screening for mutations. Thus, plants, seeds and tissues comprising a gene having the desired mutation may be obtained.

The method may comprise the steps of mutagenizing plant seeds (e.g. EMS mutagenesis), pooling of plant individuals or DNA, PCR amplification of a region of interest, heteroduplex formation and high-throughput detection, identification of the mutant plant, sequencing of the mutant PCR product. It is understood that other mutagenesis and selection methods may equally be used to generate such modified plants. Seeds may, for example, be radiated or chemically treated and the plants may be screened for a modified phenotype.

Fast neutron deletion mutagenesis may be used in a reverse genetics sense (i.e. with PCR) to identify plant lines carrying a deletion in the endogenous gene. See for example Ohshima et al. (1998) Virology 213:472-481; Okubara et al. (1994) Genetics 137:867-874; and Quesada et al. (2000) Genetics 154:421-4315.

In another approach, dominant mutants may be used to trigger RNA silencing due to gene inversion and recombination of a duplicated gene locus. See for example Kusaba et al. (2003) Plant Cell 15:1455-1467.

Modified plants may be distinguished from non-modified plants, i.e., wild type plants, by molecular methods, such as the mutation(s) present in the DNA, and by the modified phenotypic characteristics. The modified plants may be homozygous or heterozygous for the mutation.

In one embodiment the method of reducing the content of at least one tobacco specific nitrosamine (TSNA) or a precursor of a TSNA in tobacco does not comprise treating the plant with a chemical (e.g. an agrochemical).

Other ways of reducing or preventing the expression or activity will be apparent to one skilled in the art and include the use of micro RNA silencing, RNAi, antisense, tDNA insertions, virus-induced gene silencing (VIGs), or dominant negative constructs (or antimorphic mutations).

In one embodiment the nucleic acid encoding a protein comprising the amino acid sequence shown as SEQ ID No. 3, 6, 9, 12, 15, 18, 21, 24, 27 or 30 or an amino acid sequence which has at least 70% sequence identity thereto may be modified or mutated by a targeted mutagenesis based system.

In one embodiment the nucleic acid encoding a protein comprising the amino acid sequence shown as SEQ ID No. 3, 6, 9, 12, 15, 18, 21, 24, 27 or 30 or an amino acid sequence which has at least 70% sequence identity thereto may be modified (e.g. mutated or at least partially deleted) by gene editing such as a CRISPR based system.

In one embodiment the nucleic acid encoding a protein comprising the amino acid sequence shown as SEQ ID No. 3, 6, 9, 12, 15, 18, 21, 24, 27 or 30 or an amino acid sequence which has at least 70% sequence identity thereto may be modified (e.g. mutated or at least partially deleted) by zinc finger nuclease, TALENs, meganucleases, mutagenic oligonucleotides or TILLING.

Measurement of the level or amount of a gene product may be carried out by any suitable method, for example including comparison of mRNA transcript levels, protein or peptide levels, and/or phenotype of a plant, between a modified plant and comparable plant which has not been modified according to the present invention.

The ability of a protein to function as a cation efflux protein may be determined using any method known in the art for example including plant assays (such as those described by Lang et al, 2011. Journal of Experimental Botany, Volume 62, Issue 13 pages 4467-4480) or yeast complementation assays (such as those described by Pervans et al. 2001, PNAS, vol. 98 no. 17).

In one embodiment, the ability of a protein to function as a cation efflux protein may be determined using a plant assay.

Suitably, shoot biomass production of a plant comprising the polynucleotide of interest may be measured under metal stress and compared to shoot biomass production of a wild-type plant. An increase in shoot biomass production under metal stress relative to the shoot biomass production of a wild-type plant is indicative of the polynucleotide of interest having cation efflux transporter functionality.

Suitably, transcript expression of the polynucleotide of interest may be measured under metal stress (such as Zn, Cd, Co, Ni) and compared to basal levels of wild type plants. An increase in transcript level of the polynucleotide of interest under metal stress compared to shoot biomass production of a wild-type plant is indicative of the polynucleotide of interest having a cation efflux transporter functionality.

In one embodiment, the ability of a protein to function as a cation efflux protein may be measured using a yeast complementation assay, such as the assay described herein. Suitably, the cDNA of the polynucleotide of interest may be cloned into a yeast expression vector and transformed into a *cot1zrc1* double mutant yeast strain (sensitive to Co, Ni, Cd, and Zn). Suitably, the yeast are on plates around cation-soaked filter discs,

An assay may be conducted by comparing the zone of yeast strain growth inhibition for yeast comprising the polynucleotide of interest compared to yeast comprising a control vector. When the area of zone of inhibition for yeast comprising the polynucleotide of interest is smaller than the area of zone of inhibition for yeast comprising a control vector, this is indicative of the polynucleotide of interest having a cation efflux transporter functionality.

In one aspect, the present invention provides a method for decreasing the concentration and/or total content of a tobacco specific nitrosamine (TSNA) or a precursor of a TSNA in a tobacco plant.

The method may comprise expressing within the plant a polynucleotide (e.g. an exogenous polynucleotide) comprising:
a) a truncated cation efflux protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated cation efflux protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
c) a protein comprising the amino acid sequence shown as SEQ ID No. 33, or a sequence which has at least 70% or at least 75%, at least 80%, at least 85%, at least 90%, at least 95%, at least 97%, or at least 99% sequence identity thereto; or
d) a protein encoded by a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

In one aspect, the present invention provides a method for increasing the concentration and/or total content of nicotine in a tobacco plant. Suitably said method may be used to produce nicotine for use in non-combustible systems.

The method may comprise expressing within the plant a polynucleotide (e.g. an exogenous polynucleotide) comprising:
a) a truncated cation efflux protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated cation efflux protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
c) a protein comprising the amino acid sequence shown as SEQ ID No. 33, or a sequence which has at least 70% or at least 75%, at least 80%, at least 85%, at least 90%, at least 95%, at least 97%, or at least 99% sequence identity thereto; or
d) a protein encoded by a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

### PROMOTERS

The nucleic acid sequence may be operably linked to with a heterologous promoter for directing transcription of said nucleic acid sequence in said plant.

For example, in some embodiments, a promoter may be operably linked to nucleotide sequence in a construct or vector which is used to decrease the concentration and/or total content of a tobacco specific nitrosamine (TSNA) or a precursor of a TSNA in a tobacco plant or part thereof.

In one embodiment, a promoter is operably linked to a nucleic acid sequence which encodes a deregulated cation efflux protein according to the present invention.

Suitably, a promoter may be operably linked to:
a. A nucleotide sequence comprising SEQ ID No. 34;
b. A nucleotide sequence having at least 70% sequence identity to the entirety of the sequence set forth in SEQ ID No. 34, wherein said nucleotide sequence encodes a polypeptide involved in cation efflux in a plant;
c. A nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
d. A nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or2-100, or2-114, or2-117, or2-117, or2-120, or 2-125, or 2-128, or 2-130 of SEQ ID No. 3, 6, 9, 12, 15, 18, 21, 24, 27 or 30;
e. A nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
f. A nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant.

In some embodiments, a promoter may be operably linked to nucleotide sequence in a construct or vector which is used to increase the concentration and/or total content of nicotine in a cell or cell culture or tobacco plant or part thereof.

In some embodiments the promoter may be selected from the group consisting of: a constitutive promoter, a tissue-specific promoter, a developmentally-regulated promoter and an inducible promoter.

In one embodiment the promoter may be a constitutive promoter.

A constitutive promoter directs the expression of a gene throughout the various parts of a plant continuously during plant development, although the gene may not be expressed at the same level in all cell types. Examples of known constitutive promoters include those associated with the cauliflower mosaic virus 35S transcript (Odell JT, Nagy F, Chua NH. (1985). Identification of DNA sequences required for activity of the cauliflower mosaic virus 35S promoter. Nature. 313 810-2), the rice actin 1 gene (Zhang W, McElroy D, Wu R. (1991). Analysis of rice Act1 5' region activity in transgenic rice plants. Plant Cell 3 1155-65) and the maize ubiquitin 1 gene (Cornejo MJ, Luth D, Blankenship KM, Anderson OD, Blechl AE. (1993). Activity of a maize ubiquitin promoter in transgenic rice. Plant Molec. Biol. 23 567-81). Constitutive promoters such as the Carnation Etched Ring Virus (CERV) promoter (Hull R, Sadler J, LongstaffM (1986) (CaMV/35S), figwort mosaic virus 35S promoter. The sequence of carnation etched ring virus DNA: comparison with cauliflower mosaic virus and retroviruses. EMBO Journal, 5(2):3083-3090).

The constitutive promoter may be selected from a: a carnation etched ring virus (CERV) promoter, a cauliflower mosaic virus (CaMV 35S promoter), a promoter from the rice actin 1 gene or the maize ubiquitin 1 gene.

The promoter may be a tissue specific promoter. A tissue-specific promoter is one which directs the expression of a gene in one (or a few) parts of a plant, usually throughout the lifetime of those plant parts. The category of tissue-specific promoter commonly also includes promoters whose specificity is not absolute, i.e. they may also direct expression at a lower level in tissues other than the preferred tissue. Tissue specific promoters include the phaseolin-promoter, legumin b4-promoter, usp-promoter, sbp-promoter, ST-LS1 promoter, B33 (patatin class I promoter).

In another embodiment the promoter may be a developmentally-regulated promoter.

A developmentally-regulated promoter directs a change in the expression of a gene in one or more parts of a plant at a specific time during plant development. The gene may be expressed in that plant part at other times at a different (usually lower) level, and may also be expressed in other plant parts.

In one embodiment the promoter may be an inducible promoter.

An inducible promoter is capable of directing the expression of a gene in response to an inducer. In the absence of the inducer the gene will not be expressed. The inducer may act directly upon the promoter sequence, or may act by counteracting the effect of a repressor molecule. The inducer may be a chemical agent such as a metabolite, a protein, a growth regulator (such as auxin and salicylic acid which activate the OCS promoter), or a toxic element, a physiological stress such as heat, light (such as the soybean SSU promoter), wounding (e.g. the nos, nopaline synthase promoter), or osmotic pressure, or an indirect consequence of the action of a pathogen or pest. A developmentally-regulated promoter might be described as a specific type of inducible promoter responding to an endogenous inducer produced by the plant or to an environmental stimulus at a particular point in the life cycle of the plant. Examples of known inducible promoters include those associated with wound response, such as described by Warner SA, Scott R, Draper J. ((1993) Plant J. 3 191-201), temperature response as disclosed by Benfey & Chua (1989) (Benfey, P.N., and Chua, N-H. ((1989) Science 244 174-181), and chemically induced, as described by Gatz ((1995) Methods in Cell Biol. 50 411-424).

The present invention also provides a construct or vector comprising a nucleic acid sequence encoding a protein according to the present invention.

In one embodiment, the construct or vector according to the present invention may comprise:
a. A nucleotide sequence comprising SEQ ID No. 34;
b. A nucleotide sequence having at least 70% sequence identity to the entirety of the sequence set forth in SEQ ID No. 34, wherein said nucleotide sequence encodes a polypeptide involved in cation efflux in a plant;
c. A nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
d. A nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
e. A nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
f. A nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant.

The present invention further provides the use of a nucleic acid sequence according to the present invention reduce the content of a tobacco specific nitrosamine (TSNA) or a precursor of a TSNA in tobacco.

The present invention also provides a chimeric construct comprising a promoter operably linked to a nucleic acid sequence according to the present invention.

A suitable promoter sequence may be constitutive, non-constitutive, tissue-specific, developmentally-regulated or inducible/repressible.

In one embodiment a suitable promoter may be a promoter selected from the group consisting of: the cauliflower mosaic virus 35S promoter, the Carnation Etch Ring Virus (CERV) promoter, the pea plastocyanin promoter, the rubisco promoter, the nopaline synthase promoter, the chlorophyll a/b binding promoter, the high molecular weight glutenin promoter, the α, β-ghadin promoter, the hordein promoter, the patatin promoter, or a senescence-specific promoter.

The construct may be comprised in a vector. Suitably the vector may be a plasmid.

Exogenous polynucleotides may be introduced into plants according to the present invention by means of suitable vector, e.g. plant transformation vectors. A plant transformation vector may comprise an expression cassette comprising 5'-3' in the direction of transcription, a promoter sequence, a gene of interest (e.g. nucleic acid sequence encoding a protein according to the present invention) coding sequence, optionally including introns, and, optionally a 3' untranslated, terminator sequence including a stop signal for RNA polymerase and a polyadenylation signal for polyadenylase. The promoter sequence may be present in one or more copies, and such copies may be identical or variants of a promoter sequence as described above. The terminator sequence may be obtained from plant, bacterial or viral genes. Suitable terminator sequences are the pea rbcS E9 terminator sequence, the nos terminator sequence derived from the nopaline synthase gene of Agrobacterium tumefaciens and the 35S terminator sequence from cauliflower mosaic virus, for example. A person skilled in the art will be readily aware of other suitable terminator sequences.

The expression cassette may also comprise a gene expression enhancing mechanism to increase the strength of the promoter. An example of such an enhancer element is one derived from a portion of the promoter of the pea plastocyanin gene, and which is the subject of International patent Application No. WO 97/20056. Suitable enhancer elements may be the nos enhancer element derived from the nopaline synthase gene of Agrobacterium tumefaciens and the 35S enhancer element from cauliflower mosaic virus, for example. These regulatory regions may be derived from the same gene as the promoter DNA sequence or may be derived from different genes, for example from a plant of the family Solanaceae. All of the regulatory regions should be capable of operating in cells of the tissue to be transformed.

The promoter DNA sequence may be derived from the same gene as the gene of interest (e.g. the gene the promoter is going to direct, for instance a gene encoding a the modification of a plant to increase the activity or expression of a protein comprising the sequence shown as SEQ ID No. 3, 6, 9, 12, 15, 18, 21, 24, 27 or 30 or an amino acid sequence which has at least 70% sequence identity thereto) coding sequence used in the present invention or may be derived from a different gene, from for example from a plant of the family Solanaceae. The expression cassette may be incorporated into a basic plant transformation vector, such as pBIN 19 Plus, pBl 101, or other suitable plant transformation vectors known in the art. In addition to the expression cassette, the plant transformation vector will contain such sequences as are necessary for the transformation process. These may include the Agrobacterium vir genes, one or more T-DNA border sequences, and a selectable marker or other means of identifying transgenic plant cells.

The term "plant transformation vector" means a construct capable of *in vivo* or *in vitro* expression. Preferably, the expression vector is incorporated in the genome of the organism. The term "incorporated" preferably covers stable incorporation into the genome.

Techniques for transforming plants are well known within the art and include Agrobacterium-mediated transformation, for example. The basic principle in the construction of genetically modified plants is to insert genetic information in the plant genome so as to obtain a stable maintenance of the inserted genetic material. A review of the general techniques may be found in articles by Potrykus (Annu Rev Plant Physiol Plant Mol Biol [1991] 42:205-225) and Christon (AgroFood-Industry Hi-Tech March/April1994 17-27).

Typically, in Agrobacterium-mediated transformation a binary vector carrying a foreign DNA of interest, is transferred from an appropriate Agrobacterium strain to a target plant by the cocultivation of the Agrobacterium with explants from the target plant. Transformed plant tissue is then regenerated on selection media, which selection media comprises a selectable marker and plant growth hormones. An alternative is the floral dip method (Clough & Bent, 1998) whereby floral buds of an intact plant are brought into contact with a suspension of the Agrobacterium strain containing the chimeric gene, and following seed set, transformed individuals are germinated and identified by growth on selective media. Direct infection of plant tissues by Agrobacterium is a simple technique which has been widely employed and which is described in Butcher D. N. et al., (1980), Tissue Culture Methods for Plant Pathologists, eds.: D. S. Ingrams and J.P. Helgeson, 203-208.

Further suitable transformation methods include direct gene transfer into protoplasts using polyethylene glycol or electroporation techniques, particle bombardment, micro-injection and the use of silicon carbide fibres for example.

Transforming plants using ballistic transformation, including the silicon carbide whisker technique are taught in Frame B R, Drayton P R, Bagnaall S V, Lewnau C J, Bullock W P, Wilson H M, Dunwell J M, Thompson J A & Wang K (1994). Production of fertile transgenic maize plants by silicon carbide whisker-mediated transformation is taught in The Plant Journal 6: 941-948) and viral transformation techniques is taught in for example Meyer P, Heidmmm I & Niedenhof I (1992). The use of cassava mosaic virus as a vector system for plants is taught in Gene 110: 213-217. Further teachings on plant transformation may be found in EP-A-0449375.

In a further aspect, the present invention relates to a vector system which carries a nucleotide sequence encoding a gene of interest (e.g. a nucleic acid sequence according to the present invention) and introducing it into the genome of an organism, such as a plant. The vector system may comprise one vector, but it may comprise two vectors. In the case of two vectors, the vector system is normally referred to as a binary vector system. Binary vector systems are described in further detail in Gynheung Anetal, (1980), Binary Vectors, Plant Molecular Biology Manual A3, 1-19.

One extensively employed system for transformation of plant cells uses the Ti plasmid from Agrobacterium tumefaciens or a Ri plasmid from Agrobacterium rhizogenes Anetal., (1986), Plant Physiol. 81, 301-305 and Butcher D. N. et al., (1980), Tissue Culture Methods for Plant Pathologists, eds.: D. S. Ingrams and J.P. Helgeson, 203-208. After each introduction method of the desired exogenous gene according to the present invention in the plants, the presence and/or insertion of further DNA sequences may be necessary. The use of T-DNA for the transformation of plant cells has been intensively studied and is described in EP-A-120516; Hoekema, in: The Binary Plant Vector System Offset-drukkerij Kanters B. B., Amsterdam, 1985, Chapter V; Fraley, et al., Crit. Rev. Plant Sci., 4:1-46; and Anetal., EMBO J (1985) 4:277-284.

Plant cells transformed with an exogenous gene encoding a protein of interest (e.g. a protein according to the present invention) may be grown and maintained in accordance with well-known tissue culturing methods such as by culturing the cells in a suitable culture medium supplied with the necessary growth factors such as amino acids, plant hormones, vitamins, etc.

The term "transgenic plant" in relation to the present invention includes any plant that comprises an exogenous gene encoding a gene of interest, e.g. a protein according to the present invention, as described herein. Preferably the exogenous gene is incorporated in the genome of the plant.

The terms "transgenic plant" and "exogenous gene" do not cover native nucleotide coding sequences in their natural environment when they are under the control of their native promoter which is also in its natural environment.

Thus in one embodiment the present invention relates to a method for producing a transgenic plant comprising introducing, into an unmodified plant, an exogenous gene (chimeric construct or vector) encoding a protein according to the present invention.

In one embodiment the present invention relates to a method for producing a transgenic plant comprising transforming a plant cell with a construct or vector (e.g. a chimaeric construct) comprising a nucleic acid encoding a protein according to the present invention; and regenerating a plant from the transformed plant cell.

In another aspect there is provided the use of an exogenous nucleic acid sequence (construct or vector or chimaeric construct) in accordance with the present invention for decreasing the content of a tobacco specific nitrosamine (TSNA) or a precursor of a TSNA in a tobacco plant, e.g. by transformation of the plant with the exogenous nucleic acid sequence (construct or vector or chimaeric construct).

In one embodiment the present invention further relates to a host cell comprising an exogenous nucleic acid sequence (construct or vector or chimaeric construct) in accordance with the present invention.

In one embodiment, a mutation in the amino acid sequence shown as SEQ ID No. 3, 6, 9, 12, 15, 18, 21, 24, 27 or 30 or a sequence which has at least 70% sequence identity thereto may deregulate the protein in relation to a protein shown as SEQ ID No. 3, 6, 9, 12, 15, 18, 21, 24, 27 or 30 or a sequence which has at least 70% sequence identity thereto.

In some embodiments the mutation in the amino acid sequence shown as SEQ ID No. 3, 6, 9, 12, 15, 18, 21, 24, 27 or 30 or a sequence which has at least 70% sequence identity thereto may not alter the level or expression but may decrease the activity of the protein in relation to a protein shown as SEQ ID No. 3, 6, 9, 12, 15, 18, 21, 24, 27 or 30 or a sequence which has at least 70% sequence identity thereto.

### BIOMASS PRODUCTION

In one aspect, the present invention provides a method of producing a biomass comprising: growing a cell which has been engineered to express a deregulated cation efflux protein under conditions to produce a biomass.

In one embodiment, the present invention provides a method of producing a biomass having modified (e.g. increased) concentration and/or total content of nicotine, comprising growing a cell which have been engineered to:
a) express a deregulated cation efflux protein;
b) express a truncated protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
c) express a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
d) express a protein comprising the amino acid sequence shown as SEQ ID No. 33, or a sequence which has at least 70% or at least 75%, at least 80%, at least 85%, at least 90%, at least 95%, at least 97%, or at least 99% sequence identity thereto;
e) express a protein encoded by a polynucleotide comprising the sequence shown as SEQ ID No. 34, or a sequence which has at least 70% sequence identity thereto or at least 75%, at least 80%, at least 85%, at least 90%, at least 95%, at least 97%, or at least 99% sequence identity thereto.

Suitably, the biomass may contain a higher concentration and/or total content of nicotine compared with the biomass produced by a comparable cell which has not been modified in accordance with the present invention.

Suitably the cell for use in biomass production may be a plant cell, such as a tobacco cell. Suitably the cell for use in biomass production may be a yeast cell.

In one embodiment the cell (e.g. yeast cell) may be further modified to comprise one or more sequences that increases nicotinic alkaloid biosynthesis. Suitably these one or more sequences may be incorporated into a nucleic acid construct that is suitable for cell (e.g. yeast cell) transformation. The one or more sequences may be overexpressed in the cell(e.g. yeast cell). The sequences may be selected from one or more of the following genes: MPO (or Methylputrescine Oxidase or MPO1 or MPO2); A622 (or Isoflavone reductase-like protein or Isoflavone reductase homolog or Isoflavone reductase-like protein); BBL (or Berberine bridge enzyme or Berberine bridge enzyme-like or BBE or NBB1); PMT (or Putrescine N-Methyltransferase or putrescine methyltransferase or S-adenosyl-L-methionine:putrescine N-methyltransferase or PMT or PMT1 or PMT2 or PMT3 or PMT4) and QPT (or quinolinate phosphoribosyltransferase). In one embodiment the sequences may be selected from one or more of the following genes: BBL, A622, PMT and MPO (MPO1 or MPO2). Genes suitable for modification of in this way may be taught in US2016032299 for example.

### COMMERCIALLY DESIRABLE TRAITS

The term "commercially desirable traits" will include traits such as yield, quality (e.g. leaf quality, suitably cured leaf quality), abiotic (for instance drought) stress tolerance, herbicide tolerance and/or biotic (for instance insect, bacteria or fungus) stress tolerance and/or disease tolerance.

Leaf quality may be measured based on colour, texture and aroma of the cured leaf, for example according to United States Department of Agriculture (USDA) grades and standards.

Tobacco grades are evaluated based on factors including, but not limited to, the leaf stalk position, leaf size, leaf colour, leaf uniformity and integrity, ripeness, texture, elasticity, sheen (related with the intensity and the depth of coloration of the leaf as well as the shine), hygroscopicity (the faculty of the tobacco leaves to absorb and to retain the ambient moisture), and green nuance or cast.

Leaf grade can be determined using standard methods known in the art, for example, using an Official Standard Grade published by the Agricultural Marketing Service of the US Department of Agriculture (7 U.S.C. §511 ). See, e.g., Official Standard Grades for Burley Tobacco (U.S. Type 31 and Foreign Type 93), effective November 5, 1990 (55 F.R. 40645); Official Standard Grades for Flue-Cured Tobacco (U.S. Types 11, 12, 13, 14 and Foreign Type 92), effective March 27, 1989 (54 F.R. 7925); Official Standard Grades for Pennsylvania SeedleafTobacco (U.S. Type 41), effective January 8, 1965 (29 F.R. 16854); Official Standard Grades for Ohio Cigar-Leaf Tobacco (U.S. Types 42, 43, and 44), effective December 8, 1963 (28 F.R. 11719 and 28 F.R. 11926); Official Standard Grades for Wisconsin Cigar-Binder Tobacco (U.S. Types 54 and 55), effective November 20, 1969 (34 F.R. 17061); Official Standard Grades for Wisconsin Cigar-Binder Tobacco (U.S. Types 54 and 55), effective November 20, 1969 (34 F.R. 17061); Official Standard Grades for Georgia and Florida ShadeGrown Cigar-Wrapper Tobacco (U.S. Type 62), Effective April 1971. A USDA grade index value can be determined according to an industry accepted grade index. See, e.g., Bowman et al,Tobacco Science, 32:39-40(1988); Legacy Tobacco Document Library (Bates Document #523267826-523267833, July 1, 1988, Memorandum on the Proposed Burley Tobacco Grade Index); and Miller et al., 1990, Tobacco Intern., 192:55-57.

In one aspect, a USDA grade index is a 0-100 numerical representation of federal grade received and is a weighted average of all stalk positions. A higher grade index indicates higher quality. Alternatively, leaf grade may be determined via hyper-spectral imaging. See e.g., WO 2011/027315.

In one embodiment, a tobacco plant of the present invention provides tobacco of commercially acceptable grade.

Suitably, the tobacco plant of the present invention provides cured tobacco of commercially acceptable grade.

In one embodiment, a tobacco plant of the present invention is capable of producing leaves having a USDA grade index value of at least about 70% of the USDA grade index value of leaves of a comparable plant when grown in similar growth conditions. Suitably, tobacco plants disclosed herein may be capable of producing leaves having a USDA grade index value of at least about 65%, at least about 70%, at least about 75%, at least about 80%, at least about 85%, at least about 90%, at least about 95%, or at least about 98% of the USDA grade index value of a control plant when grown in similar growth conditions. Suitably, tobacco plants disclosed herein may be capable of producing leaves having a USDA grade index value of between 65% and 130%, between 70% and 130%, between 75% and 130%, between 80% and 130%, between 85% and 130%, between 90% and 130%, between 95% and 130%, between 100% and 130%, between 105% and 130%, between 110% and 130%, between 115% and 130%, or between 120% and 130% of the USDA grade index value of a comparable plant.

In one aspect, the tobacco plant of the present invention is capable of producing leaves having a USDA grade index value of at least 50. Suitably, tobacco plants disclosed herein may be capable of producing leaves having a USDA grade index value of 55 or more, 60 or more, 65 or more, 70 or more, 75 or more, 80 or more, 85 or more, 90 or more, and 95 or more.

Unless specified otherwise, used herein, tobacco yield refers to cured leaf yield which is calculated based on the weight of cured tobacco leaves per acre under standard field conditions following standard agronomic and curing practice.

In one aspect, a tobacco plant of the present invention has a yield between 50% and 150%, between 55% and 145%, between 60% and 140%, between 65% and 135%, between 70% and 130%, between 75% and 125%, between 80% and 120%, between 85% and 115%, between 90% and 110%, between 95% and 105%, 50% and 100%, between 55% and 100%, between 60% and 100%, between 65% and 100%, between 70% and 100%, between 75% and 100%, between 80% and 100%, between 85% and 100%, between 90% and 100%, between 95% and 100%, between 100% and 150%, between 105% and 150%, between 110% and 150%, between 115% and 150%, between 120% and 150%, between 125% and 150%, between 130% and 150%, between 135% and 150%, between 140% and 150%, or between 145% and 150% of the yield of a comparable plant when grown in similar field conditions.

In another aspect, the yield of a tobacco plant of the present invention is approximately 1.5, 1.6, 1.7, 1.8, 1.9, 2.0, 2.1, 2.2, 2.3, 2.4, 2.5, 2.6, 27, 2.8, 29, or 3.0 times of the yield of the a comparable plant when grown in similar field conditions.

In another aspect, the yield of a tobacco plant of the present invention is comparable to the yield of a cured comparable plant when grown in similar field conditions.

In one aspect, a tobacco plant of the present invention provides a yield selected from the group consisting of about between 1200 and 3500, between 1300 and 3400, between 1400 and 3300, between 1500 and 3200, between 1600 and 3100, between 1700 and 3000, between 1800 and 2900, between 1900 and 2800, between 2000 and 2700, between 2100 and 2600, between 2200 and 2500, and between 2300 and 2400 lbs/acre.

In another aspect, a tobacco plant of the present invention provides a yield selected from the group consisting of about between 1200 and 3500, between 1300 and 3500, between 1400 and 3500, between 1500 and 3500, between 1600 and 3500, between 1700 and 3500, between 1800 and 3500, between 1900 and 3500, between 2000 and 3500, between 2100 and 3500, between 2200 and 3500, between 2300 and 3500, between 2400 and 3500, between 2500 and 3500, between 2600 and 3500, between 2700 and 3500, between 2800 and 3500, between 2900 and 3500, between 3000 and 3500, and between 3100 and 3500 Ibs/acre.

In a further aspect, a tobacco plant of the present invention provides a yield selected from the group consisting of about between 1200 and 3500, between 1200 and 3400, between 1200 and 3300, between 1200 and 3200, between 1200 and 3100, between 1200 and 3000, between 1200 and 2900, between 1200 and 2800, between 1200 and 2700, between 1200 and 2600, between 1200 and 2500, between 1200 and 2400, between 1200 and 2300, between 1200 and 2200, between 1200 and 2100, between 1200 and 2000, between 1200 and 1900, between 1200 and 1800, between 1200 and 1700, between 1200 and 1600, between 1200 and 1500, and between 1200 and 1400 Ibs/acre.

### PLANT BREEDING

Provided herein, but not forming part of the claimed invention, is a method of producing a tobacco plant having reduced content of a tobacco specific nitrosamine (TSNA) or a precursor of a TSNA, comprising:
a. crossing a donor tobacco plant which produces a reduced content of a tobacco specific nitrosamine (TSNA) or a precursor of a TSNA and comprises a deregulated cation efflux protein with a recipient tobacco plant that does not produce reduced content of a tobacco specific nitrosamine (TSNA) or a precursor of a TSNA and possesses commercially desirable traits;
b. isolating genetic material from a progeny of said donor plant crossed with said recipient plant; and
c. performing molecular marker-assisted selection with a molecular marker comprising:
d. identifying an introgressed region comprising a mutation in a polynucleotide sequence encoding a protein defined in a.

The molecular marker assisted selection may comprise performing PCR to identify a introgressed nucleic acid sequence comprising
a. A nucleotide sequence comprising SEQ ID No. 34;
b. A nucleotide sequence having at least 70% sequence identity to the entirety of the sequence set forth in SEQ ID No. 34, wherein said nucleotide sequence encodes a polypeptide involved in cation efflux in a plant;
c. A nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
d. A nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or2-117, or2-117, or 2-120, or 2-125, or 2-128, or 2-130 of SEQ ID No. 3, 6, 9, 12, 15, 18, 21, 24, 27 or 30;
e. A nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
f. A nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant;

### TOBACCO PLANT AND PART THEREOF

The term "tobacco plant" as used herein refers to a plant in the genus *Nicotiana* that is used in the production of tobacco industry products. Non-limiting examples of suitable tobacco plants include *N. tabacum* and *N. rustica* (for example, LA B21 , LN KY171 , TI 1406, Basma, Galpao, Perique, Beinhart 1000-1 , and Petico).

Thus, in one embodiment a tobacco plant does include *Nicotiana plumbaginifolia.*

The tobacco material can be derived from varieties of *Nicotiana tabacum* species, commonly known as Burley varieties, flue or bright varieties, dark varieties and oriental/Turkish varieties. In some embodiments, the tobacco material is derived from a Burley, Virginia, flue-cured, air-cured, fire-cured, Oriental, or a dark tobacco plant. The tobacco plant may be selected from Maryland tobacco, rare tobacco, speciality tobacco, expanded tobacco or the like.

The use of tobacco cultivars and elite tobacco cultivars is also contemplated herein. The tobacco plant for use herein may therefore be a tobacco variety or elite tobacco cultivar. Particularly useful *Nicotiana tabacum* varieties include Burley type, dark type, flue-cured type, and Oriental type tobaccos.

In some embodiments, the tobacco plant may be, for example, selected from one or more of the following varieties: N. tabacum AA 37-1 , N. tabacum B 13P, N. tabacum Xanthi (Mitchell-Mor), N.tabacum KT D#3 Hybrid 107, N. tabacum Bel-W3, N.tabacum 79-615, N.tabacum Samsun Holmes NN, F4 from cross N.tabacum BU21 x N.tabacum Hoja Parado, line 97, N.tabacum KTRDC#2 Hybrid 49, N.tabacum KTRDC#4 Hybrid 1 10, N.tabacum Burley 21 , N.tabacum PM016, N.tabacum KTRDC#5 KY 160 SI, N.tabacum KTRDC#7 FCA, N.tabacum KTRDC#6 TN 86 SI, N.tabacum PM021 , N.tabacum K 149, N.tabacum K 326, N.tabacum K 346, N.tabacum K 358, N.tabacum K 394, N.tabacum K 399, N.tabacum K 730, N.tabacum KY 10, N.tabacum KY 14, N.tabacum KY 160, N.tabacum KY 17, N.tabacum KY 8959, N.tabacum KY 9, N.tabacum KY 907, N.tabacum MD 609, N.tabacum McNair 373, N.tabacum NC 2000, N.tabacum PG 01 , N.tabacum PG 04, N.tabacum P01 , N.tabacum P02, N.tabacum P03, N.tabacum RG 1 1 , N.tabacum RG 17, N.tabacum RG 8, N.tabacum Speight G-28, N.tabacum TN 86, N.tabacum TN 90, N.tabacum VA 509, N.tabacum AS44, N.tabacum Banket A1 , N.tabacum Basma Drama B84/31 , N.tabacum Basma I Zichna ZP4/B, N.tabacum Basma Xanthi BX 2A, N.tabacum Batek, N.tabacum Besuki Jember, N.tabacum C104, N.tabacum Coker 319, N.tabacum Coker 347, N.tabacum Criollo Misionero, N.tabacum PM092, N.tabacum Delcrest, N.tabacum Djebel 81 , N.tabacum DVH 405, N.tabacum Galpao Comum, N.tabacum HB04P, N.tabacum Hicks Broadleaf, N.tabacum Kabakulak Elassona, N.tabacum PM102, N.tabacum Kutsage E1 , N.tabacum KY 14xL8, N.tabacum KY 171 , N.tabacum LA BU 21 , N.tabacum McNair 944, N.tabacum NC 2326, N.tabacum NC 71 , N.tabacum NC 297, N.tabacum NC 3, N.tabacum PVH 03, N.tabacum PVH 09, N.tabacum PVH 19, N.tabacum PVH 21 10, N.tabacum Red Russian, N.tabacum Samsun, N.tabacum Saplak, N.tabacum Simmaba, N.tabacum Talgar 28, N.tabacum PM132, N.tabacum Wislica, N.tabacum Yayaldag, N.tabacum NC 4, N.tabacum TR Madole, N.tabacum Prilep HC-72, N.tabacum Prilep P23, N.tabacum Prilep PB 156/1 , N.tabacum Prilep P12-2/1 , N.tabacum Yaka JK-48, N.tabacum Yaka JB 125/3, N.tabacum TI-1068, N.tabacum KDH-960, N.tabacum TI-1070, N.tabacum TW136, N.tabacum PM204, N.tabacum PM205, N.tabacum Basma, N.tabacum TKF 4028, N.tabacum L8, N.tabacum TKF 2002, N.tabacum TN90, N.tabacum GR141 , N.tabacum Basma xanthi, N.tabacum GR149, N.tabacum GR153, and N. tabacum Petit Havana.

Non-limiting examples of varieties or cultivars are: BD 64, CC 101 , CC 200, CC 27, CC 301 , CC 400, CC 500, CC 600, CC 700, CC 800, CC 900, Coker 176, Coker 319, Coker 371 Gold, Coker 48, CD 263, DF91 1 , DT 538 LC Galpao tobacco, GL 26H, GL 350, GL 600, GL 737, GL 939, GL 973, HB 04P, HB 04P LC, HB3307PLC, Hybrid 403LC, Hybrid 404LC, Hybrid 501 LC, K 149, K 326, K 346, K 358, K394, K 399, K 730, KDH 959, KT 200, KT204LC, KY10, KY14, KY 160, KY 17, KY 171 , KY 907, KY907LC, KTY14xL8 LC, Little Crittenden, McNair 373, McNair 944, msKY 14xL8, Narrow Leaf Madole, Narrow Leaf Madole LC, NBH 98, N-126, N-777LC, N-7371 LC, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, PD 7302 LC, PD 7309 LC, PD 7312 LC 'Periq'e' tobacco, PVH03, PVH09, PVH19, PVH50, PVH51, R 610, R 630, R 7-1 1 , R 7-12, RG 17, RG 81, RG H51 , RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, VA 309, VA359, AA 37-1, B 13P, Xanthi (Mitchell-Mor), Bel-W3, 79-615, Samsun Holmes NN, KTRDC number 2 Hybrid 49, Burley 21 , KY 8959, KY 9, MD 609, PG 01 , PG 04, P01 , P02, P03, RG 1 1, RG 8, VA 509, AS44, Banket A1 , Basma Drama B84/31 , Basma I Zichna ZP4/B, Basma Xanthi BX 2A, Batek, Besuki Jember, C104, Coker 347, Criollo Misionero, Delcrest, Djebel 81, DVH 405, Galpao Comum, HB04P, Hicks Broadleaf, Kabakulak Elassona, Kutsage E1, LA BU 21, NC 2326, NC 297, PVH 21 10, Red Russian, Samsun, Saplak, Simmaba, Talgar 28, Wislica, Yayaldag, Prilep HC-72, Prilep P23, Prilep PB 156/1, Prilep P12-2/1, Yaka JK-48, Yaka JB 125/3, TI-1068, KDH-960, TI-1070, TW136, Basma, TKF 4028, L8, TKF 2002, GR141, Basma xanthi, GR149, GR153, Petit Havana. Low converter subvarieties of the above, even if not specifically identified herein, are also contemplated.

In one embodiment the tobacco plant is a Burley type tobacco plant, suitably a Burley PH2517.

In one embodiment the plant propagation material may be obtainable from a tobacco plant of the invention.

A "plant propagation material" as used herein refers to any plant matter taken from a plant from which further plants may be produced. Suitably, a plant propagation material may be selected from a seed, plant calli and plant clumps.

Suitably the plant propagation material may be a seed. Suitably, a plant propagation material may be plant calli. Suitably the plant propagation material may be plant clumps.

In one embodiment the cell (e.g. tobacco cell), tobacco plant and/or plant propagation material may be obtainable (e.g. obtained) by a method according to the invention. In one embodiment the cell (e.g., tobacco cell), tobacco plant and/or plant propagation material of the invention may comprise:
A nucleic acid sequence encoding a deregulated cation efflux protein;
a nucleotide sequence comprising SEQ ID No. 34;
a nucleotide sequence having at least 70% sequence identity to the entirety of the sequence set forth in SEQ ID No. 34, wherein said nucleotide sequence encodes a polypeptide involved in cation efflux in a plant;
a nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
a nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
a nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30; or
a nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant.

Suitably a tobacco plant according to the present invention may have a e.g. reduced content of a tobacco specific nitrosamine (TSNA) or a precursor of a TSNA when compared to an unmodified tobacco plant, wherein the modification is the expression of a deregulation cation efflux protein according to the present invention or the expression of a nucleic acid sequence according to the present invention.

In one embodiment the tobacco plant in accordance with the present invention comprises a tobacco cell of the invention.

In another embodiment the plant propagation material may be obtainable (e.g. obtained) from a tobacco plant of the invention.

In one embodiment there is provided the use of a tobacco plant as described herein to breed a tobacco plant.

The present invention also provides in another embodiment the use of a tobacco plant of the foregoing embodiments for the production of a tobacco industry product.

In another embodiment there is provided the use of a tobacco plant of the invention to grow a crop.

In one embodiment there is provided the use of a cell as provided for in the foregoing embodiments for production of a tobacco industry product.

In one embodiment the present invention provides a cell culture (e.g. in *in vitro* culture).

The tobacco cell culture may be a cell suspension culture. These cells cultured *in vitro* may be incorporated into a tobacco industry product, e.g. as a substitute for conventional tobacco particles, shreds, fine cut or long cut tobacco lamina, as an additive ingredient or as both a substitute and an additive. Suitably, the cell culture may produce nicotine.

In one embodiment there is provided the use of a cell culture, e.g. a harvested and/or processed cell culture according to the present invention for the production of a tobacco industry product.

The cells harvested from an *in vitro* culture may be dried, e.g. freeze-dried, for example to produce a powder.

In one embodiment, the cell culture is a tobacco cell culture.

The skilled person will be aware of known methods for establishing *in vitro* cultures of tobacco cells. By way of example only the following method may be used: collecting seeds form a tobacco plant of interest and sterilising their exterior to eliminate unwanted organisms, planting said seeds to grown a tobacco plant of interest, removing tissue from the tobacco plant (for example, from the tobacco stem) for use as an explant, establishing a callus culture form the tobacco explant, establishing a cell suspension culture from the callus culture, and harvesting culture material (e.g. including tobacco cells) to produce a tobacco cell culture. The tobacco cells can be harvested by various methods, including filtration, e.g. vacuum filtration. The sample may be washed in the filter by adding water and the remaining liquid removed with the filtration, e.g. vacuum filtration.

The harvested tobacco cell culture may be further processed, e.g. dried, such as air-dried and/or freeze-dried. The harvested tobacco cell culture or dried harvested tobacco cell culture may be incorporated into tobacco industry products according to the present invention.

In one embodiment, the cell culture is a yeast cell culture. In one embodiment the cell (e.g. yeast cell) may be further modified to comprise one or more sequences that increases nicotinic alkaloid biosynthesis. Suitably these one or more sequences may be incorporated into a nucleic acid construct that is suitable for cell (e.g. yeast cell) transformation. The one or more sequences may be overexpressed in the cell (e.g. yeast cell). The sequences may be selected from one or more of the following genes: MPO (or Methylputrescine Oxidase or MPO1 or MPO2); A622 (or Isoflavone reductase-like protein or Isoflavone reductase homolog or Isoflavone reductase-like protein); BBL (or Berberine bridge enzyme or Berberine bridge enzyme-like or BBE or NBB1); PMT (or Putrescine N-Methyltransferase or putrescine methyltransferase or S-adenosyl-L-methionine:putrescine N-methyltransferase or PMT or PMT1 or PMT2 or PMT3 or PMT4) and QPT (or quinolinate phosphoribosyltransferase). In one embodiment the sequences may be selected from one or more of the following genes: BBL, A622, PMT and MPO (MPO1 or MPO2). Genes suitable for modification of in this way may be taught in US2016032299 for example.

### PRODUCTS

The present invention also provides for products obtainable or obtained from tobacco according to the present invention.

In one embodiment there is provided the use of a tobacco plant of the invention to produce a tobacco leaf.

Suitably the tobacco leaf may be subjected to downstream applications such as processing.

Thus in one embodiment the use of the foregoing embodiment may provide a processed tobacco leaf. Suitably the tobacco leaf may be subjected to curing, fermenting, pasteurising or combinations thereof. In another embodiment the tobacco leaf may be cut. In some embodiments the tobacco leaf may be cut before or after being subjected to curing, fermenting, pasteurising or combinations thereof.

In one embodiment the present invention provides a harvested leaf of a tobacco plant of the invention.

In a further embodiment the harvested leaf may be obtainable (e.g. obtained) from a tobacco plant propagated from a propagation material of the present invention.

In another embodiment there is provided a harvest leaf obtainable from a method or use of the present invention.

Suitably the harvested leaf may be a cut harvested leaf.

In some embodiments the harvested leaf may comprise viable tobacco cells. In other embodiments the harvested leaf may be subjected to further processing.

There is also provided a processed tobacco leaf.

The processed tobacco leaf may be obtainable from a tobacco plant of the invention. Suitably the processed tobacco leaf may be obtainable from a tobacco plant obtained in accordance with any of the methods and/or uses of the present invention.

In another embodiment the processed tobacco leaf may be obtainable from a tobacco plant propagated form a tobacco plant propagation material according to the present invention. The processed tobacco leaf of the present invention may be obtainable by processing a harvested leaf of the invention.

The term "processed tobacco leaf" as used herein refers to a tobacco leaf that has undergone one or more processing steps to which tobacco is subjected to in the art. A "processed tobacco leaf' comprises no or substantially no viable cells.

The term "viable cells" refers to cells which are able to grow and/or are metabolically active. Thus, if a cell is said to not be viable, also referred to as "non-viable" then a cell does not display the characteristics of a viable cell.

The term "substantially no viable cells" means that less than about 5% of the total cells are viable. Preferably, less than about 3%, more preferably less than about 1%, even more preferably less than about 0.1% of the total cells are viable.

In one embodiment the processed tobacco leaf may be processed by one or more of: curing, fermenting and/or pasteurising.

Suitably the processed tobacco leaf may be processed by curing.

Tobacco leaf may be cured by any method known in the art. In one embodiment tobacco leaf may be cured by one or more of the curing methods selected from the group consisting of: air curing, fire curing, flue curing and sun curing.

Suitably the tobacco leaf may be air cured.

Typically air curing is achieved by hanging tobacco leaf in well-ventilated barns and allowing to dry. This is usually carried out over a period of four to eight weeks. Air curing is especially suitable for burley tobacco.

Suitably the tobacco leaf may be fire cured. Fire curing is typically achieved by hanging tobacco leaf in large barns where fires of hardwoods are kept on continuous or intermittent low smoulder and usually takes between three days and ten weeks, depending on the process and the tobacco.

In another embodiment the tobacco leaf may be flue cured. Flue curing may comprise stringing tobacco leaves onto tobacco sticks and hanging them from tier-poles in curing barns. The barns usually have a flue which runs from externally fed fire boxes. Typically this results in tobacco that has been heat-cured without being exposed to smoke. Usually the temperature will be raised slowly over the course of the curing with the whole process taking approximately 1 week.

Suitably the tobacco leaf may be sun cured. This method typically involves exposure of uncovered tobacco to the sun.

Suitably the processed tobacco leaf may be processed by fermenting.

Fermentation can be carried out in any manner known in the art. Typically during fermentation, the tobacco leaves are piled into stacks (a bulk) of cured tobacco covered in e.g. burlap to retain moisture. The combination of the remaining water inside the leaf and the weight of the tobacco generates a natural heat which ripens the tobacco. The temperature in the centre of the bulk is monitored daily. In some methods every week, the entire bulk is opened. The leaves are then removed to be shaken and moistened and the bulk is rotated so that the inside leaves go outside and the bottom leaves are placed on the top of the bulk. This ensures even fermentation throughout the bulk. The additional moisture on the leaves, plus the actual rotation of the leaves themselves, generates heat, releasing the tobacco's natural ammonia and reducing nicotine, while also deepening the colour and improving the tobacco's aroma. Typically the fermentation process continues for up to 6 months, depending on the variety of tobacco, stalk position on the leaf, thickness and intended use of leaf.

Suitably the processed tobacco leaf may be processed by pasteurising. Pasteurising may be particularly preferred when the tobacco leaf will be used to make a smokeless tobacco industry product, most preferably snus.

Tobacco leaf pasteurisation may be carried out by any method known in the art. For example pasteurisation may be carried out as detailed in J Foulds, L Ramstrom, M Burke, K Fagerstrom. Effect of smokeless tobacco (snus) on smoking and public health in Sweden. Tobacco Control (2003) 12:349-359.

During the production of snus pasteurisation is typically carried out by a process in which the tobacco is heat treated with steam for 24-36 hours (reaching temperatures of approximately 100°C). This results in an almost sterile product and without wishing to be bound by theory one of the consequences of this is believed to be a limitation of further TSNA formation.

In one embodiment the pasteurisation may be steam pasteurisation.

In some embodiments the processed tobacco leaf may be cut. The processed tobacco leaf may be cut before or after processing. Suitably, the processed tobacco leaf may be cut after processing.

In one embodiment, the use of the foregoing embodiment may provide reconstituted tobacco. "Reconstituted" as used herein may also be referred to as recon, recycled or homogenized sheet tobacco and refers to tobacco material generated from remnants of tobacco leaf after processing. Reconstituted tobacco allows the production of a consistent, high quality blend and allows the adjustment of the ratio of individual components.

Reconstituted tobacco may be nano fibre recon (nanofibers can be extracted in solid or liquid form), paper making recon (which uses stems, scraps, and midribs, etc. as the raw material) or slurry type recon (which uses a mixture of fines and tobacco stems, ground to power, mixed with water and vegetable binding agent. The soluble residue is formed to sheets by extracting the water.)

Any method known in the art may be used for making reconstituted tobacco, for example see CORESTA Congress, Sapporo, 2012, Smoke Science/Product Technology Groups, SSPT 12.

In some embodiments the tobacco plant, harvested leaf of a tobacco plant and/or processed tobacco leaf may be used to extract nicotine. The extraction of nicotine can be achieved using any method known in the art. For example a method for extracting nicotine from tobacco is taught in US 2,162,738.

In one aspect, the present invention provides cured tobacco material made from a tobacco plant or part thereof according to the invention.

In another aspect, the present invention provides a tobacco blend comprising tobacco material made from a tobacco plant or part thereof according to the present invention, or from a tobacco cell culture according to the present invention. In one aspect, the present invention provides a tobacco blend comprising cured tobacco material according to the present invention.

Suitably, the tobacco blend according to the present invention may comprise approximately 10%, 20%, 30%, 40%, 50%, 60%, 70%, 80% or 90% tobacco from a tobacco plant or part thereof according to the present invention, or from a tobacco cell culture according to the present invention. Suitably, the tobacco blend may comprise approximately 10% tobacco from a tobacco plant or part thereof according to the present invention, or from a tobacco cell culture according to the present invention. Suitably, the tobacco blend may comprise approximately 20% tobacco from a tobacco plant or part thereof according to the present invention, or from a tobacco cell culture according to the present invention. Suitably, the tobacco blend may comprise approximately 30% tobacco from a tobacco plant or part thereof according to the present invention, or from a tobacco cell culture according to the present invention. Suitably, the tobacco blend may comprise approximately 40% tobacco from a tobacco plant or part thereof according to the present invention, or from a tobacco cell culture according to the present invention. Suitably, the tobacco blend may comprise approximately 50% tobacco from a tobacco plant or part thereof according to the present invention, or from a tobacco cell culture according to the present invention. Suitably, the tobacco blend may comprise approximately 60% tobacco from a tobacco plant or part thereof according to the present invention, or from a tobacco cell culture according to the present invention. Suitably, the tobacco blend may comprise approximately 70% tobacco from a tobacco plant or part thereof according to the present invention, or from a tobacco cell culture according to the present invention. Suitably, the tobacco blend may comprise approximately 80% tobacco from a tobacco plant or part thereof according to the present invention, or from a tobacco cell culture according to the present invention. Suitably, the tobacco blend may comprise approximately 90% tobacco from a tobacco plant or part thereof according to the present invention, or from a tobacco cell culture according to the present invention.

In one aspect, a tobacco blend product of the present invention comprises at least about 5, 10, 20, 30, 40, 50, 60, 70, 80, 90, or 95 percent by dry weight of tobacco cured from a tobacco plant or part thereof according to the present invention, or a tobacco cell culture according to the present invention.

Suitably, the cured tobacco material may be air cured. Suitably, the cured tobacco material may be flue cured. Suitably, the cured tobacco material may be sun cured. Suitably, the cured tobacco material may be fire cured.

A tobacco industry product or smoking article according to the present invention may comprise the tobacco material (e.g. cured tobacco material or reconstituted tobacco material) according to the present invention.

In another aspect the present invention provides a tobacco industry product.

In one embodiment the tobacco industry product according to the present invention may be a blended tobacco industry product. Suitably, the tobacco blend may comprise cured tobacco material according to the present invention.

In one embodiment the tobacco industry product may be prepared from a tobacco plant of the invention or a part thereof.

Suitably the tobacco plant or part thereof may be propagated from a tobacco plant propagation material according to the present invention.

The term "part thereof" as used herein in the context of a tobacco plant refers to a portion of the tobacco plant. Suitably, the "part thereof" may be a leaf, root or stem of a tobacco plant or the flowers. Suitably, the "part thereof" may be a leaf, root or stem of a tobacco plant.

### TOBACCO INDUSTRY PRODUCT

As used herein, the term "tobacco industry product" is intended to include combustible smoking articles such as cigarettes, cigarillos, cigars, tobacco for pipes or for roll-your-own cigarettes, (whether based on tobacco, tobacco derivatives, expanded tobacco, reconstituted tobacco, tobacco substitutes or other smokable material), non-combustible aerosol provision systems such as heating products that release compounds from substrate materials without burning such as electronic cigarettes, tobacco heating products, and hybrid systems to generate aerosol from a combination of substrate materials, for example hybrid systems containing a liquid or gel or solid substrate, as well as aerosolizable substrate materials used within these aerosol provision systems; and aerosol-free delivery articles such as lozenges, gums, patches, articles comprising breathable powders and smokeless tobacco industry products such as snus and snuff, which aerosol-free delivery articles may or may not deliver nicotine.

In one embodiment the tobacco industry product may be prepared from (e.g. may comprise) a tobacco plant of the invention or a part thereof.

Suitably the tobacco plant or part thereof may be propagated from a tobacco plant propagation material according to the present invention.

The term "part thereof" as used herein in the context of a tobacco plant refers to a portion of the tobacco plant. Preferably the "part thereof' is a leaf of a tobacco plant.

In another embodiment the tobacco industry product may be prepared from a harvested leaf of the invention. In a further embodiment the tobacco industry product may be prepared from a processed tobacco leaf of the invention.

Suitably the tobacco industry product may be prepared from a tobacco leaf processed by one or more of: curing, fermenting and/or pasteurising.

Suitably the tobacco industry product may comprise a cut tobacco leaf, optionally processed as per the foregoing embodiment.

In another embodiment, the tobacco industry product may be prepared from a tobacco cell culture according to the present invention.

In another embodiment, the tobacco industry product may be prepared from (e.g. may comprise) a cured tobacco material according to the present invention.

In another embodiment, the tobacco industry product may be prepared from (e.g. may comprise) a tobacco blend according to the present invention.

In one embodiment the tobacco industry product may be a smoking article.

As used herein, the term "smoking article" can include smokeable products, such as rolling tobacco, cigarettes, cigars and cigarillos whether based on tobacco, tobacco derivatives, expanded tobacco, reconstituted tobacco or tobacco substitutes.

In another embodiment the tobacco industry product may be a smokeless tobacco industry product.

The term "smokeless tobacco industry product" as used herein refers to a tobacco industry product that is not intended to be smoked and/or subjected to combustion.

Smokeless tobacco industry products (including heat-not-burn materials) may contain tobacco in any form, including dried particles, shreds, granules, powders, or slurry, deposited on, mixed in, surrounded by, or combined with other ingredients in any format, such as flakes, films, tabs, foams, or beads.

In one embodiment a smokeless tobacco industry product may include snus, snuff, chewing tobacco or the like.

In one embodiment, the tobacco industry product is a combustible smoking article, selected from the group consisting of a cigarette, a cigarillo and a cigar.

In one embodiment, the tobacco industry product comprises one or more components of a combustible smoking article, such as a filter, a filter rod, a filter rod segments, tobacco, a tobacco rod, a tobacco rod segment, a spill, an additive release component such as a capsule, a thread, beads, a paper such as a plug wrap, a tipping paper or a cigarette paper. In one embodiment, the tobacco industry product is a non-combustible aerosol provision system.

In one embodiment, the tobacco industry product comprises one or more components of a non-combustible aerosol provision system, such as a heater and an aerosolizable substrate.

In one embodiment, the aerosol provision system is an electronic cigarette also known as a vaping device.

In one embodiment the electronic cigarette comprises a heater, a power supply capable of supplying power to the heater, an aerosolizable substrate such as a liquid or gel, a housing and optionally a mouthpiece.

In one embodiment the aerosolizable substrate is contained in a substrate container. In one embodiment the substrate container is combined with or comprises the heater.

In one embodiment, the tobacco industry product is a heating product which releases one or more compounds by heating, but not burning, a substrate material. The substrate material is an aerosolizable material which may be for example tobacco or other non-tobacco products, which may or may not contain nicotine. In one embodiment, the heating product is a tobacco heating product.

In one embodiment, the heating product is an electronic device.

In one embodiment, the tobacco heating product comprises a heater, a power supply capable of supplying power to the heater, an aerosolizable substrate such as a solid or gel material.

In one embodiment the heating product is a non-electronic article.

In one embodiment the heating product comprises an aerosolizable substrate such as a solid or gel material and a heat source which is capable of supplying heat energy to the aerosolizable substrate without any electronic means, such as by burning a combustion material, such as charcoal.

In one embodiment the heating product also comprises a filter capable of filtering the aerosol generated by heating the aerosolizable substrate.

In some embodiments the aerosolizable substrate material may comprise a vapour or aerosol generating agent or a humectant, such as glycerol, propylene glycol, triacetin or diethylene glycol.

In one embodiment, the tobacco industry product is a hybrid system to generate aerosol by heating, but not burning, a combination of substrate materials. The substrate materials may comprise for example solid, liquid or gel which may or may not contain nicotine. In one embodiment, the hybrid system comprises a liquid or gel substrate and a solid substrate. The solid substrate may be for example tobacco or other non-tobacco products, which may or may not contain nicotine. In one embodiment, the hybrid system comprises a liquid or gel substrate and tobacco.

In a further embodiment the tobacco industry product may be a tobacco heating device or hybrid device or e-cigarette or the like.

Typically in tobacco heating devices or hybrid devices, an aerosol is generated by the transfer of heat from a heat source to a physically separate aerosol-forming substrate or material, which may be located within, around or downstream of the heat source. During smoking, volatile compounds are released from the aerosol-forming substrate by heat transfer from the heat source and entrained in air drawn through the smoking article. As the released compounds cool, they condense to form an aerosol that is inhaled by the user. Aerosol-generating articles and devices for consuming or smoking tobacco heating devices are known in the art. They can include, for example, electrically heated aerosol-generating devices in which an aerosol is generated by the transfer of heat from one or more electrical heating elements of the aerosol-generating device to the aerosol-forming substrate of a tobacco heating device.

Suitably the tobacco heating device may be an aerosol-generating device.

Preferably the tobacco heating device may be a heat-not-burn device. Heat-not-burn devices are known in the art and release compounds by heating, but not burning, tobacco.

An example of a suitable, heat-not-burn device may be one taught in WO2013/034459 or GB2515502.

In one embodiment the aerosol-forming substrate of a tobacco heating device may be a tobacco industry product in accordance with the present invention.

In one embodiment the tobacco heating device may be a hybrid device.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise Thus, for example, reference to "a protein" or "a nucleic acid sequence" includes a plurality of such candidate agents and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention will now be described, by way of example only, with reference to the following Figures and Examples.

### EXAMPLES

### Example 1

### Methods and Materials

### Plant material

Plant material from *Nicotiana* including *Nicotiana tabacum* e.g.: Burley and Virginia varieties and other species e.g. *Nicotiana rustica.*

### Bacterial Strains

The *Escherichia coli (E. coli*) strain TOP10 F- [F- mcrA Δ (mrr-hsdRMS-mcrBC) Φ80lacZΔM15 Δ lacX74 recA1 araD139 Δ( araleu)7697 galU galK rpsL (StrR) endA1 nupG] were used for cloning and plasmid DNA production. *E. coli* strain TOP10 were transformed by means of a chemical method.

*Agrobacterium tumefaciens* strain GV3101::pMP90 was used for transformation of binary vectors for in planta assays.

### Alkaloid measurement

Relative content of pyridine alkaloids was determined by reversed phase high performance liquid chromatography with tandem mass spectrometry (LC-MS/MS). Chromatographic separation is achieved using a Gemini-NX column (100 mm × 3.0 mm, particle size 3 µm, Phenomenex) and gradient chromatographic separation using 6.5 mM ammonium acetate buffer (aq) (pH10) and Methanol.

Mass Spectrometer operates in electrospray (ESI) positive mode using scheduled MRM data acquisition. Two MRM transitions are monitored for each analyte and one for the isotope labelled internal standard.

| Analyte | Precursor Ion Daughter Ion (quant/confirm) | |
|---|---|---|
| Nicotine | 163.1 | 130/106 |
| Nicotine d4 | 167.1 | 134.1 |
| Anabasine | 163.1 | 80/120 |
| Anatabine | 161.1 | 144/80 |
| Nornicotine | 149.1 | 80/130 |
| Nornicotine | d4153.1 | 84.1 |
| PON | 176.1 | 106.0/148 |
| PON d4 | 183.1 | 110.0 |

### Statistical Analysis

Statistical significances based on one-way ANOVA analyses is performed with Prism 5.01 software (GraphPad Software).

### Virus-induced gene silencing (VIGS)

The TRV vector comprising both SEQ ID No. 49 (TRV RNA1) and SEQ ID No. 50 (TRV RNA2) comprising SEQ ID No. 51 (targeted nucleotide sequence) were separately propagated in A. *tumefaciens.* These cultures were mixed (1:1) and syringe-infiltration into 2-week-old tobacco plants. The silencing effect was assessed two weeks post-virus infection by assessing the expression level of the target gene.

### Results

Nornicotine content of 5-week-old tobacco leaves expressing the VIGS target sequence (SEQ ID No. 51) and the VIGS control sequence (SEQ ID No. 52) is shown in Figure 2.

The nornicotine content is represented relative to a control (SEQ ID No. 52) and comprises three biological replicates analysed by one-way ANOVA and Tukey's multiple-comparison post-test. Values are shown as means ± SEM. Asterisks indicate statistical significance of P value ≤ 0.001.

### Conclusions

Silencing of a cation efflux protein encoded by SEQ ID No. 2 results in an increase in nornicotine content.

### Example 2

### Methods and Materials

### Cloning

### GDB-Cation efflux expression vector

The gene sequence (SEQ ID No. 2) was amplified from a Gateway^{™} compatible cDNA library using primers (M13.fw [SEQ ID No. 36] and M13.rv [SEQ ID No. 37]) located outside the attB1 and attB2 sites flanking the gene sequence. The gene sequence was then transferred to the GDB expression vector (SEQ ID No. 38).

### GDB-Cation efflux_AS expression vector

AS construct (SEQID No. 39) was generated by two-step amplification and Gateway^{™} cloning, using the GDB-Cation efflux expression vector as template, a first set of gene-specific primers (Cation efflux_AS.fw [SEQ ID No. 40] and Cation efflux_AS.rv [SEQ ID No. 41]) and a second set of Gateway^{™} compatible primers (attB1 [SEQ ID No. 42] and attB2 [SEQ ID No. 43]). The amplification product was inserted into the Gateway^{™} pDONR^{™}/Zeo vector (ThermoFisher Scientific). The sequence is then transferred to the GDB expression vector (SEQ ID No. 38).

### GDB-Cation efflux_Δ117C expression vector

The Δ117C variant (SEQ ID No. 32) is generated by two-step PCR amplification and Gateway^{™} cloning, using the GDB-Cation efflux expression vector as template, a first set of gene-specific primers (Cation efflux_Δ117C.fw [SEQ ID No. 45] and Cation efflux_Δ117C.rv [SEQ ID No. 46]) and a second set of Gateway^{™} compatible primers (attB1 [SEQ ID No. 42] and attB2 [SEQ ID No. 43]). The amplification product was inserted into the Gateway^{™} pDONR^{™}/Zeo vector (ThermoFisher Scientific). The sequence is then transferred to the GDB expression vector (SEQ ID No. 38).

### GDB-Cation efflux_Δ117N expression vector

The Δ117N variant (SEQ ID No. 34) was generated by two-step PCR amplification and Gateway^{™} cloning, using the GDB-Cation efflux expression vector as template, a first set of gene-specific primers (Cation efflux_Δ117N.fw [SEQ ID No. 47] and Cation efflux_Δ117N.rv [SEQ ID No. 48) and a second set of Gateway^{™} compatible primers (attB1 [SEQ ID No. 42] and attB2 [SEQ ID No. 43]). The amplification product was inserted into the Gateway^{™} pDONR^{™}/Zeo vector (ThermoFisher Scientific). The sequence was then transferred to the GDB expression vector (SEQ ID No. 38).

The resulting plasmids were sequenced and transformed into *Agrobacterium tumefaciens* GV3101 pMP90 by heat shock and transiently expression in TN90 leaves.

### Transient gene expression

*Agrobacterium tumefaciens* GV3101 strains carrying the construct of interest were grown overnight in Luria-Bertani (LB) medium supplemented with appropriate antibiotics. Cultures are spun down and re-suspended in buffer containing 10 mM MgCl2, 10 mM MES pH 5.6 and 100 µM acetosyringone to OD600=0.6 and incubated for one hour at room temperature. Infiltration is performed with a needleless syringe into TN90 leaves. Samples are taken 5 days post-infiltration.

Tests were performed in two biological replicates comprising twelve technical replicates each.

### Statistical Analysis

Statistical significances based on one-way ANOVA analyses is performed with Prism 5.01 software (GraphPad Software).

### Results

Nornicotine content of 5-week-old TN90 leaves expressing the Indicated constructs is shown in Figure 3. The nornicotine content is represented relative to a luciferase control (SEQ ID No. 44) and results comprise three biological replicates analysed by t-test. Values are shown as means ± SEM. Asterisks indicate statistical significance of P value ≤ 0.05.

### Conclusions

Deletion of the N-terminal domain results in a decrease in nornicotine content.

A proposed mechanism for cation efflux_Δ117C and cation efflux_Δ117N expression is shown in Figure 4.

### Example 3

In order to determine the ligand transported by SEQ ID No.3, 3D modelling of the protein structure was performed using Phyre2 (Kelley LA et al. Nature Protocols 10, 845-858 (2015)) and Swiss-Model. Phyre2 uses the alignment of Hidden Markov Models (HMM) via HHsearch (Söding, J. Bioinformatics 21, 951-960 (2005)) to significantly determine accuracy of alignment and detection rate. Material and methods used by Swiss-Model are described on swissmodel.expasy.org

### Results

Protein modelling using Phyre² indicated Zinc transport with 100% confidence. Protein modelling using evolutionary related structures matching SED ID No. 3 indicates two Zn ion binding sites upon formation of homodimers (Swiss-Model, see for example, Figure 4c).

### Conclusions

These results indicate that SEQ ID No.3 is likely to be involved in zinc efflux.

### Example 4

To assess the correlation between the substrate of the identified metal transporter and nornicotine content in planta, transgenic tobacco plants overexpressing the wild type cation efflux (encoded by SEQ ID No. 2), cation efflux Δ117C variant (SEQ ID No. 32), and cation efflux Δ117N variant (SEQ ID No. 34) were treated with various concentrations of the substrate metal: Zn (3mM, 5mM, 10mM), as well as Cd (70 µM, 100 µM, 150 µM), or Ni (50 µM, 300 µM, 1mM) and nornicotine levels were measured as described in Example 2.

### Results

The nornicotine content of tobacco plants treated with the indicated concentrations of zinc, cadmium or nickel is shown in Figure 68.

Zinc treatment induces an increase in nornicotine levels. This effect is reverted upon expression of a constitutive high affinity Δ117N zinc transporter as shown in Figures 69 a-c.

### Conclusions

Zinc levels correlate with nornicotine levels, indicating that zinc acts as a regulator in nicotine conversion. This is further evidenced by the insensitivity to zinc and the reduced symptoms to zinc treatment when expressing a constitutive high affinity Δ117N zinc transporter, which detoxifies cytoplasmic zinc ions via efflux.

### Example 5

Since zinc acts as catalytic or structural cofactor for many proteins, depletion of intracellular zinc content, resulting in zinc deficiency, induces retarded growth and apoptosis in severe cases (Siklar et al., 2003 J Trop Pediatr. 49(3):187-8; Eide 2009 Journal of Biological Chemistry 10; 284(28): 18565-18569). Zinc starvation is reported to strongly impair yeast growth and induces autophagy (Kawamata et al. 2017 J Biol Chem. 2017 May 19;292(20):8520-8530).

To confirm that Δ117N acts as a constitutive high affinity zinc transporter, the cation efflux protein, the Δ117C and the Δ117N variants according to the present invention were engineered into yeast and the colony growth was monitored.

### Yeast

For yeast expression, BY4742 strain is transformed by means of a chemical method.

### Cloning

### Cation efflux yeast expression vector

The gene sequence (SEQ ID No. 2) was amplified from a Gateway^{™} compatible cDNA library using In-Fusion PCR primers (SEQ ID No. 50 and SEQ ID No. 51), which allow In-Fusion cloning into a pYES2 vector (pYES3/UT.1 [SEQ ID No. 52]) (Thermo Fisher Scientific). Prior to In-Fusion cloning the vector is digested with Hindlll and BamHl restriction enzymes (Promega).

### Cation efflux_Δ117C yeast expression vector

The Cation efflux_Δ117C yeast expression vector was generated by In-Fusion cloning using the GDB-Cation efflux expression vector as template and In-Fusion PCR primers (SEQ ID No. 53 and SEQ ID No. 54), which allow In-Fusion cloning into a pYES2 vector (SEQ ID No. 55). Prior to In-Fusion cloning the vector is digested with Hindlll and BamHl restriction enzymes (Promega).

### Cation efflux_Δ117N yeast expression vector

The Cation efflux_Δ117N yeast expression vector was generated by In-Fusion cloning using the GDB-Cation efflux expression vector as template and In-Fusion PCR primers (SEQ ID No. 56 SEQ ID No. 57), which allow In-Fusion cloning into a pYES2 vector (SEQ ID No. 55). Prior to In-Fusion cloning the vector is digested with Hindlll and BamHl restriction enzymes (Promega).

Activity of a cation efflux protein is measured using a yeast metal tolerance assay.

### Yeast Metal Tolerance Assays

Yeast wild type strains were transformed with the constructs of interest. Yeast transformation was performed using S.c. EasyComp^{™} Transformation Kit (Thermo Fisher).

Positive colonies were selected on synthetic dropout (SD) plates containing the appropriate selective markers. Yeast strains expressing empty vector, wild-type cation transporter (Seq ID No. 2), or mutated variants (Δ117C and Δ117N) were pre-cultured in SD liquid medium containing the appropriate selective markers at 28 °C for 16 h. The optical density was normalized to 0.5, and the strains were grown for 4 hours with selective media + galactose 2% to induce gene expression 5 µL of three 10-fold dilutions of the culture (of optical density at 600 nm of 0.005 0.0005, and 0.00005) are spotted onto SD-agar plates containing the appropriate selective markers, galactose, and ZnCl2 (3 mM and 5 mM)

### Results

Growth of wild type yeast expressing the indicated constructs is shown in Figure 70.

### Conclusions

Expression of the Δ117N cation efflux variant in yeast leads to reduced growth. The Δ117N cation efflux variant is therefore identified as a deregulated, constitutive high affinity transporter.

Various modifications and variations of the described methods and system of the present invention will be apparent to those skilled in the art.

Although the present invention has been described in connection with specific preferred embodiments, it should be understood that the invention as claimed should not be unduly limited to such specific embodiments.

## Claims

1. A method of reducing the content of at least one tobacco specific nitrosamine (TSNA) or a precursor of a TSNA in tobacco comprising expressing a deregulated cation efflux protein in a tobacco plant or plant part thereof or plant cell, wherein the deregulated cation efflux protein is a constitutively high affinity cation efflux transporter.

2. A method according to claim 1, wherein the deregulated cation efflux protein exhibits increased metal ion transport compared to a wild type cation efflux transporter.

3. A method according to any preceding claim,
wherein the deregulated cation efflux protein lacks a functional regulatory domain, preferably the deregulated cation efflux protein lacks at least part of a regulatory domain; and/or
wherein the deregulated cation efflux protein lacks a functional cytoplasmic domain, preferably the deregulated cation efflux protein lacks at least part of the cytoplasmic domain.

4. A method according to any preceding claim,
wherein the deregulated cation efflux transporter comprises one or more mutations compared to the amino acid sequence shown as SEQ ID No. 3 and is deregulated when compared to a wild-type cation efflux protein comprising the amino acid sequence shown as SEQ ID No. 3; or
wherein the deregulated cation efflux transporter comprises one or more mutations compared to the amino acid sequence shown as SEQ ID No. 6 and is deregulated when compared to a wild-type cation efflux protein comprising the amino acid sequence shown as SEQ ID No. 6; or
wherein the deregulated cation efflux transporter comprises one or more mutations compared to the amino acid sequence shown as SEQ ID No. 9 and is deregulated when compared to a wild-type cation efflux protein comprising the amino acid sequence shown as SEQ ID No. 9; or
wherein the deregulated cation efflux transporter comprises one or more mutations compared to the amino acid sequence shown as SEQ ID No. 12 and is deregulated when compared to a wild-type cation efflux protein comprising the amino acid sequence shown as SEQ ID No. 12; or
wherein the deregulated cation efflux transporter comprises one or more mutations compared to the amino acid sequence shown as SEQ ID No. 15 and is deregulated when compared to a wild-type cation efflux protein comprising the amino acid sequence shown as SEQ ID No. 15; or
wherein the deregulated cation efflux transporter comprises one or more mutations compared to the amino acid sequence shown as SEQ ID No. 18 and is deregulated when compared to a wild-type cation efflux protein comprising the amino acid sequence shown as SEQ ID No. 18; or
wherein the deregulated cation efflux transporter comprises one or more mutations compared to the amino acid sequence shown as SEQ ID No. 21 and is deregulated when compared to a wild-type cation efflux protein comprising the amino acid sequence shown as SEQ ID No. 21; or
wherein the deregulated cation efflux transporter comprises one or more mutations compared to the amino acid sequence shown as SEQ ID No. 24 and is deregulated when compared to a wild-type cation efflux protein comprising the amino acid sequence shown as SEQ ID No. 24; or
wherein the deregulated cation efflux transporter comprises one or more mutations compared to the amino acid sequence shown as SEQ ID No. 27 and is deregulated when compared to a wild-type cation efflux protein comprising the amino acid sequence shown as SEQ ID No. 27; or
wherein the deregulated cation efflux transporter comprises one or more mutations compared to the amino acid sequence shown as SEQ ID No. 30 and is deregulated when compared to a wild-type cation efflux protein comprising the amino acid sequence shown as SEQ ID No. 30.

5. A method according to any preceding claim wherein the deregulated cation efflux transport protein comprises one or more mutations compared to the amino acid sequence shown as SEQ ID No. 3, 6, 9, 12, 15, 18, 21, 24, 27 or 30 and is a sequence which has at least 70% identity thereto.

6. A method according to any preceding claim which method comprises introducing into the genome of said plant or plant cell a mutation within a polynucleotide encoding a cation efflux protein such that the polynucleotide encodes a deregulated cation efflux protein.

7. A method according to claim 5 or claim 6, wherein the mutation produces a deletion, a splice mutant or codon encoding a non-tolerated amino acid substitution in the polynucleotide encoding said protein.

8. A method according to any preceding claim, wherein the deregulated cation efflux protein comprises an amino acid sequence which lacks at least part of the N terminus when compared with an amino acid sequence shown as SEQ ID No. 3, 6, 9, 12, 15, 18, 21, 24, 27 or 30 or a sequence which has at least 70% identity thereto.

9. A method according to any preceding claim, which method comprises introducing into the genome of said plant or plant cell an exogenous polynucleotide sequence which encodes a deregulated cation efflux protein.

10. A method according to any preceding claim, wherein the deregulated cation efflux protein is:
a) a truncated cation efflux protein which lacks at least amino acids corresponding to 2-25, or 2-50, or 2-75, or 2-100, 2-114, 2-117, 2-120, 2-125, 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, 2-114 or 2-117 of SEQ ID No. 3, 6, 9, 12, 15, 18, 21, 24, 27 or 30;
b) a truncated cation efflux protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30.

11. A method according to any one of the preceding claims wherein the TSNA is N'-nitrosonornicotine (NNN) and/or the precursor is nornicotine.

12. A method according to any preceding claim
wherein the deregulated cation efflux protein comprises the amino acid sequence shown as SEQ ID No. 33, or a sequence which has at least 70%, at least 75%, at least 80%, at least 85%, at least 90%, at least 95%, at least 97%, or at least 99% sequence identity thereto; or
wherein the deregulated cation efflux protein is encoded by a polynucleotide comprising the sequence shown as SEQ ID No. 34, or a sequence which has at least 70%, at least 75%, at least 80%, at least 85%, at least 90%, at least 95%, at least 97%, or at least 99% sequence identity thereto.

13. A tobacco plant or part thereof:
a. which has been modified to achieve a reduction in a TSNA or a precursor of a TSNA compared with an unmodified plant, wherein the modified plant or part thereof comprises a deregulated cation efflux protein as defined in claim 1;
b. which has a mutation within a gene of the plant encoding a cation efflux protein which mutation deregulates said cation efflux protein in accordance with claim 1, wherein the gene prior to mutation comprises the sequence shown as SEQ ID No. 1, SEQ ID No. 2, SEQ ID No. 4, SEQ ID No. 5, SEQ ID No. 7, SEQ ID No. 8, SEQ ID No. 10, SEQ ID No. 11, SEQ ID No. 13, SEQ ID No. 14, SEQ ID No. 16, SEQ ID No. 17, SEQ ID No. 19, SEQ ID No. 20, SEQ ID No. 22, SEQ ID No. 23, SEQ ID No. 25, SEQ ID No. 26, SEQ ID No. 28 or SEQ ID No. 29, or a sequence which has at least 70% sequence identity thereto;
c. obtained or obtainable by the method according to any one of claims 1-12,
d. comprising an exogenous gene which encodes a deregulated cation efflux protein as defined in claim 1 wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
e. comprising a deregulated cation efflux protein as defined in claim 1 which comprises the amino acid sequence set forth in SEQ ID No. 33 or a sequence with at least 70% identity thereto, or a polynucleotide sequence set forth in SEQ ID No. 34 or a sequence with at least 70% identity thereto; or
f. comprising a construct or vector comprising a polynucleotide encoding a deregulated cation efflux protein as defined in claim 1 wherein said polynucleotide comprises a nucleotide sequence selected from the group consisting of:
(i) a nucleotide sequence comprising SEQ ID No. 34;
(ii) a nucleotide sequence having at least 70% sequence identity to the entirety of the sequence set forth in SEQ ID No. 34, wherein said nucleotide sequence encodes a polypeptide involved in cation efflux in a plant;
(iii) a nucleotide sequence encoding a polypeptide comprising the amino acid sequence shown herein as SEQ ID No. 33, or a fragment thereof comprising at least 200, or at least 250, or at least 300, or at least 350, or at least 400 contiguous residues of SEQ ID No. 33;
(iv) a nucleotide sequence encoding a truncated polypeptide which lacks at least amino acids corresponding to 2-25, or 2-50, or 2-75, or 2-100, or 2-114, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
(v) a nucleotide sequence encoding a truncated polypeptide which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30;
(vi) a nucleotide sequence encoding a polypeptide comprising an amino acid sequence which corresponds to amino acids 125-324 of SEQ ID No. 3, 6, 9, 12, 15, 18, 21, 24, 27 or 30 and/or comprising an amino acid sequence which corresponds to amino acids 323-413 of SEQ ID No. 3, 6, 9, 12, 15, 18, 21, 24, 27 or 30 wherein said nucleotide sequence encodes a polypeptide involved in cation efflux in a plant; or
(vii) a nucleotide sequence according to any of preceding items (i) to (vi) which further comprises a mutation in said nucleotide sequence, e.g. where said mutation produces a deletion, a splice mutant or codon encoding a non-tolerated amino acid substitution in the polynucleotide encoding said protein;
or comprising a polynucleotide encoding a polypeptide that is a deregulated cation efflux protein as defined in claim 1 wherein said polypeptide comprises an amino acid sequence selected from the group consisting of:
(viii) an amino acid sequence comprising SEQ ID No. 33;
(ix) an amino acid sequence that is at least 70% identical to an amino acid sequence set forth in SEQ ID No. 33;
(x) an amino acid sequence comprising amino acids which correspond to amino acids 125-324 of SEQ ID No. 3, 6, 9, 12, 15, 18, 21, 24, 27 or 30 and/or comprising amino acids which correspond to amino acids 323-413 of SEQ ID No. 3, 6, 9, 12, 15, 18, 21, 24, 27 or 30 wherein said polypeptide is involved in cation efflux in a plant;
(xi) an amino acid sequence which is a fragment of SEQ ID No. 3, 6, 9, 12, 15, 18, 21, 24, 27 or 30 comprising at least 200, or at least 250, or at least 300, or at least 350, or at least 400 contiguous residues of SEQ ID No. 3, 6, 9, 12, 15, 18, 21, 24, 27 or 30;
(xii) an amino acid which is a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30;
(xiii) an amino acid which is a truncated amino acid sequence which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which consists essentially of or consists of at least 375, at least 350, or at least 300, or at least 250, or at least 200, or at least 150 amino acids from the C-terminal of SEQ ID No. 3, 6, 9, 12, 15, 18, 21, 24, 27 or 30; or
(xiv) an amino acid sequence having at least 70% sequence identity to the entirety of the sequence set forth in SEQ ID No. 33,
with the proviso that the tobacco plant is not exclusively obtained by an essentially biological process.

14. A plant propagation material (e.g. a plant seed) obtainable from a plant according to claim 13, with the proviso that the tobacco plant is not exclusively obtained by an essentially biological process, and the said propagation material expresses a deregulated cation efflux protein as defined in claim 1.

15. The method according to any one of claims 1-12, or the tobacco plant or tobacco plant part of claim 13, or the plant propagation material according to claim 14, wherein the plant is from the species *Nicotiana tabacum* or *Nicotiana rustica.*

16. Use of a tobacco plant or tobacco plant part thereof according to claim 13, or a deregulated cation efflux protein as defined in any one of claims 1 to 12, for the production of a tobacco industry product.

17. Use of a tobacco plant according to claim 13 or a plant propagation material according to claim 14 to grow a crop or to produce a processed (preferably cured) tobacco leaf.

18. A harvested leaf of a tobacco plant according to claim 13 or obtainable from a tobacco plant propagated from a propagation material according to claim 14 or obtainable from a tobacco plant obtainable by a method according to any one of claims 1 to 12 or obtainable by a use according to claim 16 or claim 17, optionally wherein the harvested leaf is a cut harvested leaf, with the proviso that the tobacco plant is not exclusively obtainable by an essentially biological process, and it expresses a deregulated cation efflux protein as defined in claim 1.

19. A processed tobacco leaf (preferably a non-viable processed tobacco leaf):
a. obtainable from processing a tobacco plant according to claim 13;
b. obtainable from a tobacco plant propagated from a plant propagation material according to claim 14; or
c. obtainable by processing a harvested leaf according to claim 18, optionally wherein the plant or leaf is processed by curing, fermentation, pasteurising or combinations thereof and/or optionally wherein the processed tobacco leaf is cut processed tobacco leaf, with the proviso that the tobacco plant is not exclusively obtained by an essentially biological process, and said plant comprises a deregulated cation efflux protein as defined in claim 1.

20. Cured tobacco material made from a plant or a part thereof according to claim 13 or is obtained (or obtainable) from the plant propagation material according to claim 14 or obtained (or obtainable) by the method according to any one of claims 1-12.

21. A tobacco blend comprising said cured tobacco material of claim 20.

22. A tobacco industry product:
a. prepared from a tobacco plant according to claim 13 or a part thereof;
b. prepared from a tobacco plant or a part thereof (preferably the leaves harvested from the plant) obtained or obtainable by the method according to any one of claims 1-12;
c. prepared from the plant (preferably the leaves) propagated from a plant propagation material according to claim 14;
d. prepared from a harvested leaf according to claim 18;
e. prepared from a processed leaf according to claim 19;
f. prepared from a cured tobacco material according to claim 20 ; or
g. prepared from a tobacco blend according to claim 21,
optionally wherein the tobacco industry product is a combustible smoking article or a smokeless tobacco industry product or a non-combustible aerosol provision system, such as a tobacco heating device (e.g. an aerosol-generating device).

23. A combustible smoking article, non-combustible aerosol provisioning system, smokeless tobacco industry product or tobacco heating device comprising a plant or a portion thereof from the species *Nicotiana tabacum* or *Nicotiana rustica* according to any one of claim 13, or obtainable (e.g. obtained) from a plant propagation product according to claim 14, or obtainable (e.g. obtained) from a method according to any one of claims 1 to 12.

24. A mutant of a plant carrying a heritable mutation in a nucleotide sequence of at least one gene encoding a deregulated cation efflux protein as defined in claim 1 comprising the amino acid sequence shown as SEQ ID No. 33, or sequence which has at least 70% identity thereto, or a nucleotide sequence which encodes a deregulated cation efflux protein as defined in claim 1 wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30; wherein said heritable mutation decreases the content of at least one TSNA or a precursor of a TSNA relative to a comparable plant which does not carry said heritable mutation, or
a progeny or seed of said mutant plant which carries the heritable mutation, with the proviso that the tobacco plant is not exclusively obtained by an essentially biological process.

25. A harvested leaf, a processed leaf or cured tobacco material produced from a plant comprising a mutation in a nucleotide sequence encoding a deregulated cation efflux protein as defined in claim 1 comprising the amino acid sequence shown as SEQ ID No. 33, or sequence which has at least 70% identity thereto, or a nucleotide sequence which encodes a deregulated cation efflux protein as defined in claim 1 wherein said protein comprises a truncated amino acid sequence which lacks at least amino acids corresponding to 2-25, or 2-50, or 2-75, or 2-100, or 2-114, or 2-117, or 2-117, or 2-120, or 2-125, or 2-128, or 2-130 from the N-terminal side of SEQ ID No. 3, 6, 9, 12, 15, 18, 21, 24, 27 or 30, or a protein which has at least 70% (preferably at least 75%, at least 80%, at least 85%, at least 90%, at least 95%, at least 97%, or at least 99%) sequence identity to a truncated protein which lacks at least amino acids corresponding to amino acids 2-25, or 2-50, or 2-75, or 2-100, or 2-114, or 2-117, or 2-117, or 2-120, or 2-125, or 2-128, or 2-130 of SEQ ID No. 3, 6, 9, 12, 15, 18, 21, 24, 27 or 30; wherein said harvested leaf, processed leaf or cured tobacco has decreased content of at least one TSNA or a precursor of a TSNA relative to a comparable plant which does not carry said modification, with the proviso that the tobacco plant is not exclusively obtained by an essentially biological process.

## Patentansprüche

1. Verfahren zum Reduzieren des Gehalts mindestens eines tabakspezifischen Nitrosamins (TSNA) oder eines Vorläufers eines TSNA in Tabak, das Exprimieren eines deregulierten Kationenefflux-Proteins in einer Tabakpflanze oder einem Pflanzenteil davon oder einer Pflanzenzelle umfasst, wobei das deregulierte Kationenefflux-Protein ein Kationenefflux-Transporter mit konstitutiv hoher Affinität ist.

2. Verfahren nach Anspruch 1, wobei das deregulierte Kationenefflux-Protein im Vergleich zu einem Wildtyp-Kationenefflux-Transporter erhöhten Metallionentransport vorweist.

3. Verfahren nach einem vorstehenden Anspruch,
wobei dem deregulierten Kationenefflux-Protein eine funktionelle regulatorische Domäne fehlt, wobei dem deregulierten Kationenefflux-Protein vorzugsweise mindestens ein Teil einer regulatorischen Domäne fehlt; und/oder
wobei dem deregulierten Kationenefflux-Protein eine funktionelle cytoplasmatische Domäne fehlt, wobei dem deregulierten Kationenefflux-Protein vorzugsweise mindestens ein Teil der cytoplasmatischen Domäne fehlt.

4. Verfahren nach einem vorstehenden Anspruch,
wobei der deregulierte Kationenefflux-Transporter eine oder mehrere Mutationen im Vergleich zu der als SEQ ID Nr. 3 gezeigten Aminosäuresequenz umfasst und im Vergleich zu einem Wildtyp-Kationenefflux-Protein, das die als SEQ ID Nr. 3 gezeigte Aminosäuresequenz umfasst, dereguliert ist; oder
wobei der deregulierte Kationenefflux-Transporter eine oder mehrere Mutationen im Vergleich zu der als SEQ ID Nr. 6 gezeigten Aminosäuresequenz umfasst und im Vergleich zu einem Wildtyp-Kationenefflux-Protein, das die als SEQ ID Nr. 6 gezeigte Aminosäuresequenz umfasst, dereguliert ist; oder
wobei der deregulierte Kationenefflux-Transporter eine oder mehrere Mutationen im Vergleich zu der als SEQ ID Nr. 9 gezeigten Aminosäuresequenz umfasst und im Vergleich zu einem Wildtyp-Kationenefflux-Protein, das die als SEQ ID Nr. 9 gezeigte Aminosäuresequenz umfasst, dereguliert ist; oder
wobei der deregulierte Kationenefflux-Transporter eine oder mehrere Mutationen im Vergleich zu der als SEQ ID Nr. 12 gezeigten Aminosäuresequenz umfasst und im Vergleich zu einem Wildtyp-Kationenefflux-Protein, das die als SEQ ID Nr. 12 gezeigte Aminosäuresequenz umfasst, dereguliert ist; oder
wobei der deregulierte Kationenefflux-Transporter eine oder mehrere Mutationen im Vergleich zu der als SEQ ID Nr. 15 gezeigten Aminosäuresequenz umfasst und im Vergleich zu einem Wildtyp-Kationenefflux-Protein, das die als SEQ ID Nr. 15 gezeigte Aminosäuresequenz umfasst, dereguliert ist; oder
wobei der deregulierte Kationenefflux-Transporter eine oder mehrere Mutationen im Vergleich zu der als SEQ ID Nr. 18 gezeigten Aminosäuresequenz umfasst und im Vergleich zu einem Wildtyp-Kationenefflux-Protein, das die als SEQ ID Nr. 18 gezeigte Aminosäuresequenz umfasst, dereguliert ist; oder
wobei der deregulierte Kationenefflux-Transporter eine oder mehrere Mutationen im Vergleich zu der als SEQ ID Nr. 21 gezeigten Aminosäuresequenz umfasst und im Vergleich zu einem Wildtyp-Kationenefflux-Protein, das die als SEQ ID Nr. 21 gezeigte Aminosäuresequenz umfasst, dereguliert ist; oder
wobei der deregulierte Kationenefflux-Transporter eine oder mehrere Mutationen im Vergleich zu der als SEQ ID Nr. 24 gezeigten Aminosäuresequenz umfasst und im Vergleich zu einem Wildtyp-Kationenefflux-Protein, das die als SEQ ID Nr. 24 gezeigte Aminosäuresequenz umfasst, dereguliert ist; oder
wobei der deregulierte Kationenefflux-Transporter eine oder mehrere Mutationen im Vergleich zu der als SEQ ID Nr. 27 gezeigten Aminosäuresequenz umfasst und im Vergleich zu einem Wildtyp-Kationenefflux-Protein, das die als SEQ ID Nr. 27 gezeigte Aminosäuresequenz umfasst, dereguliert ist; oder
wobei der deregulierte Kationenefflux-Transporter eine oder mehrere Mutationen im Vergleich zu der als SEQ ID Nr. 30 gezeigten Aminosäuresequenz umfasst und im Vergleich zu einem Wildtyp-Kationenefflux-Protein, das die als SEQ ID Nr. 30 gezeigte Aminosäuresequenz umfasst, dereguliert ist.

5. Verfahren nach einem vorstehenden Anspruch, wobei das deregulierte Kationenefflux-Transportprotein eine oder mehrere Mutationen im Vergleich zu der als SEQ ID Nr. 3, 6, 9, 12, 15, 18, 21, 24, 27 oder 30 gezeigten Aminosäuresequenz umfasst und eine Sequenz ist, die mindestens 70 % Identität damit aufweist.

6. Verfahren nach einem vorstehenden Anspruch, wobei das Verfahren Einbringen einer Mutation innerhalb eines Polynukleotids, das ein Kationenefflux-Protein codiert, in das Genom der Pflanze oder Pflanzenzelle umfasst, sodass das Polynukleotid ein dereguliertes Kationenefflux-Protein codiert.

7. Verfahren nach Anspruch 5 oder Anspruch 6, wobei die Mutation eine Deletion, eine Spleißmutante oder ein Codon erzeugt, das eine nicht tolerierte Aminosäuresubstitution im Polynukleotid codiert, das das Protein codiert.

8. Verfahren nach einem vorstehenden Anspruch, wobei das deregulierte Kationenefflux-Protein eine Aminosäuresequenz umfasst, der im Vergleich zu einer als SEQ ID Nr. 3, 6, 9, 12, 15, 18, 21, 24, 27 oder 30 gezeigten Aminosäuresequenz oder einer Sequenz, die mindestens 70 % Identität damit aufweist, mindestens ein Teil des N-Terminus fehlt.

9. Verfahren nach einem vorstehenden Anspruch, wobei das Verfahren Einbringen einer exogenen Polynukleotidsequenz, die ein dereguliertes Kationenefflux-Protein codiert, in das Genom der Pflanze oder Pflanzenzelle umfasst.

10. Verfahren nach einem vorstehenden Anspruch, wobei das deregulierte Kationenefflux-Protein Folgendes ist:
a) ein verkürztes Kationenefflux-Protein, dem mindestens Aminosäuren fehlen, die 2-25, oder 2-50, oder 2-75, oder 2-100, 2-114, 2-117, 2-120, 2-125, 2-128 oder 2-130 von der Seite des N-Terminus von SEQ ID Nr. 3, 6, 9, 12, 15, 18, 21, 24, 27 oder 30 entsprechen, oder ein Protein, das mindestens 70 % (vorzugsweise mindestens 75 %, mindestens 80 %, mindestens 85 %, mindestens 90 %, mindestens 95 %, mindestens 97 % oder mindestens 99 %) Sequenzidentität mit einem verkürzten Protein aufweist, dem mindestens Aminosäuren fehlen, die Aminosäuren 2-25, oder 2-50, oder 2-75, oder 2-100, 2-114 oder 2-117 von SEQ ID Nr. 3, 6, 9, 12, 15, 18, 21, 24, 27 oder 30 entsprechen;
b) ein verkürztes Kationenefflux-Protein, das im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht, oder ein Protein, das mindestens 70 % (vorzugsweise mindestens 75%, mindestens 80 %, mindestens 85 %, mindestens 90 %, mindestens 95%, mindestens 97 % oder mindestens 99 %) Sequenzidentität mit einem verkürzten Protein aufweist, das im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht.

11. Verfahren nach einem der vorstehenden Ansprüche, wobei das TSNA ein N'-Nitrosonornicotin (NNN) ist und/oder der Vorläufer Nornicotin ist.

12. Verfahren nach einem vorstehenden Anspruch,
wobei das deregulierte Kationenefflux-Protein die als SEQ ID Nr. 33 gezeigte Aminosäuresequenz umfasst, oder eine Sequenz, die mindestens 70 %, mindestens 75 %, mindestens 80 %, mindestens 85 %, mindestens 90 %, mindestens 95 %, mindestens 97 % oder mindestens 99 % Sequenzidentität damit aufweist; oder
wobei das deregulierte Kationenefflux-Protein durch ein Polynukleotid codiert wird, das die als SEQ ID Nr. 34 gezeigte Sequenz umfasst, oder eine Sequenz, die mindestens 70 %, mindestens 75 %, mindestens 80 %, mindestens 85%, mindestens 90 %, mindestens 95 %, mindestens 97 %, oder mindestens 99 % Sequenzidentität damit aufweist.

13. Tabakpflanze oder ein Teil davon:
a. die modifiziert wurde, um eine Verringerung eines TSNA oder eines Vorläufers eines TSNA im Vergleich zu einer nicht modifizierten Pflanze zu erreichen, wobei die modifizierte Pflanze oder der Teil davon ein dereguliertes Kationenefflux-Protein wie in Anspruch 1 definiert umfasst;
b. die eine Mutation innerhalb eines Gens der Pflanze aufweist, das ein Kationenefflux-Protein codiert, wobei die Mutation das Kationenefflux-Protein nach Anspruch 1 dereguliert, wobei das Gen vor Mutation die als SEQ ID Nr. 1, SEQ ID Nr. 2, SEQ ID Nr. 4, SEQ ID Nr. 5, SEQ ID Nr. 7, SEQ ID Nr. 8, SEQ ID Nr. 10, SEQ ID Nr. 11, SEQ ID Nr. 13, SEQ ID Nr. 14, SEQ ID Nr. 16, SEQ ID Nr. 17, SEQ ID Nr. 19, SEQ ID Nr. 20, SEQ ID Nr. 22, SEQ ID Nr. 23, SEQ ID Nr. 25, SEQ ID Nr. 26, SEQ ID Nr. 28 oder SEQ ID Nr. 29 gezeigte Sequenz oder eine Sequenz, die mindestens 70 % Sequenzidentität damit aufweist, umfasst;
c. die durch das Verfahren nach einem der Ansprüche 1-12 erhalten wurde oder erhältlich ist,
d. die ein exogenes Gen umfasst, das ein dereguliertes Kationenefflux-Protein wie in Anspruch 1 definiert codiert, wobei das Protein eine verkürzte Aminosäuresequenz umfasst, der mindestens Aminosäuren fehlen, die 2-25, oder 2-50, oder 2-75, oder 2-100, oder 2-114, oder 2-117, oder 2-117, oder 2-120, oder 2-125, oder 2-128, oder 2-130 von der Seite des N-Terminus von SEQ ID Nr. 3, 6, 9, 12, 15, 18, 21, 24, 27 oder 30 entsprechen, oder ein Protein, das mindestens 70 % (vorzugsweise mindestens 75 %, mindestens 80 %, mindestens 85 %, mindestens 90 %, mindestens 95 %, mindestens 97 % oder mindestens 99 %) Sequenzidentität mit einem verkürzten Protein aufweist, dem mindestens Aminosäuren fehlen, die Aminosäuren 2-25, oder 2-50, oder 2-75, oder 2-100, oder 2-114, oder 2-117, oder 2-117, oder 2-120, oder 2-125, oder 2-128 oder 2-130 von SEQ ID Nr. 3, 6, 9, 12, 15, 18, 21, 24, 27 oder 30 entsprechen;
e. die ein dereguliertes Kationenefflux-Protein wie in Anspruch 1 definiert umfasst, das die in SEQ ID Nr. 33 aufgeführte Aminosäuresequenz oder eine Sequenz mit mindestens 70 % Identität damit, oder eine in SEQ ID Nr. 34 aufgeführte Polynukleotidsequenz oder eine Sequenz mit mindestens 70 % Identität damit umfasst; oder
f. die ein Konstrukt oder einen Vektor umfasst, der ein Polynukleotid umfasst, das ein dereguliertes Kationenefflux-Protein wie in Anspruch 1 definiert codiert, wobei das Polynukleotid eine Nukleotidsequenz umfasst, die aus der Gruppe ausgewählt ist, bestehend aus:
(i) einer Nukleotidsequenz, die SEQ ID Nr. 34 umfasst;
(ii) einer Nukleotidsequenz, die mindestens 70 % Sequenzidentität mit der Gesamtheit der in SEQ ID Nr. 34 aufgeführten Sequenz aufweist, wobei die Nukleotidsequenz ein Polypeptid codiert, das am Kationenefflux in einer Pflanze beteiligt ist;
(iii) einer Nukleotidsequenz, die ein Polypeptid codiert, das die hierin als SEQ ID Nr. 33 gezeigte Aminosäuresequenz umfasst, oder ein Fragment davon, das mindestens 200, oder mindestens 250, oder mindestens 300, oder mindestens 350 oder mindestens 400 zusammenhängende Reste von SEQ ID Nr. 33 umfasst;
(iv) einer Nukleotidsequenz, die ein verkürztes Polypeptid codiert, dem mindestens Aminosäuren fehlen, die 2-25, oder 2-50, oder 2-75, oder 2-100, oder 2-114, oder 2-117, oder 2-120, oder 2-125, oder 2-128 oder 2-130 von der Seite des N-Terminus der SEQ ID Nr. 3, 6, 9, 12, 15, 18, 21, 24, 27 oder 30 entsprechen, oder ein Protein, das mindestens 70 % (vorzugsweise mindestens 75%, mindestens 80 %, mindestens 85 %, mindestens 90 %, mindestens 95%, mindestens 97 % oder mindestens 99 %) Sequenzidentität mit einem verkürzten Protein aufweist, dem mindestens Aminosäuren fehlen, die Aminosäuren 2-25, oder 2-50, oder 2-75, oder 2-100, oder 2-114, oder 2-117, oder 2-117, oder 2-120, oder 2-125, oder 2-128 oder 2-130 von SEQ ID Nr. 3, 6, 9, 12, 15, 18, 21, 24, 27 oder 30 entsprechen;
(v) einer Nukleotidsequenz, die ein verkürztes Polypeptid codiert, das im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht, oder ein Protein, das mindestens 70 % (vorzugsweise mindestens 75 %, mindestens 80 %, mindestens 85 %, mindestens 90 %, mindestens 95 %, mindestens 97 % oder mindestens 99 %) Sequenzidentität mit einem verkürzten Protein aufweist, das im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht.
(vi) einer Nukleotidsequenz, die ein Polypeptid codiert, das eine Aminosäuresequenz umfasst, die Aminosäuren 125-324 von SEQ ID Nr. 3, 6, 9, 12, 15, 18, 21, 24, 27 oder 30 entspricht und/oder eine Aminosäuresequenz umfasst, die Aminosäuren 323-413 von SEQ ID Nr. 3, 6, 9, 12, 15, 18, 21, 24, 27 oder 30 entspricht, wobei die Nukleotidsequenz ein Polypeptid codiert, das am Kationenefflux in einer Pflanze beteiligt ist; oder
(vii) einer Nukleotidsequenz nach einem der vorstehenden Punkte (i) bis (vi), die weiter eine Mutation in der Nukleotidsequenz umfasst, wo z. B. die Mutation eine Deletion, eine Spleißmutante oder ein Codon erzeugt, das eine nicht tolerierte Aminosäuresubstitution in dem Polynukleotid codiert, das das Protein codiert;
oder ein Polynukleotid umfasst, das ein Polypeptid codiert, das ein dereguliertes Kationenefflux-Protein wie in Anspruch 1 definiert ist, wobei das Polypeptid eine Aminosäuresequenz umfasst, die aus der Gruppe ausgewählt ist, bestehend aus:
(viii) einer Aminosäuresequenz, die SEQ ID Nr. 33 umfasst;
(ix) einer Aminosäuresequenz, die zu mindestens 70 % mit einer in SEQ ID Nr. 33 aufgeführten Aminosäuresequenz identisch ist;
(x) einer Aminosäuresequenz, die Aminosäuren umfasst, die Aminosäuren 125-324 von SEQ ID Nr. 3, 6, 9, 12, 15, 18, 21, 24, 27 oder 30 entsprechen und/oder Aminosäuren umfasst, die Aminosäuren 323-413 von SEQ ID Nr. 3, 6, 9, 12, 15, 18, 21, 24, 27 oder 30 entsprechen, wobei das Polypeptid am Kationenefflux in einer Pflanze beteiligt ist;
(xi) einer Aminosäuresequenz, die ein Fragment von SEQ ID Nr. 3, 6, 9, 12, 15, 18, 21, 24, 27 oder 30 ist und mindestens 200, oder mindestens 250, oder mindestens 300, oder mindestens 350 oder mindestens 400 zusammenhängende Reste von SEQ ID Nr. 3, 6, 9, 12, 15, 18, 21, 24, 27 oder 30 umfasst;
(xii) einer Aminosäure, die eine verkürzte Aminosäuresequenz ist, der mindestens Aminosäuren fehlen, die 2-25, oder 2-50, oder 2-75, oder 2-100, oder 2-114, oder 2-117, oder 2-117, oder 2-120, oder 2-125, oder 2-128 oder 2-130 von der Seite des N-Terminus von SEQ ID Nr. 3, 6, 9, 12, 15, 18, 21, 24, 27 oder 30 entsprechen, oder einem Protein, das mindestens 70 % (vorzugsweise mindestens 75%, mindestens 80 %, mindestens 85 %, mindestens 90 %, mindestens 95 %, mindestens 97 % oder mindestens 99 %) Sequenzidentität mit einem verkürzten Protein aufweist, dem mindestens Aminosäuren fehlen, die Aminosäuren 2-25, oder 2-50, oder 2-75, oder 2-100, oder 2-114, oder 2-117, oder 2-117, oder 2-120, oder 2-125, oder 2-128 oder 2-130 von SEQ ID Nr. 3, 6, 9, 12, 15, 18, 21, 24, 27 oder 30 entsprechen;
(xiii) einer Aminosäure, die eine verkürzte Aminosäuresequenz ist, die im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht, oder einem Protein, das mindestens 70 % (vorzugsweise mindestens 75 %, mindestens 80 %, mindestens 85 %, mindestens 90 %, mindestens 95%, mindestens 97 % oder mindestens 99 %) Sequenzidentität mit einem verkürzten Protein aufweist, das im Wesentlichen aus mindestens 375, mindestens 350, oder mindestens 300, oder mindestens 250, oder mindestens 200 oder mindestens 150 Aminosäuren von dem C-Terminus von SEQ ID Nr. 3, 6, 9, 12, 15, 18, 21, 24, 27 oder 30 besteht oder daraus besteht; oder
(xiv) einer Aminosäuresequenz, die mindestens 70 % Sequenzidentität mit der Gesamtheit der in SEQ ID Nr. 33 aufgeführten Sequenz aufweist,
unter der Voraussetzung, dass die Tabakpflanze nicht ausschließlich durch einen im Wesentlichen biologischen Prozess erhalten wird.

14. Pflanzliches Vermehrungsmaterial (z. B. ein Pflanzensamen), das aus einer Pflanze nach Anspruch 13 erhältlich ist, unter der Voraussetzung, dass die Tabakpflanze nicht ausschließlich durch einen im Wesentlichen biologischen Prozess erhalten wird, und dass das Vermehrungsmaterial ein dereguliertes Kationenefflux-Protein wie in Anspruch 1 definiert exprimiert.

15. Verfahren nach einem der Ansprüche 1-12 oder die Tabakpflanze oder der Tabakpflanzenteil nach Anspruch 13 oder das pflanzliche Vermehrungsmaterial nach Anspruch 14, wobei die Pflanze von der Art *Nicotiana tabacum* oder *Nicotiana rustica* ist.

16. Verwendung einer Tabakpflanze oder eines Tabakpflanzenteils davon nach Anspruch 13, oder eines deregulierten Kationenefflux-Proteins wie in einem der Ansprüche 1 bis 12 definiert, zur Herstellung eines Produkts der Tabakindustrie.

17. Verwendung einer Tabakpflanze nach Anspruch 13 oder eines Pflanzenvermehrungsmaterials nach Anspruch 14 zum Anbau einer Nutzpflanze oder zur Herstellung eines verarbeiteten (vorzugsweise getrockneten) Tabakblattes.

18. Geerntetes Blatt einer Tabakpflanze nach Anspruch 13 oder erhältlich von einer Tabakpflanze, die aus einem Vermehrungsmaterial nach Anspruch 14 vermehrt wurde oder von einer Tabakpflanze erhältlich ist, die durch ein Verfahren nach einem der Ansprüche 1 bis 12 erhältlich ist oder durch eine Verwendung nach Anspruch 16 oder Anspruch 17 erhältlich ist, wobei das geerntete Blatt gegebenenfalls ein geschnittenes geerntetes Blatt ist, mit der Voraussetzung, dass die Tabakpflanze nicht ausschließlich durch einen im Wesentlichen biologischen Prozess erhältlich ist, und sie ein dereguliertes Kationenefflux-Protein wie in Anspruch 1 definiert exprimiert.

19. Verarbeitetes Tabakblatt (vorzugsweise ein nicht lebensfähiges verarbeitetes Tabakblatt):
a. das durch Verarbeiten einer Tabakpflanze nach Anspruch 13 erhältlich ist;
b. das aus einerTabakpflanze, die aus einem pflanzlichen Vermehrungsmaterial nach Anspruch 14 vermehrt wurde, erhältlich ist; oder
c. das durch Verarbeiten eines geernteten Blattes nach Anspruch 18 erhältlich ist, wobei die Pflanze oder das Blatt gegebenenfalls durch Trocknung, Fermentation, Pasteurisieren oder Kombinationen davon verarbeitet wird und/oder wobei es sich bei dem verarbeiteten Tabakblatt gegebenenfalls um ein geschnittenes verarbeitetes Tabakblatt handelt, mit der Voraussetzung, dass die Tabakpflanze nicht ausschließlich durch einen im Wesentlichen biologischen Prozess erhalten wird, und dass die Pflanze ein dereguliertes Kationenefflux-Protein wie in Anspruch 1 definiert umfasst.

20. Getrocknetes Tabakmaterial, das aus einer Pflanze oder einem Teil davon nach Anspruch 13 hergestellt ist oder aus dem pflanzlichen Vermehrungsmaterial nach Anspruch 14 erhalten wird (oder erhältlich ist) oder durch das Verfahren nach einem der Ansprüche 1-12 erhalten wird (oder erhältlich ist).

21. Tabakmischung, die das getrocknete Tabakmaterial nach Anspruch 20 umfasst.

22. Produkt der Tabakindustrie:
a. hergestellt aus einer Tabakpflanze nach Anspruch 13 oder einem Teil davon;
b. hergestellt aus einer Tabakpflanze oder einem Teil davon (vorzugsweise den von der Pflanze geernteten Blättern), die durch das Verfahren nach einem der Ansprüche 1-12 erhalten wird oder erhältlich ist;
c. hergestellt aus der Pflanze (vorzugsweise den Blättern), die aus einem pflanzlichen Vermehrungsmaterial nach Anspruch 14 vermehrt wurde;
d. hergestellt aus einem geernteten Blatt nach Anspruch 18;
e. hergestellt aus einem verarbeiteten Blatt nach Anspruch 19;
f. hergestellt aus einem getrockneten Tabakmaterial nach Anspruch 20; oder
g. hergestellt aus einer Tabakmischung nach Anspruch 21, wobei das Produkt der Tabakindustrie gegebenenfalls ein brennbarer Rauchartikel oder ein rauchfreies Produkt der Tabakindustrie oder ein nicht brennbares Aerosolbereitstellungssystem, wie beispielsweise eine Tabakerhitzungsvorrichtung (z. B. eine Aerosol-erzeugende Vorrichtung), ist.

23. Brennbarer Rauchartikel, nicht brennbares Aerosolbereitstellungssystem, rauchfreies Produkt der Tabakindustrie oder Tabakerhitzungsvorrichtung, der/das eine Pflanze oder einen Teil davon der Art *Nicotiana tabacum* oder *Nicotiana rustica* nach Anspruch 13 umfasst oder aus einem Pflanzenvermehrungsprodukt nach Anspruch 14 erhältlich ist (z. B. erhalten wird) oder durch ein Verfahren nach einem der Ansprüche 1 bis 12 erhältlich ist (z. B. erhalten wird).

24. Mutant einer Pflanze, die eine vererbbare Mutation in einer Nukleotidsequenz von mindestens einem Gen trägt, die ein dereguliertes Kationenefflux-Protein wie in Anspruch 1 definiert codiert, das die Aminosäuresequenz umfasst, die als SEQ ID Nr. 33 gezeigt ist, oder eine Sequenz, die mindestens 70 % Identität damit aufweist, oder eine Nukleotidsequenz, die ein dereguliertes Kationenefflux-Protein wie in Anspruch 1 definiert codiert, wobei das Protein eine verkürzte Aminosäuresequenz umfasst, der mindestens Aminosäuren fehlen, die 2-25, oder 2-50, oder 2-75, oder 2-100, oder 2-114, oder 2-117, oder 2-117, oder 2-120, oder 2-125, oder 2-128, oder 2-130 von der Seite des N-Terminus von SEQ ID Nr. 3, 6, 9, 12, 15, 18, 21, 24, 27 oder 30 entsprechen, oder ein Protein, das mindestens 70 % (vorzugsweise mindestens 75%, mindestens 80 %, mindestens 85 %, mindestens 90 %, mindestens 95 %, mindestens 97 % oder mindestens 99 %) Sequenzidentität mit einem verkürzten Protein, dem mindestens Aminosäuren fehlen, die Aminosäuren 2-25, oder 2-50, oder 2-75, oder 2-100, oder 2-114, oder 2-117, oder 2-117, oder 2-120, oder 2-125, oder 2-128 oder 2-130 von SEQ ID Nr. 3, 6, 9, 12, 15, 18, 21, 24, 27 oder 30 entsprechen; wobei die vererbbare Mutation den Gehalt mindestens eines TSNA oder eines Vorläufers eines TSNA im Vergleich zu einer vergleichbaren Pflanze verringert, die die vererbbare Mutation nicht trägt, oder
eine Nachkommenschaft oder ein Samen der mutierten Pflanze, die die vererbbare Mutation trägt, unter der Voraussetzung, dass die Tabakpflanze nicht ausschließlich durch einen im Wesentlichen biologischen Prozess erhalten wird.

25. Geerntetes Blatt, verarbeitetes Blatt oder getrocknetes Tabakmaterial, das aus einer Pflanze hergestellt wurde, die eine Mutation in einer Nukleotidsequenz umfasst, die ein dereguliertes Kationenefflux-Protein wie in Anspruch 1 definiert codiert, das die Aminosäuresequenz umfasst, die als SEQ ID Nr. 33 gezeigt ist, oder eine Sequenz, die mindestens 70 % Identität damit aufweist, oder eine Nukleotidsequenz, die ein dereguliertes Kationenefflux-Protein wie in Anspruch 1 definiert codiert, wobei das Protein eine verkürzte Aminosäuresequenz umfasst, der mindestens Aminosäuren fehlen, die 2-25, oder 2-50, oder 2-75, oder 2-100, oder 2-114, oder 2-117, oder 2-117, oder 2-120, oder 2-125, oder 2-128, oder 2-130 von der Seite des N-Terminus von SEQ ID Nr. 3, 6, 9, 12, 15, 18, 21, 24, 27 oder 30 entsprechen, oder ein Protein, das mindestens 70 % (vorzugsweise mindestens 75 %, mindestens 80 %, mindestens 85 %, mindestens 90 %, mindestens 95 %, mindestens 97 % oder mindestens 99 %) Sequenzidentität mit einem verkürzten Protein, dem mindestens Aminosäuren fehlen, die Aminosäuren 2-25, oder 2-50, oder 2-75, oder 2-100, oder 2-114, oder 2-117, oder 2-117, oder 2-120, oder 2-125, oder 2-128 oder 2-130 von SEQ ID Nr. 3, 6, 9, 12, 15, 18, 21, 24, 27 oder 30 entsprechen; wobei das geerntete Blatt, verarbeitete Blatt oder der getrocknete Tabak im Vergleich zu einer vergleichbaren Pflanze, die die Modifikation nicht trägt, einen verringerten Gehalt mindestens eines TSNA oder eines Vorläufers eines TSNA aufweist, unter der Voraussetzung, dass die Tabakpflanze nicht ausschließlich durch einen im Wesentlichen biologischen Prozess erhalten wird.

## Revendications

1. Procédé de réduction de la teneur d'au moins une nitrosamine spécifique du tabac (TSNA) ou d'un précurseur d'une TSNA dans le tabac comprenant l'expression d'une protéine d'efflux cationique dérégulée dans une plante de tabac ou une partie végétale de celle-ci ou une cellule végétale, dans lequel la protéine d'efflux cationique dérégulée est un transporteur d'efflux cationique de haute affinité constitutive.

2. Procédé selon la revendication 1, dans lequel la protéine d'efflux cationique dérégulée présente un transport d'ions métalliques accru par rapport à un transporteur d'efflux cationique de type sauvage.

3. Procédé selon une quelconque revendication précédente,
dans lequel la protéine d'efflux cationique dérégulée est dépourvue d'un domaine de régulation fonctionnel, de préférence la protéine d'efflux cationique dérégulée est dépourvue d'au moins une partie d'un domaine de régulation ; et/ou
dans lequel la protéine d'efflux cationique dérégulée est dépourvue d'un domaine cytoplasmique fonctionnel, de préférence la protéine d'efflux cationique dérégulée est dépourvue d'au moins une partie du domaine cytoplasmique.

4. Procédé selon une quelconque revendication précédente,
dans lequel le transporteur d'efflux cationique dérégulé comprend une ou plusieurs mutations par rapport à la séquence d'acides aminés représentée par SEQ ID NO 3 et est dérégulé par rapport à une protéine d'efflux cationique de type sauvage comprenant la séquence d'acides aminés représentée par SEQ ID NO 3 ; ou
dans lequel le transporteur d'efflux cationique dérégulé comprend une ou plusieurs mutations par rapport à la séquence d'acides aminés représentée par SEQ ID NO 6 et est dérégulé par rapport à une protéine d'efflux cationique de type sauvage comprenant la séquence d'acides aminés représentée par SEQ ID NO 6 ; ou
dans lequel le transporteur d'efflux cationique dérégulé comprend une ou plusieurs mutations par rapport à la séquence d'acides aminés représentée par SEQ ID NO 9 et est dérégulé par rapport à une protéine d'efflux cationique de type sauvage comprenant la séquence d'acides aminés représentée par SEQ ID NO 9 ; ou
dans lequel le transporteur d'efflux cationique dérégulé comprend une ou plusieurs mutations par rapport à la séquence d'acides aminés représentée par SEQ ID NO 12 et est dérégulé par rapport à une protéine d'efflux cationique de type sauvage comprenant la séquence d'acides aminés représentée par SEQ ID NO 12 ; ou
dans lequel le transporteur d'efflux cationique dérégulé comprend une ou plusieurs mutations par rapport à la séquence d'acides aminés représentée par SEQ ID NO 15 et est dérégulé par rapport à une protéine d'efflux cationique de type sauvage comprenant la séquence d'acides aminés représentée par SEQ ID NO 15 ; ou
dans lequel le transporteur d'efflux cationique dérégulé comprenant une ou plusieurs mutations par rapport à la séquence d'acides aminés représentée par SEQ ID NO 18 et est dérégulé par rapport à une protéine d'efflux cationique de type sauvage comprenant la séquence d'acides aminés représentée par SEQ ID NO 18 ; ou
dans lequel le transporteur d'efflux cationique dérégulé comprend une ou plusieurs mutations par rapport à la séquence d'acides aminés représentée par SEQ ID NO 21 et est dérégulé par rapport à une protéine d'efflux cationique de type sauvage comprenant la séquence d'acides aminés représentée par SEQ ID NO 21 ; ou
dans lequel le transporteur d'efflux cationique dérégulé comprend une ou plusieurs mutations par rapport à la séquence d'acides aminés représentée par SEQ ID NO 24 et est dérégulé par rapport à une protéine d'efflux cationique de type sauvage comprenant la séquence d'acides aminés représentée par SEQ ID NO 24 ; ou
dans lequel le transporteur d'efflux cationique dérégulé comprend une ou plusieurs mutations par rapport à la séquence d'acides aminés représentée par SEQ ID NO 27 et est dérégulé par rapport à une protéine d'efflux cationique de type sauvage comprenant la séquence d'acides aminés représentée par SEQ ID NO 27 ; ou
dans lequel le transporteur d'efflux cationique dérégulé comprend une ou plusieurs mutations par rapport à la séquence d'acides aminés représentée par SEQ ID NO 30 et est dérégulé par rapport à une protéine d'efflux cationique de type sauvage comprenant la séquence d'acides aminés représentée par SEQ ID NO 30.

5. Procédé selon une quelconque revendication précédente, dans lequel la protéine de transport d'efflux cationique dérégulée comprend une ou plusieurs mutations par rapport à la séquence d'acides aminés représentée par SEQ ID NO 3, 6, 9, 12, 15, 18, 21, 24, 27 ou 30 et est une séquence qui présente au moins 70 % d'identité avec celle-ci.

6. Procédé selon une quelconque revendication précédente, lequel procédé comprend l'introduction, dans le génome de ladite plante ou de ladite cellule végétale, d'une mutation dans un polynucléotide codant pour une protéine d'efflux cationique de telle sorte que le polynucléotide code pour une protéine d'efflux cationique dérégulée.

7. Procédé selon la revendication 5 ou la revendication 6, dans lequel la mutation produit une délétion, un mutant d'épissage ou un codon codant pour une substitution d'acide aminé non tolérée dans le polynucléotide codant pour ladite protéine.

8. Procédé selon une quelconque revendication précédente, dans lequel la protéine d'efflux cationique dérégulée comprend une séquence d'acides aminés qui est dépourvue d'au moins une partie de l'extrémité N-terminale par rapport à une séquence d'acides aminés représentée par SEQ ID NO 3, 6, 9, 12, 15, 18, 21, 24, 27 ou 30 ou une séquence qui présente au moins 70 % d'identité avec celle-ci.

9. Procédé selon une quelconque revendication précédente, lequel procédé comprend l'introduction, dans le génome de ladite plante ou de ladite cellule végétale, d'une séquence polynucléotidique exogène qui code pour une protéine d'efflux cationique dérégulée.

10. Procédé selon une quelconque revendication précédente, dans lequel la protéine d'efflux cationique dérégulée est :
a) une protéine d'efflux cationique tronquée qui est dépourvue au moins d'acides aminés correspondant à 2-25, ou 2-50, ou 2-75, ou 2-100, 2-114, 2-117, 2-120, 2-125, 2-128 ou 2-130 du côté extrémité N-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, 2-114 ou 2-117 de SEQ ID NO 3, 6, 9, 12, 15, 18, 21, 24, 27 ou 30 ;
b) une protéine d'efflux cationique tronquée qui consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui se consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30.

11. Procédé selon l'une quelconque des revendications précédentes, dans lequel la TSNA est la N'-nitrosonornicotine (NNN) et/ou le précurseur est la nornicotine.

12. Procédé selon une quelconque revendication précédente,
dans lequel la protéine d'efflux cationique dérégulée comprend la séquence d'acides aminés représentée par SEQ ID NO 33, ou une séquence qui présente au moins 70 %, au moins 75 %, au moins 80 %, au moins 85 %, au moins 90 %, au moins 95 %, au moins 97 %, ou au moins 99 % d'identité de séquence avec celle-ci ; ou
dans lequel la protéine d'efflux cationique dérégulée est codée par un polynucléotide comprenant la séquence représentée par SEQ ID NO 34, ou une séquence qui présente au moins 70 %, au moins 75 %, au moins 80 %, au moins 85 %, au moins 90 %, au moins 95 %, au moins 97 %, ou au moins 99 % d'identité de séquence avec celui-ci.

13. Plante de tabac ou partie de celle-ci :
a. qui a été modifiée pour présenter une réduction d'une TSNA ou d'un précurseur d'une TSNA par rapport à une plante non modifiée, dans laquelle la plante modifiée, ou une partie de celle-ci, comprend une protéine d'efflux cationique dérégulée telle que définie dans la revendication 1 ;
b. qui présente une mutation dans un gène de la plante codant pour une protéine d'efflux cationique, laquelle mutation dérégule ladite protéine d'efflux cationique selon la revendication 1, dans laquelle le gène avant la mutation comprend la séquence représentée par SEQ ID NO 1, SEQ ID NO 2, SEQ ID NO 4, SEQ ID NO 5, SEQ ID NO 7, SEQ ID NO 8, SEQ ID NO 10, SEQ ID NO 11, SEQ ID NO 13, SEQ ID NO 14, SEQ ID NO 16, SEQ ID NO 17, SEQ ID NO 19, SEQ ID NO 20, SEQ ID NO 22, SEQ ID NO 23, SEQ ID NO 25, SEQ ID NO 26, SEQ ID NO 28 ou SEQ ID NO 29, ou une séquence qui présente au moins 70 % d'identité de séquence avec celle-ci ;
c. obtenue, ou pouvant être obtenue, par le procédé selon l'une quelconque des revendications 1-12,
d. comprenant un gène exogène qui code pour une protéine d'efflux cationique dérégulée telle que définie dans la revendication 1, dans laquelle ladite protéine comprend une séquence d'acides aminés tronquée qui est dépourvue au moins des acides aminés correspondant à 2-25, ou 2-50, ou 2-75, ou 2-100, ou 2-114, ou 2-117, ou 2-117, ou 2-120, ou 2-125, ou 2-128, ou 2-130 du côté extrémité N-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, ou 2-114, ou 2-117, ou 2-117, ou 2-120, ou 2-125, ou 2-128, ou 2-130 de SEQ ID NO 3, 6, 9, 12, 15, 18, 21, 24, 27 ou 30 ;
e. comprenant une protéine d'efflux cationique dérégulée telle que définie dans la revendication 1, qui comprend la séquence d'acides aminés présentée dans SEQ ID NO 33 ou une séquence présentant au moins 70 % d'identité avec celle-ci, ou une séquence polynucléotidique présentée dans SEQ ID NO 34 ou une séquence présentant au moins 70 % d'identité avec celle-ci ; ou
f. comprenant une construction ou un vecteur comprenant un polynucléotide codant pour une protéine d'efflux cationique dérégulée telle que définie dans la revendication 1, dans laquelle ledit polynucléotide comprend une séquence nucléotidique sélectionnée dans le groupe consistant en :
(i) une séquence nucléotidique comprenant SEQ ID NO 34 ;
(ii) une séquence nucléotidique présentant au moins 70 % d'identité de séquence avec l'intégralité de la séquence présentée dans SEQ ID NO 34, dans laquelle ladite séquence nucléotidique code pour un polypeptide impliqué dans un efflux cationique dans une plante ;
(iii) une séquence nucléotidique codant pour un polypeptide comprenant la séquence d'acides aminés présentée ici sous la référence SEQ ID NO 33, ou un fragment de celle-ci comprenant au moins 200, ou au moins 250, ou au moins 300, ou au moins 350, ou au moins 400 résidus contigus de SEQ ID NO 33 ;
(iv) une séquence nucléotidique codant pour un polypeptide tronqué qui est dépourvu au moins des acides aminés correspondant à 2-25, ou 2-50, ou 2-75, ou 2-100, ou 2-114, ou 2-117, ou 2-120, ou 2-125, ou 2-128, ou 2-130 du côté extrémité N-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, ou 2-114, ou 2-117, ou 2-117, ou 2-120, ou 2-125, ou 2-128, ou 2-130 de SEQ ID NO 3, 6, 9, 12, 15, 18, 21, 24, 27 ou 30 ;
(v) une séquence nucléotidique codant pour un polypeptide tronqué qui consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30 ;
(vi) une séquence nucléotidique codant pour un polypeptide comprenant une séquence d'acides aminés qui correspond aux acides aminés 125-324 de SEQ ID NO 3, 6, 9, 12, 15, 18, 21, 24, 27 ou 30 et/ou comprenant une séquence d'acides aminés qui correspond aux acides aminés 323-413 de SEQ ID NO 3, 6, 9, 12, 15, 18, 21, 24, 27 ou 30, dans laquelle ladite séquence nucléotidique code pour un polypeptide impliqué dans l'efflux cationique dans une plante ; ou
(vii) une séquence nucléotidique selon l'un des précédents éléments (i) à (vi) qui comprend en outre une mutation dans ladite séquence nucléotidique, par exemple lorsque ladite mutation produit une délétion, un mutant d'épissage ou un codon codant pour une substitution d'acide aminé non tolérée dans le polynucléotide codant pour ladite protéine ;
ou comprenant un polynucléotide codant pour un polypeptide qui est une protéine d'efflux cationique dérégulée telle que définie dans la revendication 1, dans laquelle ledit polypeptide comprend une séquence d'acides aminés sélectionnée dans le groupe consistant en :
(viii) une séquence d'acides aminés comprenant SEQ ID NO 33 ;
(ix) une séquence d'acides aminés qui est au moins identique à 70 % à une séquence d'acides aminés présentée dans SEQ ID NO 33 ;
(x) une séquence d'acides aminés comprenant des acides aminés qui correspondent aux acides aminés 125-324 de SEQ ID NO 3, 6, 9, 12, 15, 18, 21, 24, 27 ou 30 et/ou comprenant des acides aminés qui correspondent aux acides aminés 323-413 de SEQ ID NO 3, 6, 9, 12, 15, 18, 21, 24, 27 ou 30, dans laquelle ledit polypeptide est impliqué dans l'efflux cationique dans une plante ;
(xi) une séquence d'acides aminés qui est un fragment de SEQ ID NO 3, 6, 9, 12, 15, 18, 21, 24, 27 ou 30 comprenant au moins 200, ou au moins 250, ou au moins 300, ou au moins 350, ou au moins 400 résidus contigus de SEQ ID NO 3, 6, 9, 12, 15, 18, 21, 24, 27 ou 30 ;
(xii) un acide aminé qui est une séquence d'acides aminés tronquée qui est dépourvue au moins des acides aminés correspondant à 2-25, ou 2-50, ou 2-75, ou 2-100, ou 2-114, ou 2- 117, ou 2-117, ou 2-120, ou 2-125, ou 2-128, ou 2-130 du côté extrémité N-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, ou 2-114, ou 2-117, ou 2-117, ou 2-120, ou 2-125, ou 2-128, ou 2-130 de SEQ ID NO 3, 6, 9, 12, 15, 18, 21, 24, 27 ou 30 ;
(xiii) un acide aminé qui est une séquence d'acides aminés tronquée qui consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui consiste essentiellement en, ou consiste en au moins 375, au moins 350, ou au moins 300, ou au moins 250, ou au moins 200, ou au moins 150 acides aminés provenant de l'extrémité C-terminale de SEQ ID NO 3, 6, 9, 12, 15, 18, 21, 24, 27 ou 30 ; ou
(xiv) une séquence d'acides aminés présentant au moins 70 % d'identité de séquence avec l'intégralité de la séquence présentée dans SEQ ID NO 33,
à condition que la plante de tabac ne soit pas exclusivement issue d'un processus essentiellement biologique.

14. Matériel de propagation de plante (par exemple, une graine de plante) pouvant être obtenu à partir d'une plante selon la revendication 13, à condition que la plante de tabac ne soit pas exclusivement issue d'un processus essentiellement biologique, et ledit matériel de propagation exprime une protéine d'efflux cationique dérégulée telle que définie dans revendication 1.

15. Procédé selon l'une quelconque des revendications 1-12, ou plante de tabac ou partie végétale de tabac selon la revendication 13, ou matériel de propagation de plante selon la revendication 14, dans lequel la plante appartient à l'espèce *Nicotiana tabacum* ou *Nicotiana rustica.*

16. Utilisation d'une plante de tabac ou d'une partie végétale de tabac selon la revendication 13, ou d'une protéine d'efflux cationique dérégulée telle que définie dans l'une quelconque des revendications 1 à 12, pour la production d'un produit de l'industrie du tabac.

17. Utilisation d'une plante de tabac selon la revendication 13 ou d'un matériel de propagation de plante selon la revendication 14 pour faire pousser une culture ou pour produire une feuille de tabac transformée (de préférence séchée).

18. Feuille récoltée d'une plante de tabac selon la revendication 13 ou pouvant être obtenue à partir d'une plante de tabac propagée à partir d'un matériel de propagation selon la revendication 14 ou pouvant être obtenue à partir d'une plante de tabac pouvant être obtenue par un procédé selon l'une quelconque des revendications 1 à 12 ou pouvant être obtenue par une utilisation selon la revendication 16 ou la revendication 17, facultativement dans laquelle la feuille récoltée est une feuille récoltée coupée, à condition que la plante de tabac ne puisse pas être obtenue exclusivement par un processus essentiellement biologique, et qu'elle exprime une protéine d'efflux cationique dérégulée telle que définie dans la revendication 1.

19. Feuille de tabac transformée (de préférence une feuille de tabac transformée non viable) :
a. pouvant être obtenue à partir du traitement d'une plante de tabac selon la revendication 13 ;
b. pouvant être obtenue à partir d'une plante de tabac propagée à partir d'un matériel de propagation de plante selon la revendication 14 ; ou
c. pouvant être obtenue par traitement d'une feuille récoltée selon la revendication 18, facultativement dans laquelle la plante est transformée, ou la feuille est transformée, par séchage, fermentation, pasteurisation ou des combinaisons de ceux-ci et/ou facultativement, dans laquelle la feuille de tabac transformée est une feuille de tabac transformée coupée, à condition que la plante de tabac ne soit pas exclusivement issue d'un processus essentiellement biologique, et que ladite plante comprenne une protéine d'efflux cationique dérégulée telle que définie dans la revendication 1.

20. Matière de tabac séché fabriquée à partir d'une plante ou d'une partie de celle-ci selon la revendication 13 ou est obtenue (ou peut être obtenue) à partir du matériel de propagation de plante selon la revendication 14 ou obtenue (ou peut être obtenue) par le procédé selon l'une quelconque des revendications 1-12.

21. Mélange de tabac comprenant ladite matière de tabac séchée selon la revendication 20.

22. Produit de l'industrie du tabac :
a. préparé à partir d'une plante de tabac selon la revendication 13 ou d'une partie de celle-ci ;
b. préparé à partir d'une plante de tabac ou d'une partie de celle-ci (de préférence, les feuilles récoltées sur la plante) obtenue ou pouvant être obtenue par le procédé selon l'une quelconque des revendications 1-12 ;
c. préparé à partir de la plante (de préférence, les feuilles) propagée à partir d'un matériel de propagation de plante selon la revendication 14 ;
d. préparé à partir d'une feuille récoltée selon la revendication 18 ;
e. préparé à partir d'une feuille transformée selon la revendication 19 ;
f. préparé à partir d'une matière de tabac séchée selon la revendication 20 ; ou
g. préparé à partir d'un mélange de tabac selon la revendication 21, facultativement dans lequel le produit de l'industrie du tabac est un article à fumer combustible ou un produit de l'industrie du tabac sans fumée ou un système de fourniture d'aérosol non combustible, tel qu'un dispositif de chauffage du tabac (par exemple, un dispositif de génération d'aérosol).

23. Article à fumer combustible, système de fourniture d'aérosol non combustible, produit de l'industrie du tabac sans fumée ou dispositif de chauffage du tabac comprenant une plante ou une partie de celle-ci de l'espèce *Nicotiana tabacum* ou *Nicotiana rustica* selon la revendication 13, ou pouvant être obtenue (par exemple, obtenue) à partir d'un produit de propagation de plante selon la revendication 14, ou pouvant être obtenue (par exemple, obtenue) à partir d'un procédé selon l'une quelconque des revendications 1 à 12.

24. Mutant d'une plante portant une mutation héréditaire dans une séquence nucléotidique d'au moins un gène codant pour une protéine d'efflux cationique dérégulée telle que définie dans la revendication 1, comprenant la séquence d'acides aminés représentée par SEQ ID NO 33, ou une séquence qui présente au moins 70 % d'identité avec celle-ci, ou une séquence nucléotidique qui code pour une protéine d'efflux cationique dérégulée telle que définie dans la revendication 1, dans lequel ladite protéine comprend une séquence d'acides aminés tronquée qui est dépourvue au moins des acides aminés correspondant à 2-25, ou 2-50, ou 2-75, ou 2-100, ou 2-114, ou 2-117, ou 2-117, ou 2-120, ou 2-125, ou 2-128, ou 2-130 du côté extrémité N-terminale de SEQ ID NO 3, 6, 9, 12, 15, 18, 21, 24, 27 ou 30, ou une protéine qui présente au moins 70 % (de préférence au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, ou 2-114, ou 2-117, ou 2-117, ou 2-120, ou 2-125, ou 2-128, ou 2-130 de SEQ ID NO 3, 6, 9, 12, 15, 18, 21, 24, 27 ou 30 ; dans lequel ladite mutation héréditaire diminue la teneur d'au moins une TSNA ou d'un précurseur d'une TSNA par rapport à une plante comparable qui ne porte pas ladite mutation héréditaire, ou
une descendance ou une graine de ladite plante mutante qui porte la mutation héréditaire, à condition que la plante de tabac ne soit pas exclusivement issue d'un processus essentiellement biologique.

25. Feuille récoltée, feuille transformée ou matière de tabac séchée produite à partir d'une plante comprenant une mutation dans une séquence nucléotidique codant pour une protéine d'efflux cationique dérégulée telle que définie dans la revendication 1, comprenant la séquence d'acides aminés représentée par SEQ ID NO 33, ou une séquence qui présente au moins 70 % d'identité avec celle-ci, ou une séquence nucléotidique qui code pour une protéine d'efflux cationique dérégulée telle que définie dans la revendication 1, dans laquelle ladite protéine comprend une séquence d'acides aminés tronquée qui est dépourvue au moins des acides aminés correspondant à 2-25, ou 2-50, ou 2-75, ou 2-100, ou 2-114, ou 2-117, ou 2-117, ou 2-120, ou 2-125, ou 2-128, ou 2-130 du côté extrémité N-terminale de SEQ ID NO 3, 6, 9, 12, 15, 18, 21, 24, 27 ou 30, ou une protéine qui présente au moins 70 % (de préférence, au moins 75 %, au moins 80 %, au moins 85 %, au moins 90 %, au moins 95 %, au moins 97 %, ou au moins 99 %) d'identité de séquence avec une protéine tronquée qui est dépourvue au moins des acides aminés correspondant aux acides aminés 2-25, ou 2-50, ou 2-75, ou 2-100, ou 2-114, ou 2-117, ou 2-117, ou 2-120, ou 2-125, ou 2-128, ou 2-130 de SEQ ID NO 3, 6, 9, 12, 15, 18, 21, 24, 27 ou 30 ; dans laquelle ladite feuille récoltée, ladite feuille transformée ou le tabac séché présente une teneur réduite d'au moins une TSNA ou d'un précurseur d'une TSNA par rapport à une plante comparable qui ne porte pas ladite modification, à condition que la plante de tabac ne soit pas exclusivement issue d'un processus essentiellement biologique.
